# EUROPEAN PATENT SPECIFICATION

(11) **EP 4 278 351 B1**
(45) Date of publication and mention of the grant of the patent: **25.02.2026**
(21) Application number: 22701882.7
(22) Date of filing: 12.01.2022
(51) Int. Cl.: G10L 25/66, A61B 5/16, A61B 5/00, A61B 5/0205, G10L 15/04, G10L 25/03, G10L 25/90, G10L 25/93

(54) **SPEECH-ANALYSIS BASED AUTOMATED PHYSIOLOGICAL AND PATHOLOGICAL ASSESSMENT**
SPRACHANALYSEBASIERTE AUTOMATISCHE PHYSIOLOGISCHE UND PATHOLOGISCHE BEURTEILUNG
ÉVALUATION AUTOMATIQUE PATHOLOGIQUE ET PHYSIOLOGIQUE BASÉE SUR L'ANALYSE DE LA PAROLE

(30) Priority: 13.01.2021 EP 21151442
(43) Date of publication of application: 22.11.2023
(73) Proprietor: F. Hoffmann-La Roche AG, 4070 Basel (CH); Universitätsspital Basel, 4031 Basel (CH)
(72) Inventor: STRAHM, Martin Christian, 4070 Basel (CH); ZHANG, Yan-Ping, 4070 Basel (CH); ZHOU, Qian, 4031 Basel (CH)
(74) Representative: Mewburn Ellis LLP
(86) International application number: PCT/EP2022/050545
(87) International publication number: WO 2022/152751

(56) References cited:
- WO-A1-2014/062441
- WO-A2-2011/011413
- US-A1- 2020 294 531
- JOHANNES SCHUMACHER ET AL: "Strong Genetic Evidence of DCDC2 as a Susceptibility Gene for Dyslexia", THE AMERICAN JOURNAL OF HUMAN GENETICS, vol. 78, no. 1, 1 January 2006 (2006-01-01), pages 52 - 62, XP055142937, ISSN: 0002-9297, DOI: 10.1086/498992
- DAVIS S B ET AL: "COMPARISON OF PARAMETRIC REPRESENTATIONS FOR MONOSYLLABIC WORD RECOGNITION IN CONTINUOUSLY SPOKEN SENTENCES", IEEE TRANSACTIONS ON ACOUSTICS, SPEECH AND SIGNAL PROCESSING, IEEE INC. NEW YORK, USA, vol. ASSP-28, no. 4, 4 August 1980 (1980-08-04), pages 357 - 366, XP002036829, ISSN: 0096-3518, DOI: 10.1109/TASSP.1980.1163420
- RUSZ JAN ET AL: "Quantitative assessment of motor speech abnormalities in idiopathic rapid eye movement sleep behaviour disorder", SLEEP MEDICINE, ELSEVIER, AMSTERDAM, NL, vol. 19, 14 September 2015 (2015-09-14), pages 141 - 147, XP029539562, ISSN: 1389-9457, DOI: 10.1016/J.SLEEP.2015.07.030

## Description

### Field of the Invention

The present invention relates to computer-implemented methods for automated assessment of the physiological and/or pathological state of a subject, comprising in particular analysing voice recordings from word-reading tests. Computing devices implementing the methods are also described. The methods and devices of the invention find applications in the clinical assessment of pathological and physiological conditions that affect breathing, vocal tone, fatigue, and/or cognitive ability.

### Background to the invention

Remote monitoring of patients with a variety of conditions has the potential to improve healthcare outcome, quality and comfort for many patients. Thus, there has been a lot of interest in developing devices and methods that patients can use to collect biomarker data themselves, which can then be assessed by the patients' medical team. The potential benefits of remote monitoring are particularly stringent in the context of chronic diseases or lifelong conditions such as heart disease or asthma. Non-invasive biomarker based approaches are particularly desirable due to their lower risk. The use of vocal analysis to collect such biomarker information has been suggested for example in the assessment of heart failure (Maor et al., 2018), asthma, chronic obstructive pulmonary disease (COPD) (Saeed et al., 2017), and more recently COVID-19 (Laguarta et al., 2020).

However, all of these approaches suffer from consistency limitations. Indeed, many of these approaches rely on spontaneous speech or sound (such as cough), or reading of a set standard passage such as the Rainbow passage (Murton et al., 2017). The use of spontaneous speech or sound suffers from high variability, both between patients and between repeated assessments of the same patient, since the content of each voice recording can vary widely. The use of a set standard passage controls for this inherent variability due to content, but suffers from interference with neuropsychological effects associated with the subjects becoming accustomed to the standard text as the test is repeated. This imposes strong limitations on the practical use of voice analysis biomarkers in the context of remote monitoring.

Therefore, there is still a need for improved methods to automatically assess pathological and physiological conditions that can be performed remotely and easily, with minimal burden on patients.

US 2020/294531 A1 describes diagnostic techniques based on speech-sample alignment. Johannes Schumacher et al. ("Strong Genetic Evidence of DCDC2 as a Susceptibility Gene for Dyslexia", The American Journal of Human Genetics, vol. 78, no. 1, 1 January 2006) describes genetic evidence of a susceptibility gene for dyslexia, and tests targeting different aspects of the dyslexia phenotype including a single-word reading test. WO 2011/011413 A2 describes methods for remote evaluation of a subject's emotive and/or physiological state through the measurement and analysis of vital signs and/or speech. Davis S B et al. ("Comparison of parametric representations for monosyllabic word recognition in continuously spoken sentences", IEEE Transactions On Acoustics, Speech And Signal Processing, IEEE INC. New York, USA, vol. ASSP-28, no. 4, 4 August 1980) describes a comparison n of several parametric representations of the acoustic signal with regard to word recognition performance in a syllable-oriented continuous speech recognition system. Rusck Jan et al. ("Quantitative assessment of motor speech abnormalities in idiopathic rapid eye movement sleep behaviour disorder", Sleep Medicine, Elsevier, Amsterdam, vol. 19, 14 September 2015) describes the objective acoustic assessment of 15 speech dimensions representing various phonatory, articulatory and prosodic deviations in RBD subjects and healthy control subjects. WO 2014/062441 A1 describes the detection of neurological diseases such as Parkinson's disease through analyzing a subject's speech for acoustic measures based on human factor cepstral coefficients.

### Statements of invention

The inventors have developed a new device and method for automated assessment of the physiological and/or pathological state of a subject, comprising in particular analysing voice recordings from word-reading tests. The invention is as defined in appended independent claims 1 and 15. Preferred embodiments are defined in the dependent claims. The inventors have identified that recordings from word-reading tests, such as the Stroop test, could be used to derive reproducible and informative biomarkers for the assessment of the pathological and/or physiological state of a subject, and in particular for the assessment of conditions that affect breathing, vocal tone, fatigue, and/or cognitive ability.

The Stroop test (Stroop, 1935), is a three-part neuropsychological test (word, color, and interference) that has been used to diagnose psychiatric and neurological disorders. For example, it forms part of the cognitive test battery performed to quantify Huntington's Disease (HD) severity according to the widely used Unified Huntington's Disease Rating Scale (UHDRS). The word and color parts of the Stroop test represent the "consistent condition" in which color-words are printed in black ink and color-patches are printed in a matching ink color. In the interference part, color-words are printed inconsistently with ink color. Patients are required to read words or name ink colors as fast as possible. The clinician interprets the response as correct or incorrect. Scores are reported as the number of correct answers in each condition for a given 45-second period. The consistent condition is considered to measure processing speed and selective attention. The interference condition requires a mental conversion between words and colors, thus, is intended to measure cognitive flexibility.

The methods described herein are based on automatically determining, from recordings of word-reading tests inspired from the Stroop test, one or more metrics that have been identified to be usable as biomarkers, the metrics selected from voice pitch, correct words rate, breathing percentage and unvoicing/voicing ratio. The methods are language-independent, fully automated, reproducible and applicable to a variety of conditions that affect breathing, vocal tone, fatigue, and/or cognitive ability. Thus it enables remote self-assessment and monitoring, in large populations, of symptoms, diagnosis or prognosis of such conditions.

Thus, according to a first aspect, there is provided a computer-implemented method of assessing the pathological and/or physiological state of a subject, the method comprising: obtaining a voice recording from a word-reading test from the subject, wherein the voice recording is from a word-reading test comprising reading a sequence of words drawn from a set of n words, wherein the sequence of words are not connected so as to form a sentence and are drawn randomly or pseudo-randomly from the set of n words; and analysing the voice recording, or a portion thereof, by: identifying a plurality of segments of the voice recording that correspond to single words or syllables; determining the value of one or more metrics selected from the breathing %, unvoicing/voicing ratio, voice pitch and correct word rate at least in part based on the identified segments; comparing the value of the one or more metrics with one or more respective reference values.

The method may have any one or more of the following features.

Identifying segments of the voice recording that correspond to single words or syllables may comprise: obtaining a power Mel-spectrogram of the voice recording; computing the maximum intensity projection of the Mel spectrogram along the frequency axis; and defining a segment boundary as the time point where the maximum intensity projection of the Mel spectrogram along the frequency axis crosses a threshold.

The approach to word/syllable segmentation described herein enables the accurate and sensitive segmentation of words (and in some cases also syllables from multi-syllables words) from voice recordings even where the speech is relatively fast paced (i.e. comprising no or short pauses between words), where existing methods typically based on energy envelopes may not perform well. It further enables the automated quantification of metrics derived from the identified voicing segments (such as e.g. the breathing %, unvoicing/voicing ratio, and rates such as the correct word rate) in a word reading task from data that can be simply and easily acquired remotely, such as e.g. by a patient recording themselves reading words displayed on a computing device (e.g. a mobile computing device such as a smartphone or tablet or a personal computer, through an application or web application, as will be described further herein).

A segment of voice recording corresponding to a single word or syllable may be defined as a segment comprised between two consecutive word/syllable boundaries. Preferably, a segment of voice recording corresponding to a single word/syllable may be defined as a segment between a first boundary where the maximum intensity projection of the Mel spectrogram crosses the threshold from a lower to a higher value, and a second boundary where the maximum intensity projection of the Mel spectrogram crosses the threshold from a higher to a lower value. Advantageously, segments of voice recording between boundaries that do not satisfy this definition may be excluded.

Determining the value of one or more metrics may comprises determining a breathing percentage associated with the recording as the percentage of time in the voice recording that is between the identified segments, or the ratio of the time between the identified segments in the recording and the sum of the time between the identified segments and within identified segments in the recording.

Determining the value of one or more metrics may comprise determining a unvoicing/voicing ratio associated with the recording as the ratio of the time between the identified segments in the recording and the time within identified segments in the recording.

Determining the value of one or more metrics may comprise determining the correct word rate associated with the voice recording by computing the ratio of the number of identified segments corresponding to correctly read words divided by the time duration between the start of the first identified segment and the end of the last identified segment.

Determining the value of one or more metrics may comprise determining a voice pitch associated with the recording by obtaining one or more estimates of the fundamental frequency for each of the identified segments. Determining the value of the voice pitch may comprise obtaining a plurality of estimates of the fundamental frequency for each of the identified segment, and applying a filter to the plurality of estimates to obtain a filtered plurality of estimates. Determining the value of the voice pitch may comprise obtaining a summarised voice pitch estimate for a plurality of segments, such as e.g. the mean, median or mode of the (optionally filtered) plurality of estimates for the plurality of segments.

Determining the value of one or more metrics may comprise determining the total or correct word rate associated with the voice recording by computing a cumulative sum of the number of identified segments corresponding to read or correctly read words in the voice recording over time, and computing the slope of a linear regression model fitted to the cumulative sum data. Advantageously, this approach results in a robust estimate of the total or correct word rate as a number of read or correctly read words per unit of time over the entire recording. The estimate thus obtained may be robust to outliers (such as e.g. distractions that may cause isolated momentary changes of the correct word rate), while being sensitive to genuine slowing of the total or correct word rate (such as e.g. where genuine fatigue, breathing and/or cognitive impairment leads to frequent segments with slow speech). Additionally, this approach is independent of the length of the recording. As such, it may enable the comparison of the total or correct word rates obtained for voice recordings of different lengths, or for different portions of the same voice recording. Further, it may be robust to external factors such as a subject pausing or not speaking for reasons not related to cognitive or breathing impairment (such as e.g. because the subject initially does not realise that the recording has started). Further, this approach is also advantageously robust to uncertainty in relation to the specific timing of the start of words and/or to variation in the duration of the words be taken into account.

Where the method comprises determining the correct word rate in the voice recording, the method may comprise: computing one or more Mel-frequency cepstral coefficients (MFCCs) for each the segments to obtain a plurality of vectors of values, each vector being associated with a segment; clustering the plurality of vector of values into n clusters, wherein each cluster has n possible labels corresponding to each of the n words; for each of the n! permutations of labels, predicting a sequence of words in the voice recording using the labels associated with the clustered vectors of values, and performing a sequence alignment between the predicted sequence of words and the sequence of words used in the word reading test; selecting the labels that result in the best alignment, wherein the matches in the alignment correspond to the correctly read words in the voice recording.

Advantageously, the approach to determining the correct word rate described herein is entirely driven by the data and is as such model and language independent. In particular, as the clustering step is an unsupervised learning step, it does not require any knowledge of the actual word that each group of segments represents (ground truth). In alternative embodiments, it is possible for the clustering to be replaced with a supervised learning approach such as a Hidden Markov Model. However, such an approach would require a re-training of the model for each language.

Advantageously, the approach to determining the correct word rate described herein is further able to deal with speech impairments such as dysarthria which may prevent conventional word recognition approaches from identifying words that are correctly read but incorrectly pronounced. It further enables the automated quantification of the correct word rate in a word reading task from data that can be simply and easily acquired remotely, such as e.g. by a patient recording themselves reading words displayed on a computing device (e.g. a mobile computing device such as a smartphone or tablet).

In embodiments, predicting a sequence of words in the voice recording using the labels associated with the clustered vectors of values comprises predicting a sequence of words that correspond to the respective cluster labels for each of the clustered vectors of values, ordered following the order of the segments from which the vectors of values were derived.

In some embodiments, predicting a sequence of words in the voice recording using the labels associated with the clustered vectors of values comprises predicting a sequence of words that correspond to the respective cluster labels for each of the clustered vectors of values that are assigned to a cluster with a confidence that meets one or more predetermined criteria. In other words, predicting a sequence of words in the voice recording using the labels associated with the clustered vectors of values comprises may comprise excluding predictions for clustered vectors of values that are not associated with any specific clusters with a confidence that meets one or more predetermined criteria. The one or more predetermined criteria may be defined using a threshold on the probability that a vector of values belong to one of the n clusters, the distance between a vector of values and a representative vector of values for one of the n clusters (e.g. the coordinates of the medoid or centroid of the cluster), or combinations thereof.

In some embodiments, predicting a sequence of words in the voice recording using the labels associated with the clustered vectors of values comprises predicting a sequence of words that correspond to the respective cluster labels for each of the clustered vectors of values. In some such embodiments, where multi-syllable words (especially multi-syllable words comprising one emphasized syllable) are used, multiple word predictions may be predicted for a multi-syllable word, since multiple segments may be identified and clustered. It was found that it is still be possible to determine the number of correctly read words in the voice recording according to the methods described herein in such situations. Indeed, as explained above, it is believed that the clustering step may be robust to the presence of "noise" coming from additional syllables, such that clusters primarily determined by single syllables in each of the n words could still be identified. Further, it is believed that the sequence alignment step would be able to deal with such additional syllables as insertions within the sequence, which insertions would be present for each of the n! permutations of labels since they result from the presence of additional predicted words that are not expected to be present in the sequence of words used in the word reading test. As such, the number of matches in the alignment would still correspond to the number of correctly read words in the voice recording.

In embodiments, computing one or more MFCCs to obtain a vector of values for a segment comprises: computing a set of *i* MFCCs for each frame of the segment and obtaining a set of *j* values for the segment by compressing the signal formed by each of the i MFCCs across the frames in the segment, to obtain a vector of *ixj* values for the segment. For example, compressing the signal formed by each of the i MFCCs across the frames in the segment may comprise performing linear interpolation of said signal.

In embodiments, computing one or more MFCCs to obtain a vector of values for a segment comprises: computing a set of *i* MFCCs for each frame of the segment and obtaining a set *of j* values for the segment for each *i* by interpolation, preferably linear interpolation, to obtain a vector of *ixj* values for the segment.

As a result, the vector of values for each of the plurality of segments all have the same length. Such vectors of values can advantageously be used as an input for any clustering approach that identifies clusters of points in a multidimensional space.

Computing one or more MFCCs to obtain a vector of values for a segment may be performed as explained above. As the skilled person understand, the used of a fixed length time window to obtain MFCCs for a segment means that the total number of MFCCs per segment may vary depending on the length of the segment. In other words, a segment will have a number of frames f, each being associated with a set of i MFCCs, where fvaries depending on the length of the segment. As a result, segments corresponding to longer syllables/words will be associated with a larger number of values than segments corresponding to shorter syllables/words. When these values are used as features representative of a segment for the purpose of clustering segments in a common space, this may be problematic. The interpolation step solves this problem. In embodiments, computing one or more MFCCs for a segment comprises computing a plurality of the second to thirteenth MFCCs for each frame of the segment. The first MFCCs is preferably not included. Without wishing to be bound by theory, it is assumed that the first MFCC represents the energy in a segment, which is primarily relevant to the recording condition and contains little information in relation to the identity of a word or syllable. By contrast, the remaining 12 MFCCs cover the human hearing range (by definition of MFCCs) and hence capture the sound features that relate to how humans produce and hear words.

In embodiments, the plurality of the second to thirteenth MFCCs comprise at least 2, at least 4, at least 6, at least 8, at least 10 or all 12 of the second to thirteenth MFCCs. The second to thirteenths MFCCs may advantageously contain information that can be used to distinguish words from a closed set of words as points in a hyperspace, using simple clustering approaches. In particular, as explained above, the second to thirteenth MFCCs cover the human hearing range and are therefore believed to capture the sound features that relate to how humans produce and hear words. As such, using those 12 MFCCs may advantageously capture the information that is believed to be relevant in differentiating one word/syllable from another in a human voice recording.

Where the segmentation method described herein is used, the MFCCs for each frame of the identified segments may already have been computed as part of the step of excluding segments that represent erroneous detections. In such embodiments, the previously computed MFCCs may advantageously be used to obtain a vector of values for the purpose of determining the number of correctly read words in the voice recording.

In embodiments, the parameter *j* is chosen such that *j* ≤ f for all segments used in the clustering step. In other words, the parameter *j* may be chosen such that the interpolation results in a compression of the signal (where for each MFCC, the signal is the value of said MFCC across the frames of the segment). In embodiments, the parameter *j* may be chosen such that the interpolation results in a compression of the signal by between 40 and 60% for all segments (or at least a set proportion, such as e.g. 90%, of the segments) used in the clustering. As the skilled person understands, using a fixed parameter *j*, the level of compression applied to a segment may depend on the length of the segment. Using a compression to between 40 and 60% of the signal may ensure that the signal in each segment is compressed to about half of its original signal density.

In convenient embodiments, *j* is chosen between 10 and 15, such as e.g. 12. Without wishing to be bound by theory, frames of 25 ms with a step size of 10 ms are commonly used for the calculation of MFCCs for a sound signal. Further, syllables (and monosyllabic words) may be about 250 ms long on average. As such, using *j=12* may result in a compression from 25 values (corresponding to 25 frames over a 250 ms segment) on average, to about half of this number of values (i.e. about 40 to 60% compression on average).

In embodiments, clustering the plurality of vector of values into n clusters is performed using k-means. Advantageously, k-means is a simple and computationally efficient approach that was found to perform well at separating words represented by vectors of MFCC values. Alternatively, other clustering approaches may be used such as partition around medoid or hierarchical clustering.

Further, the centroid of clusters obtained may correspond to a representation of the corresponding word or syllable in the MFCC space. This may provide useful information about the process (e.g. whether the segmentation and/or clustering has performed satisfactorily) and/or about the voice recording (and hence the subject). In particular, the centroid of such clusters can be compared between individuals and/or used as a further clinically informative measure (e.g. because it captures aspects of the subject's ability to articulate a syllable or word).

In embodiments, the one or more MFCCs are normalised across segments in a recording, prior to clustering and/or interpolation. In particular, each MFCC may be individually centred and standardised, resulting in each MFCC distribution having equal variance and a mean of zero. This may advantageously improve the performance of the clustering process, as it may prevent some MFCCs from "dominating" the clustering if they are distributed with high variance. In other words, this may ensure that all features in the clustering (i.e. each MFCC used) has a similar importance in the clustering.

In embodiments, performing a sequence alignment comprises obtaining an alignment score. In some such embodiments, the best alignment is the alignment that satisfies one or more predetermined criteria, at least one of these criteria applying to the alignment score. In embodiments, the best alignment is the alignment that has the highest alignment score.

In embodiments, the sequence alignment step is performed using a local sequence alignment algorithm, preferably the Smith-Waterman algorithm.

A local sequence alignment algorithm is ideally suited to the task of aligning two strings of letters selected from a closed set, where the strings are relatively short and may not necessarily have the same length (as is the case here since words may have been missed in the reading task and/or in the word segmentation process). In other words, a local sequence alignment algorithm such as the Smith-Waterman algorithm is particularly well-suited for the alignment of partially overlapping sequences, This is advantageous in the context of the present invention since alignments with mismatches and gaps are expected due to the subject achieving less than 100% correct words count and/or due to errors in the segmentation process.

In embodiments, the Smith-Waterman algorithm is used with a gap cost between 1 and 2 (preferably 2) and a match score = 3. These parameters may lead to an accurate identification of words in the voice recording, by comparison to manually annotated data. Without wishing to be bound by theory, using a higher gap cost (e.g. 2 instead of 1) may lead to a restriction of the search space, and shorter alignments. This may advantageously capture a situation where matches are expected (i.e. it is assumed that there exists a cluster label assignment that is such that many characters of the predicted sequence of words can be aligned with characters of the known sequence of words).

In embodiments, identifying segments of the voice recording that correspond to single words or syllables further comprises normalising the power Mel-spectrogram of the voice recording. Preferably, the power Mel-spectrogram is normalised against the frame that has the highest energy in the recording. In other words, each value of the power Mel-spectrogram may be divided by the highest energy value in the power Mel-spectrogram.

As the skilled person understands, a power Mel-spectrogram refers to a power spectrogram for a sound signal, on a Mel scale. Further, obtaining a Mel-spectrogram comprises defining frames along the voice recording (where a frame can correspond to the signal in a window of fixed width applied along the time axis) and computing a power spectrum on a Mel scale for each frame. This process results in a matrix of values of power per Mel unit per frame (time bin). Obtaining the maximum intensity projection against the frequency axis for such a spectrogram comprises selecting the maximum intensity on the Mel spectrum for each frame.

The normalisation advantageously eases the comparison between different voice recordings, which may be associated with the same or different subjects. This may be particularly advantageous e.g. where multiple individual recordings from the same subject are combined. For example, this may be particularly advantageous where short recordings are preferred (e.g. because the subject is frail), where a standard or otherwise desirable length of word reading test is preferable. Normalising the Mel-spectrogram against the frame that has the highest energy in the recording advantageously results in the loudest frame in the recording having a relative energy value (value after the maximum intensity projection) of 0dB for any recording. Other frames will have relative energy values below 0 dB. Further, as normalising the power Mel-spectrogram results in maximum intensity projections that represent relative energy (values of dB over time) comparable between voice recordings, a common threshold (which may be predetermined or dynamically determined) can advantageously be used for multiple recording.

Applying an outlier detection method to data derived from single word/syllable segments advantageously enables the removal of segments that correspond to erroneous detections (such as e.g. those caused by imprecise articulation, respirations and non-speech sound.) Any outlier detection methods that are applicable to a set of multidimensional observations may be used. For example, clustering approaches may be used. In embodiments, applying an outlier detection method to the plurality of vectors of values comprises excluding all segments whose vector of values is above a predetermined distance from the remaining vectors of values.

Identifying segments of the voice recording that correspond to single words or syllables may further comprise performing onset detection for at least one of the segments by computing a spectral flux function over the Mel-spectrogram of the segment, and defining a further boundary whenever an onset is detected within a segment, thereby forming two new segments.

In embodiments, identifying segments of the voice recording that correspond to single words/syllables further comprises excluding segments that represent erroneous detections by removing segments shorter than a predetermined threshold and/or with mean relative energy below a predetermined threshold. For example, segments shorter than 100 ms may advantageously be excluded. Similarly, segments with a mean relative energy below -40 dB may advantageously be excluded. Such an approach may simply and efficiently exclude segments that do correspond to words or syllables. Preferably, the segments are filtered to exclude short and/or low energy segments prior to calculating MFCCs for segments and applying an outlier detection method as explained above. Indeed, this advantageously avoids the unnecessary step of computing MFCCs for erroneous segments, and prevents such erroneous segments from introducing further noise in the outlier detection method.

In some embodiment of any aspect, the voice recording includes a reference tone. For example, the recording may have been obtained using a computing device configured to emit a reference tone shortly after starting to record the user performing the reading test. This may be useful to provide the user with an indication of when to begin the reading task. In embodiments where the voice recording includes a reference tone, one or more parameters of the method may be chosen such that the reference tone is identified as a segment that corresponds to a single words or syllable, and/or such that a segment comprising the reference tone is excluded in a process to remove erroneous detections. For example, the set of MFCCs used in the erroneous detection removal process and/or the predetermined distance used in this process may be chosen such that the segment corresponding to the reference tone is removed in each voice recording (or at least a chosen percentage of voice recordings).

Identifying segments of the voice recording that correspond to single words or syllables may further comprise excluding segments that represent erroneous detections by computing one or more Mel-frequency cepstral coefficients (MFCCs) for the segments to obtain a plurality of vectors of values, each vector being associated with a segment, and applying an outlier detection method to the plurality of vectors of values. Identifying segments of the voice recording that correspond to single words or syllables may further comprise excluding segments that represent erroneous detections by removing segments shorter than a predetermined threshold and/or with mean relative energy below a predetermined threshold.

The n words may be monosyllabic or disyllabic. The n words may each include one or more vowels that are internal to the respective word. The n words may each include a single emphasized syllable. The n words may be color words, optionally wherein the words are displayed in a single color in the word reading test, or wherein the words are displayed in a color independently chosen from a set of m colors in the word reading test.

Within the context of the present invention, the subject is a human subject. The words "subject", "patient" and "individual" are used interchangeably throughout this disclosure.

Obtaining a voice recording from a word-reading test from the subject comprises obtaining a voice recording from a first word-reading test, and a voice recording from a second word-reading test, wherein the word-reading tests comprise reading a sequence of words drawn from a set of n words that are color words, wherein the words are displayed in a single color in the first word reading test, and in a color independently chosen from a set of m colors in the second word reading test, optionally wherein the sequence of words in the second word reading test is the same as the sequence of words in the first word reading test.

The sequence of words may comprise a predetermined number of words, the predetermined number being chosen to ensure that the recording contains sufficient information to estimate the one or more metrics and/or to enable comparison of the one or more metrics with previously obtained reference values. The sequence of words may comprise at least 20, at least 30 or approximately 40 words. For example, the present inventors have found a word reading test comprising a sequence of 40 words to provide sufficient information to estimate all of the metrics of interest while representing a manageable effort even for subjects with strong dyspnea and/or fatigue, such as decompensated heart failure patients.

The predetermined number of words may be dependent on the expected physiological and/or pathological state of the subject. For example, the predetermined number of words may be chosen such that a subject with a particular disease, disorder or condition can be expected to read the sequence of words within a predetermined length of time. The expected number of words per predetermined period of time may be determined using a comparative training cohort. Preferably, a comparative training cohort is made up of individuals that have a similar condition, disease or disorder to the intended user, and/or a similar level of fatigue and/or dyspnea to the intended user. The predetermined length of time is advantageously under 120 seconds. Tests that are too long may be influenced by external parameters such as boredom or physical weakness and/or may be less convenient for the user potentially leading to decreased uptake. A predetermined length of time may be chosen from: 30 seconds, 35 seconds, 40 seconds, 45 seconds, 50 seconds, 55 seconds, or 60 seconds. A predetermined length of time and/or number of words may be chosen based on the existence of a standard and/or comparative test.

Preferably, the recording is as long as needed for the subject to read the sequence of words that are displayed to them. Thus, the computing device may record a voice recording until the subject indicates that the recording is to be stopped and/or until the subject has read the entire sequence of words displayed. For example, the computing device may record a voice recording until the subject provides an input through a user interface, indicating that they have completed the test. As another example, the computing device may record a voice recording for a predetermined length of time, and the recording may be cropped to include a number of segments corresponding to the expected number of words in the sequence of words. Alternatively, the computing device may record a voice recording until it detects that the subject has not spoken a word for a predetermined length of time. In other words, the method may comprise causing the computing device associated with the subject to record a voice recording from when the computing device receives a start signal to when the computing device receives a stop signal. The start and/or stop signal may be received from the subject through a user interface. Alternatively, the start and/or stop signal may be automatically generated. For example, the start signal may be generated by the computing device starting to display words. The stop signal may be generated for example by the computing device determining that the no voice signal has been detected for a set minimum period of time such as e.g. 2, 5, 10 or 20 seconds. Without wishing to be bound by theory, it is believed that the use of voice recordings that are expected to contain a known number of words (corresponding to the number of words in the set of words) may be particularly advantageous in any aspect of the invention. Indeed, such embodiments may advantageously simplify the alignment step since the known sequence of words would then have a known length for any recording.

The recording may comprise a plurality of recordings. Each recording may be from a word reading test comprising reading a sequence of at least 20, at least 25 or at least 30 words. For example, a word reading test comprising reading a sequence of e.g. 40 words may be divided in two tests comprising reading a sequence of 20 words. This may enable a recording from a word reading test comprising reading a sequence of a predetermined length when the subject's pathological or physiological state does not enable them to read a sequence of said predetermined length in one test. In embodiments using multiple separate voice recordings, the steps of identifying segments corresponding to single words/syllables are advantageously performed at least in part separately for the separate voice recordings. For example, steps comprising normalisation, dynamic thresholding, scaling, etc. are advantageously performed separately for each recording. In embodiments using multiple separate voice recordings, the alignment step may be performed separately for each recording. By contrast, the clustering step may advantageously be performed on the combined data from the multiple recordings.

The steps of displaying a sequence of words for the word reading test, and recording the word recording may be performed by a computing device that is remote from a computing device that performs the analysis steps. For example, the step of displaying and recording may be performed by a user's personal computing device (which may be a PC or a mobile device such as a mobile phone or tablet), while the analysis of the voice recording may be performed by a remote computer, such as a server. This may enable the remote acquisition of the clinically relevant data e.g. at a patients' home while leveraging increased computing capabilities are a remote computer, for analysis.

In embodiments, the computing device associated with the subject is a mobile computing device, such as a mobile phone or tablet. In embodiments, the step of causing a computing device associated with the subject to display a sequence of words and to record a voice recording is performed through an application, which may be a software application that runs locally on the computing device associated with the subject (sometimes referred to as "mobile app" or "native app" in the context of mobile devices), a web application that runs in a web browser, or a hybrid application that embeds a mobile website inside a native app.

In embodiments, obtaining a voice recording comprises recording a voice recording and performing the steps of analysing the voice recording, wherein the obtaining and the analysing are performed by the same computing device (i.e. locally). This may advantageously remove the need for a connection to a remote device for analysis, and the need to transfer sensitive information. The results of the analysis (e.g. correct word rate, pitch, etc.) and the voice recording or a compressed version thereof may in such embodiments still be communicated to a remote computing device for storage and/or meta-analysis.

The method may be used to assess the status of a subject who has been diagnosed as having or is at risk of having a condition that affects breathing, vocal tone, fatigue and/or cognitive ability. The method may be used to diagnose a subject as having a condition that affects breathing, vocal tone, fatigue and/or cognitive ability. Within the context of the present invention, an individual may be considered to have a condition that affects breathing, vocal tone, fatigue and/or cognitive ability if the individual's performance of a task such as a word-reading test is affected by psychological, physiological, neurological, or respiratory factors. Examples of conditions, diseases or disorders that may affect a subject's breathing, vocal tone, fatigue state or cognitive ability include:
(i) cardiovascular diseases such as heart failure, coronary heart disease, myocardial infarction (heart attack), atrial fibrillation, arrhythmia (heart rhythm disorders), heart valve disease;
(ii) respiratory diseases, disorders or conditions such as obstructive lung disease (e.g. asthma, chronic bronchitis, bronchiectasis and chronic obstructive pulmonary disease (COPD)), chronic respiratory diseases (CRDs), respiratory tract infections, and lung tumours), respiratory infections (such as e.g. COVID-19, pneumonia, etc.), obesity, dyspnea (such as e.g. dyspnea associated with heart failure, panic attacks (anxiety disorders), pulmonary embolism, physical restriction or damage to the lungs (such as e.g. broken ribs, collapsed lungs, pulmonary fibrosis, etc.), pulmonary hypertension, or any other disease, disorder or condition that affects lung / cardiopulmonary function (for example as measurable by spiroergometry), etc.;
(iii) neurovascular diseases or disorders such as stroke, neurodegenerative diseases, myopathy, diabetic neuropathy, etc.;
(iv) psychiatric diseases or disorders such as depression, sleepiness, attention deficit disorder, chronic fatigue syndrome;
(v) conditions that affect an individual's fatigue state or cognitive ability through systemic mechanisms such as pain, abnormal glucose levels (such as e.g. due to diabetes mellitus), impairment of kidney function (such as e.g. in the context of chronic renal failure or renal replacement therapy), etc.

As such, the methods described herein may find uses in the diagnosing, monitoring or treatment of any of the above conditions, diseases or disorders.

Within the context of the present invention, a word-reading test (also referred to herein as a "word reading task") refers to a test that requires an individual to read a set of words (also referred to herein as a "sequence of words") that are not connected so as to form a sentence, wherein the words are drawn from a predetermined set (the words are drawn randomly or pseudo-randomly from a set). For example, all of the words in the set of words may be nouns, such as the words for a set of colors in a chosen language.

As the skilled person understands, the method of analysing a voice-recording from a subject is a computer-implemented method. Indeed, analysis of voice recordings as described herein, including e.g. syllable detection, classification and alignment as described require the analysis of large amounts of data through complex mathematical operations that are beyond the reach of mental activity.

According to a second aspect, there is provided a computer-implemented method of monitoring a subject with heart failure, or diagnosing a subject as having worsening of heart failure or decompensated heart failure, the method comprising: assessing the pathological and/or physiological state of the subject using the method of the first aspect, wherein the one or more respective reference values are values for the same metrics previously obtained for the same subject or wherein the one or more respective reference values comprise values for the same metrics associated with decompensated heart failure patients, stable heart failure patients and/or recovering decompensated heart failure patients.According to a third aspect not part of the claims, there is provided a method of treating a subject with worsening of heart failure or decompensated heart failure, the method comprising diagnosing the subject as having worsening of heart failure or decompensated heart failure using the method of the preceding aspect; and treating the subject for the heart failure. The method may further comprise monitoring progression of the disease, monitoring the subject's treatment and/or recovery using the methods of any preceding aspect. The method may comprise monitoring the subject at a first and further time point and increasing or otherwise modifying the treatment if comparing the value of the one or more metrics associated with the first and further time points indicate that the subject's heart failure status has not improved. The method may comprise monitoring the subject at a first and further time point and maintaining or reducing the treatment if comparing the value of the one or more metrics associated with the first and further time points indicate that the subject's heart failure status has improved.

According to a fourth aspect, there is provided a method of monitoring a subject that has been diagnosed as having or being at risk of having a condition associated with dyspnea and/or fatigue, the method comprising: assessing the pathological and/or physiological state of the subject using the method of the first aspect, wherein the one or more respective reference values are values for the same metrics previously obtained for the same subject or wherein the one or more respective reference values comprise values for the same metrics associated with patients having the condition and/or values for the same metrics associated with patients that do not have the condition.

According to a fifth aspect, there is provided a method of assessing the level of dyspnea and/or fatigue in a subject, the method comprising assessing the pathological and/or physiological state of the subject using the method of the first aspect, wherein the one or more respective reference values are values for the same metrics previously obtained for the same subject or wherein the one or more respective reference values comprise values for the same metrics associated with patients having the condition and/or values for the same metrics associated with patients that do not have the condition.

According to a sixth aspect not part of the claims, there is provided a method of treating a subject that has been diagnosed as having or being at risk of having a condition associated with dyspnea and/or fatigue, the method comprising assessing the level of dyspnea and/or fatigue in a subject using the methods of the preceding aspects, and treating the subject for the condition or adjusting the subject's treatment for the condition depending on the results of the assessment. The method may comprise performing the assessment at a first and further time point and increasing or otherwise modifying the treatment if comparing the value of the one or more metrics associated with the first and further time points indicate that the subject's level of fatigue and/or dyspnea has increased or not improved. The method may comprise performing the assessment at a first and further time point and maintining or reducing the treatment if comparing the value of the one or more metrics associated with the first and further time points indicate that the subject's level of fatigue and/or dyspnea has improved or not increased. The method may have any of the features described in relation to the first aspect.

According to a seventh aspect not part of the claims, there is provided a method of diagnosing a subject as having a respiratory infection, or treating a patient that has been diagnosed with a respiratory infection, such as COVID-19, the method comprising: obtaining a voice recording from a word-reading test from the subject, wherein the voice recording is from a word-reading test comprising reading a sequence of words drawn from a set of n words; and analysing the voice recording, or a portion thereof, by: identifying a plurality of segments of the voice recording that correspond to single words or syllables; determining the value of one or more metrics selected from the breathing %, unvoicing/voicing ratio, voice pitch and correct word rate at least in part based on the identified segments; wherein the one or more metrics comprise at least the voice pitch; and comparing the value of the one or more metrics with one or more respective reference values. The method may further comprises any of the features of the first aspect.

The method may comprise treating the subject for the respiratory infection if the comparison indicates that the subject has a respiratory infection. The method may further comprise monitoring the subject's treatment and/or recovery using the methods of any preceding aspect. The method may comprise monitoring the subject at a first and further time point and increasing or otherwise modifying the treatment if comparing the value of the one or more metrics associated with the first and further time points indicate that the subject's respiratory infection has not improved. The method may comprise monitoring the subject at a first and further time point and maintaining or reducing the treatment if comparing the value of the one or more metrics associated with the first and further time points indicate that the subject's respiratory infection has improved.

According to an eight aspect, there is provided a system comprising: at least one processor; and at least one non-transitory computer readable medium containing instructions that, when executed by the at least one processor, cause the at least one processor to perform operations comprising the steps of any embodiment of the claimed methods .

One or more computer readable media are described storing instructions that, when executed by at least one processor, cause the at least one processor to perform operations comprising the steps of any embodiment of the methods of any preceding aspect

A computer program product is described comprising instructions that, when executed by at least one processor, cause the at least one processor to perform operations comprising the steps of any embodiment of the methods of any preceding aspect.

### Brief Description of the Figures

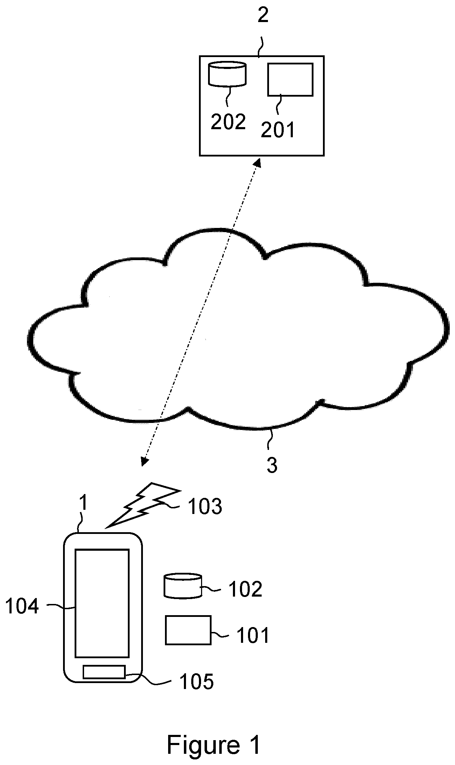

**Figure 1** shows an exemplary computing system in which embodiments of the present invention may be used.

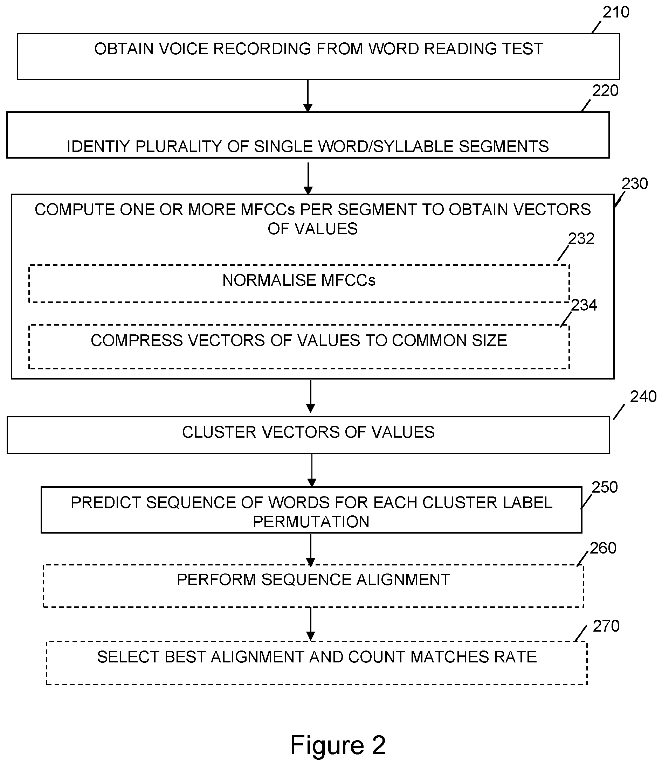

**Figure 2** is a flow chart illustrating a method of assessing the physiological and/or pathological state of a subject by determining the correct word rate from a word reading test.

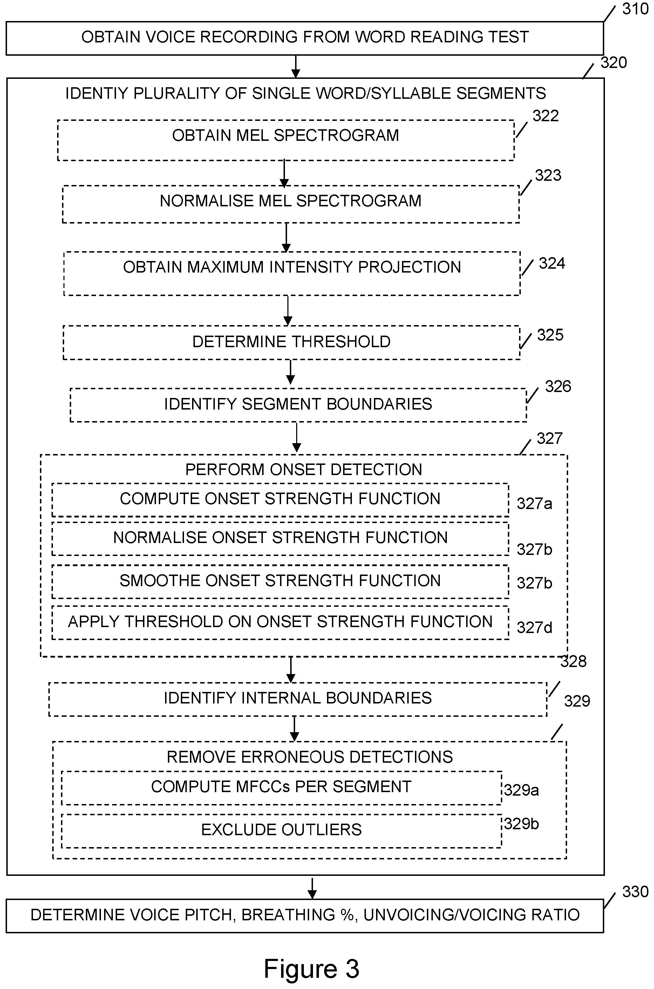

**Figure 3** is a flow chart illustrating a method of assessing the physiological and/or pathological state of a subject by determining the voice pitch, breathing % and/or unvoicing/voicing ratio from a word reading test.

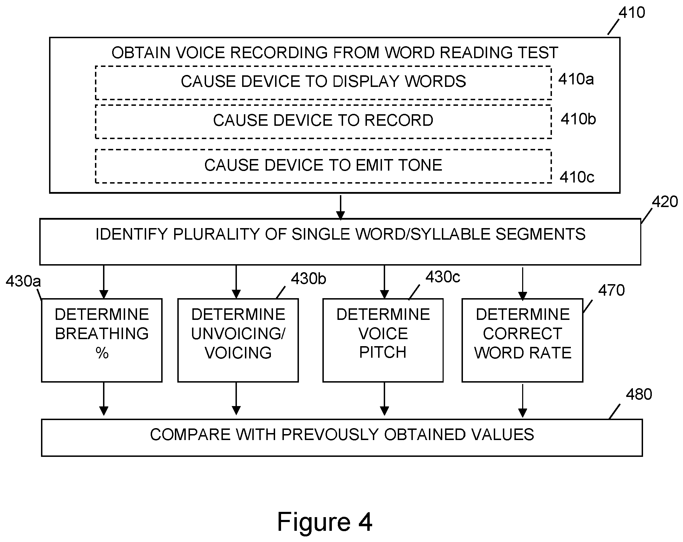

**Figure 4** illustrates schematically a method of diagnosing, prognosing or monitoring a subject.

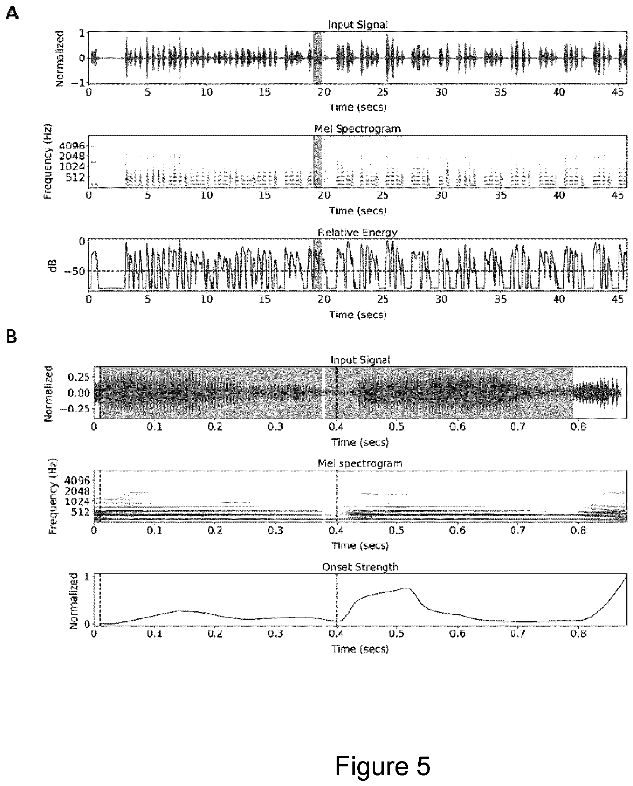

**Figures 5A and 5B** illustrate a two-step approach for identifying word boundaries according to an exemplary embodiment. (A) Coarse word boundaries were identified on the relative energy measure. A Mel-frequency spectrogram of the input audio input was constructed and the maximum intensity projection of the Mel spectrogram along frequency axis gave rise to the relative energy (B) One coarsely segmented word (highlighted in grey) was divided into two putative words based on the onset strength.

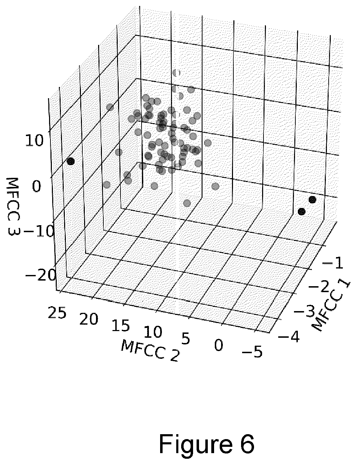

**Figure 6** illustrates an outlier removal approach according to an exemplary embodiment. All segmented words were parameterized using the first three MFCC (Mel-frequency cepstral coefficients), where inliers (putative words, n = 75) shown in grey and outliers (non-speech sounds, n = 3) in black were illustrated in a 3-D scatter plot.

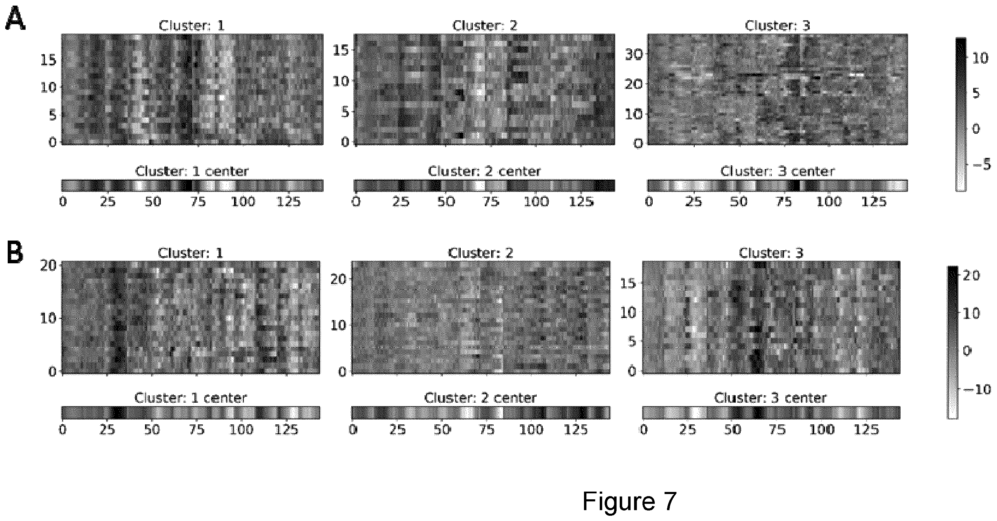

**Figures 7A and 7B** illustrate a clustering approach to identify words according to an exemplary embodiment. Putative words from one recording (where 3 different words were shown in the word reading test) were grouped into three different clusters by applying K-means clustering. Visual appearance of words in three distinctive clusters was shown in the upper graphs (one word per row) and the corresponding cluster centers were shown in the lower graphs. In particular, (A) represents 3 word-clusters from one test spoken in English (words = 75) and (B) represents 3 word-clusters from another test spoken in German (words = 64).

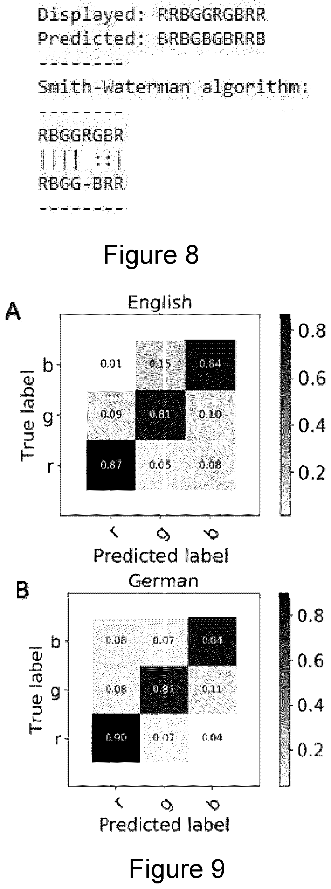

**Figure 8** illustrates a word sequence alignment approach according to an exemplary embodiment. In particular, the application of the Smith-Waterman algorithm on a 10 words sequence is shown. Alignment of displayed sequence RRBGGRGBRR and predicted sequence BRBGBGBRRB found the partially overlapping sequence and resulted in 5 correct words: matches (|), gaps (-), and mismatches (:).
**Figures 9A and 9B** show the classification accuracy of the model-free word recognition algorithm according to an exemplary embodiment. Classification accuracy of each word displayed as a normalized confusion matrix (row sum = 1). Rows represent true labels from manual annotations and columns represent predicted labels from the automated algorithm. The correct predictions are on the diagonal with a black background and the incorrect predictions are with a grey background. (A) English words: /r/ for /red/ (n = 582), /g/ for /green/ (n = 581), and /b/ for /blue/ (n = 553). (B) German words: /r/ for/rot/ (n = 460), /g/ for /grün/ (n = 459), and /b/ for /blau/ (n = 429).

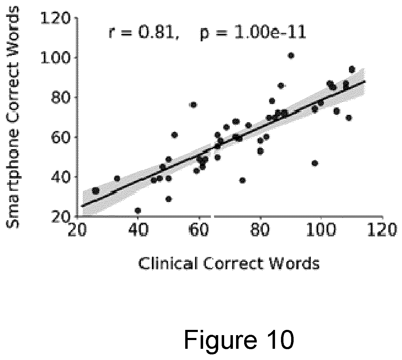

**Figure 10** shows scatter plot comparison between the clinical Stroop word score obtained for a set of patients with Huntington Disease using the UHDRS-, and automated assessment measures according to an exemplary embodiment. A linear relationship between the variables was determined through regression. The resulting regression line (black line) and a 95% confidence interval (grey shaded area) were plotted. Pearson's correlation coefficient r and the significance level of p-value are shown on the graph.

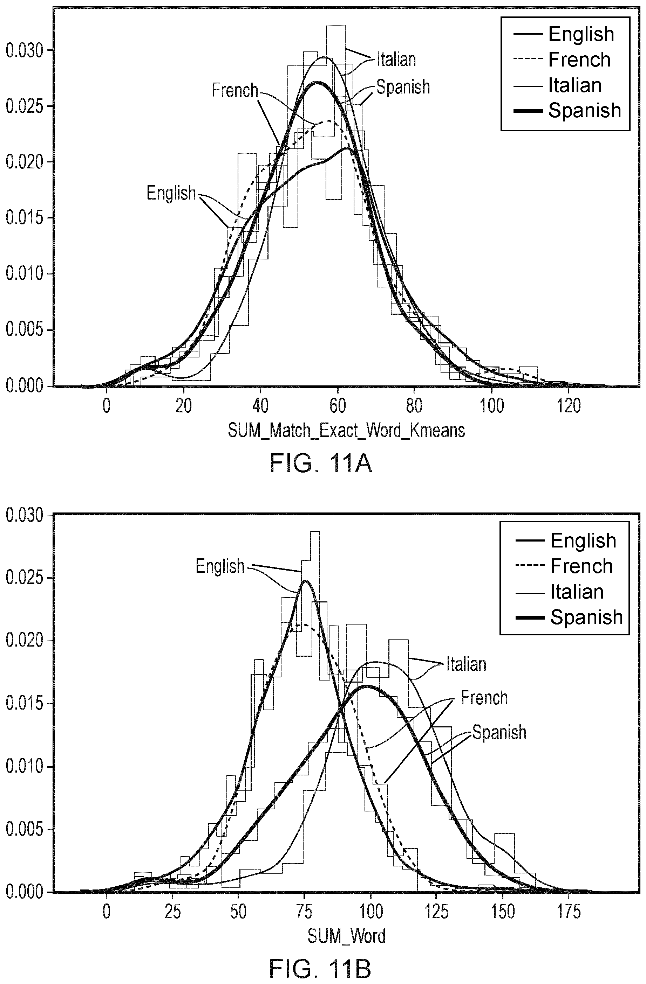

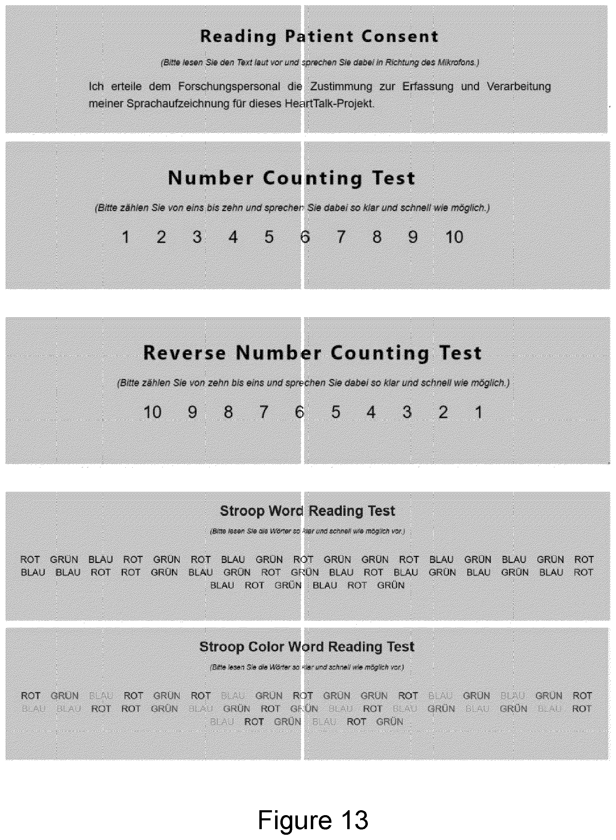

**Figures 11A and 11B** show the distribution of number of correctly read words (A) and the number of single words/syllable segments (B) identified in sets of recordings in English. French, Italian and Spanish. The data shows that the number of correctly read words identified according to the method described herein is robust to variations in the length of the words (Figure 13A), even though multiple syllables in single words are identified as separate entities (Figure 13B).

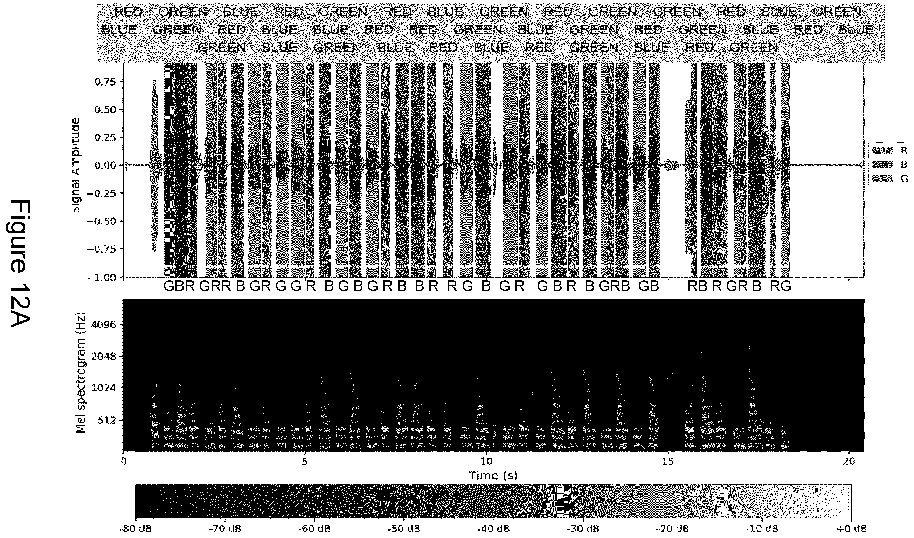

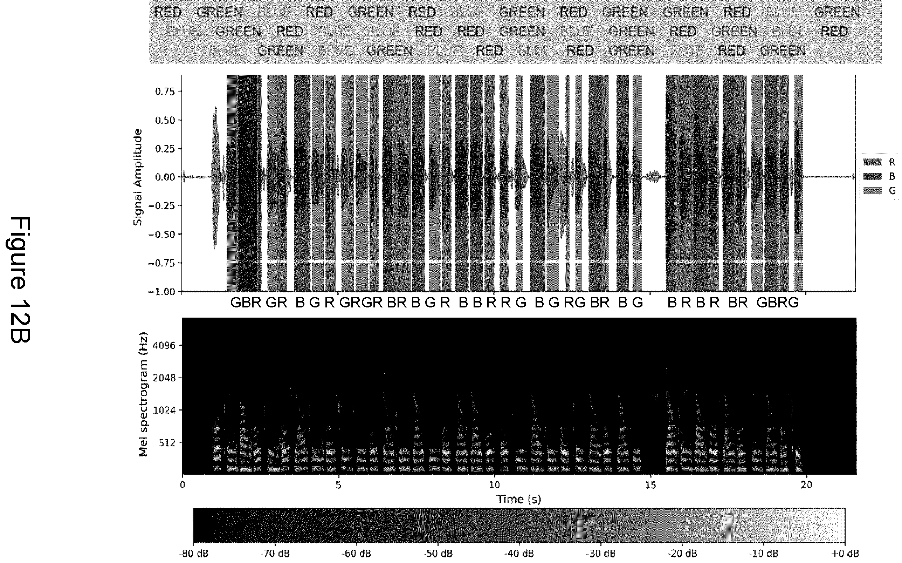

**Figures 12A** **and** **12B** show the results of matched Stroop word reading (A, consistent condition) and Stroop color words reading (B, interference condition) test from a healthy individual, analysed as described herein. Each subfigure shows the set of words displayed in each test (top panel), the normalised signal amplitude for the respective recording (middle panel), with overlaid segment identification and word prediction (illustrated as the color of each segment), and the Mel spectrogram and accompanying scale (bottom panel) for the signal shown in the middle panel. The data shows that the segment identification and correct word counting processes performs equally well for both the consistent condition and the interference condition.
**Figure 13** shows a screenshot of a web-based word reading application according to an exemplary embodiment. Participants were asked to record themselves performing 5 different reading tasks: (i) reading a fixed predetermined passage of text (a patient consent statement) - this is also referred to herein as "reading task"; (ii) reading a set of increasing consecutive numbers - this is also referred to herein as "counting task"; (iii) reading a set of decreasing consecutive numbers - this is also referred to herein as "reverse counting task"; (iv) Stroop word reading test (consistent part) - reading a randomly drawn set (fixed number) of color words displayed in black; and (v) Stroop color word reading test (interference part) - reading a randomly drawn set (fixed number) of color words displayed in randomly drawn colors.

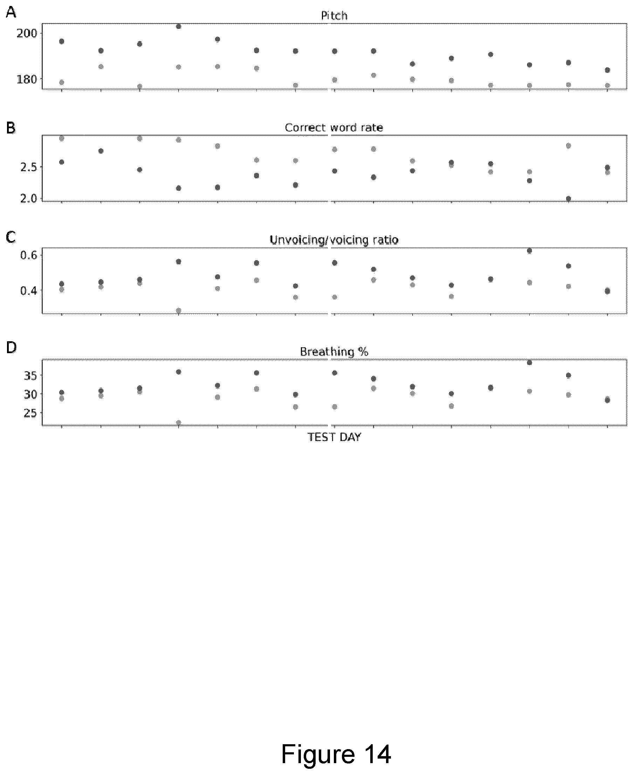

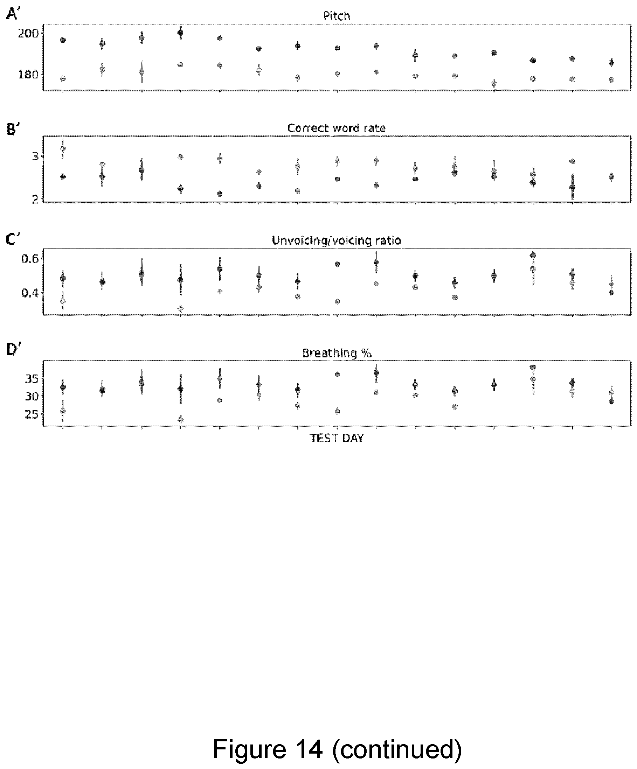

**Figures 14A-14D** show the results of analysis of voice recordings from Stroop reading tests performed by a healthy individual at rest (light grey data series) or after moderate exercise (climbing four flights of stairs - dark grey data series), analyzed as described herein. Each subfigure shows the results in terms of one of the biomarker metrics described herein. Each pair of points with the same 'TEST DAY' (x axis) shows results for the same individual on the same day, at rest and after exercise (results for the same tests are shown across subfigures at the same 'TEST DAY', n = 15 days). (A) Pitch - estimated average pitch over all voicing segments of a Stroop color word reading test (interference condition) recording (Hz), Cohen's d=2.75. (B) Correct word rate (number of correct words per second in a Stroop color word reading test recording), Cohen's d=-1.57. (C) Unvoicing/voicing ratio (unitless - sum of time between voicing segments relative to sum of time from voicing segments, in a Stroop color word reading test recording), Cohen's d=1.44. (D) Breathing % (% - sum of time between voicing segments relative to sum of time between and within voicing segments, in a Stroop color word reading test recording), Cohen's d=1.43. (A')-(D') show the same metrics as (A)-(D) but obtained using the combined results from the Stroop color word reading test recordings for which data is shown in (A)-(D) and a Stroop word reading test recording from the same test session. (A') Pitch - combined tests, Cohen's d=3.47. (B') Correct word rate - combined tests, Cohen's d= - 2.26. (C') Unvoicing/voicing ratio - combined tests, Cohen's d=1.25. (D') Breathing % - combined tests, Cohen's d=1.26.

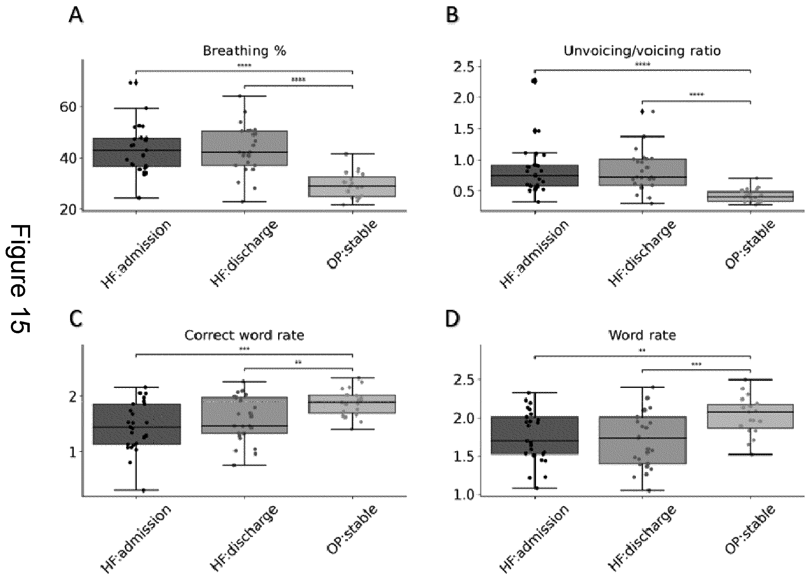

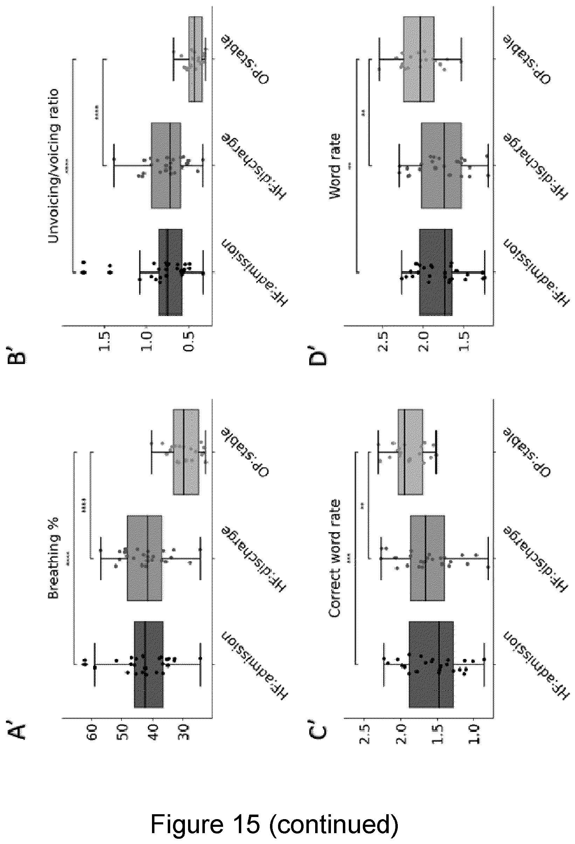

**Figures 15A-J** show the results of analysis of voice recordings from Stroop reading tests (A-D, interference condition; A'-D' combined interference and consistent condition), a reading task (E-G), and a number counting task (H-J, reverse number counting; H'-J', combined forward and reverse counting), in three heart failure patient groups: decompensated heart failure patients on admission to hospital (labelled "HF:admission", n=25), the same decompensated heart failure patients on discharge from hospital (labelled "HF:discharge", n=25), and stable outpatients (labelled "OP:stable", n=19). (A) Boxplots of breathing % (%, calculated as 100*(unvoicing/(unvoicing+voicing))), overlaid on patient data. The breathing % in the word reading test (word color reading test, interference condition) was significantly different between each of the two groups of decompensated HF patients and the stable patients (HF:admission vs OP:stable: Cohen's d=1.75, permutation test p-value=0.0000; HF:discharge vs OP:stable: Cohen's d=1.77, permutation test p-value=0.0000). (B) Boxplots of unvoicing/voicing ratio (unitless, calculated as unvoicing/voicing), overlaid on patient data. The unvoicing/voicing ratio in the word reading test (word color reading test, interference condition) was significantly different between each of the two groups of decompensated HF patients and the stable patients (HF:admission vs OP:stable: Cohen's d=1.31, permutation test p-value=0.0000; HF:discharge vs OP:stable: Cohen's d=1.52, permutation test p-value=0.0000). (C) Boxplots of correct word rate (number of correct words per second), overlaid on patient data. The correct word rate in the word reading test (word color reading test, interference condition) was significantly different between each of the two groups of decompensated HF patients and the stable patients (HF:admission vs OP:stable: Cohen's d=-1.14, permutation test p-value=0.0001; HF:discharge vs OP:stable: Cohen's d=-0.87, permutation test p-value=0.0035). (D) Boxplots of speech rate (number of words per second), overlaid on patient data. The speech rate in the word reading test (word color reading test, interference condition) was significantly different between each of the two groups of decompensated HF patients and the stable patients (HF:admission vs OP:stable: Cohen's d=-0.89, permutation test p-value=0.0019; HF:discharge vs OP:stable: Cohen's d=-0.98, permutation test p-value=0.0011). (A') Boxplots of breathing %, overlaid on patient data. The breathing % in the word reading test (word color reading test, combined interference and coherent condition) was significantly different between each of the two groups of decompensated HF patients and the stable patients (HF:admission vs OP:stable: Cohen's d=1.71, permutation test p-value=0.0000; HF:discharge vs OP:stable: Cohen's d=1.85, permutation test p-value=0.0000). (B') Boxplots of unvoicing/voicing ratio, overlaid on patient data. The unvoicing/voicing ratio in the word reading test (word color reading test, combined interference and coherent condition) was significantly different between each of the two groups of decompensated HF patients and the stable patients (HF:admission vs OP:stable: Cohen's d=1.41, permutation test p-value=0.0000; HF:discharge vs OP:stable: Cohen's d=1.71, permutation test p-value=0.0000). (C') Boxplots of correct word rate, overlaid on patient data. The correct word rate in the word reading test (word color reading test, combined interference and coherent condition) was significantly different between each of the two groups of decompensated HF patients and the stable patients (HF:admission vs OP:stable: Cohen's d=-1.09, permutation test p-value=0.0002; HF:discharge vs OP:stable: Cohen's d=-0.81, permutation test p-value=0.0053). (D') Boxplots of speech rate (number of words per second), overlaid on patient data. The speech rate in the word reading test (word color reading test, combined interference and coherent condition) was significantly different between each of the two groups of decompensated HF patients and the stable patients (HF:admission vs OP:stable: Cohen's d=-0.92, permutation test p-value=0.0019; HF:discharge vs OP:stable: Cohen's d=-0.95, permutation test p-value=0.0013). (E) Boxplots of breathing % (%), overlaid on patient data. The breathing % in the reading task was significantly different between each of the two groups of decompensated HF patients and the stable patients (HF:admission vs OP:stable: Cohen's d=1.54, permutation test p-value=0.0000; HF:discharge vs OP:stable: Cohen's d=1.28, permutation test p-value=0.0000). (F) Boxplots of unvoicing/voicing ratio (unitless), overlaid on patient data. The unvoicing/voicing ratio in the reading task was significantly different between each of the two groups of decompensated HF patients and the stable patients (HF:admission vs OP:stable: Cohen's d=1.35, permutation test p-value=0.0000; HF:discharge vs OP:stable: Cohen's d=0.89, permutation test p-value=0.0002). (G) Boxplots of speech rate (number of words per second), overlaid on patient data. The speech rate in the reading task was significantly different between each of the two groups of decompensated HF patients and the stable patients (HF:admission vs OP:stable: Cohen's d=-1.60, permutation test p-value=0.0000; HF:discharge vs OP:stable: Cohen's d=-0.64, permutation test p-value=0.0190). (H) Boxplots of breathing % (%), overlaid on patient data. The breathing % in the reverse counting task was not significantly different between the groups of decompensated HF patients and the stable patients (HF:admission vs OP:stable: Cohen's d=-0.24, permutation test p-value=0.2251; HF:discharge vs OP:stable: Cohen's d=-0.21, permutation test p-value=0.2537). (I) Boxplots of unvoicing/voicing ratio (unitless), overlaid on patient data. The unvoicing/voicing ratio in the reverse counting task was not significantly different between the two groups of decompensated HF patients and the stable patients (HF:admission vs OP:stable: Cohen's d=-0.19, permutation test p-value=0.2718; HF:discharge vs OP:stable: Cohen's d=-0.26, permutation test p-value=0.2126). (J) Boxplots of speech rate (number of words per second), overlaid on patient data. The speech rate in the reverse counting task was not significantly different between the two groups of decompensated HF patients and the stable patients (HF:admission vs OP:stable: Cohen's d=0.19, permutation test p-value=0.2754; HF:discharge vs OP:stable: Cohen's d=0.22, permutation test p-value=0.2349). (H') Boxplots of breathing % (%), overlaid on patient data. The breathing % in the combined counting tasks was not significantly different between at least one of the groups of decompensated HF patients and the stable patients. (I') Boxplots of unvoicing/voicing ratio (unitless), overlaid on patient data. The unvoicing/voicing ratio in the combined counting tasks was not significantly different between at least one of the two groups of decompensated HF patients and the stable patients. (J') Boxplots of speech rate (number of words per second), overlaid on patient data. The speech rate in the combined counting tasks was not significantly different between the two groups of decompensated HF patients and the stable patients. *p value (permutation test) < 0.05, **p value (permutation test) < 0.01, ***p value (permutation test) < 0.001, ****p value (permutation test) < 0.0001 ns=not significant (>0.05). All permutation tests were performed using 10000 permutations.

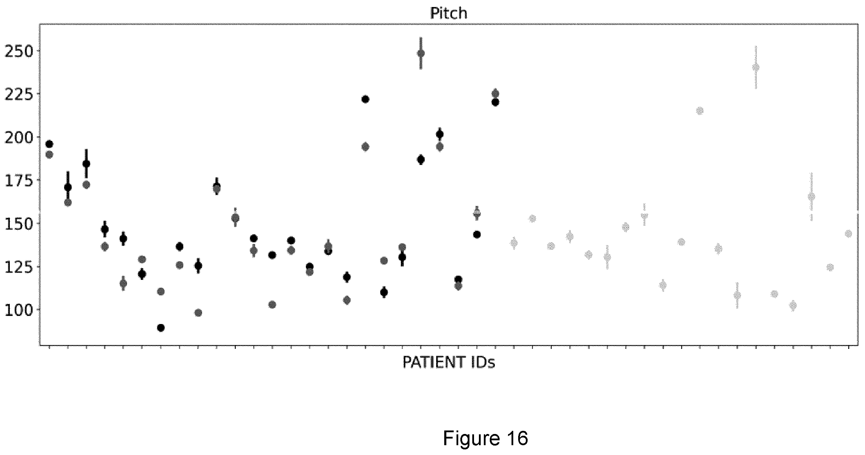

**Figure 16** shows the results of analysis of voice recordings from Stroop reading tests, in terms of mean pitch (point) and standard deviation (error bar), in three heart failure patient groups: decompensated heart failure patients on admission to hospital (black data series, n=25), the same decompensated heart failure patients on discharge from hospital (dark grey data series, n=25) - data series on the left hand of the plot, two points per patient (at admission and at discharge), and stable outpatients (light grey data series, n=19 - data series on the right hand of the plot). The error bar indicates the standard deviation between normal condition and interference condition.

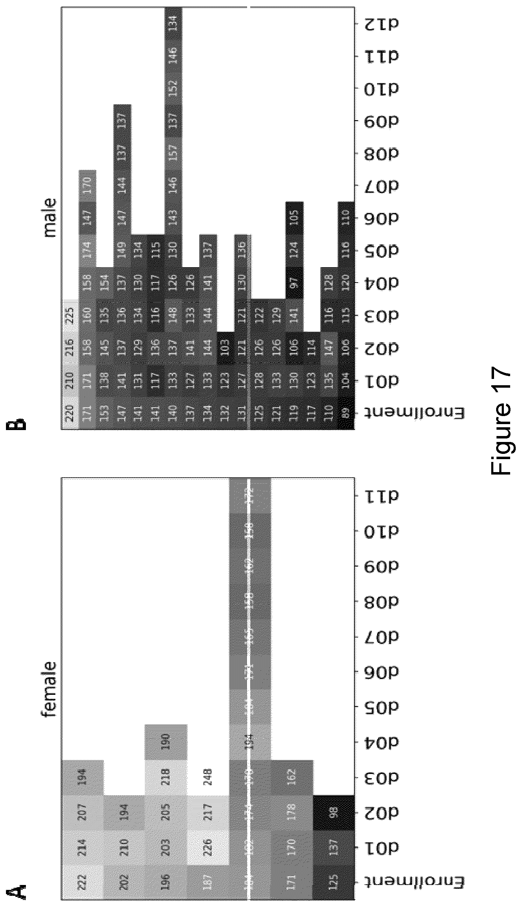

**Figure 17** shows the results of analysis of voice recordings from Stroop reading tests, in terms of mean pitch, in selected decompensated heart failure patients from hospital admission (labelled as "enrollment" to discharge (last data point for every respective patient). A. female patients (n = 7). B. male patients (n=17).

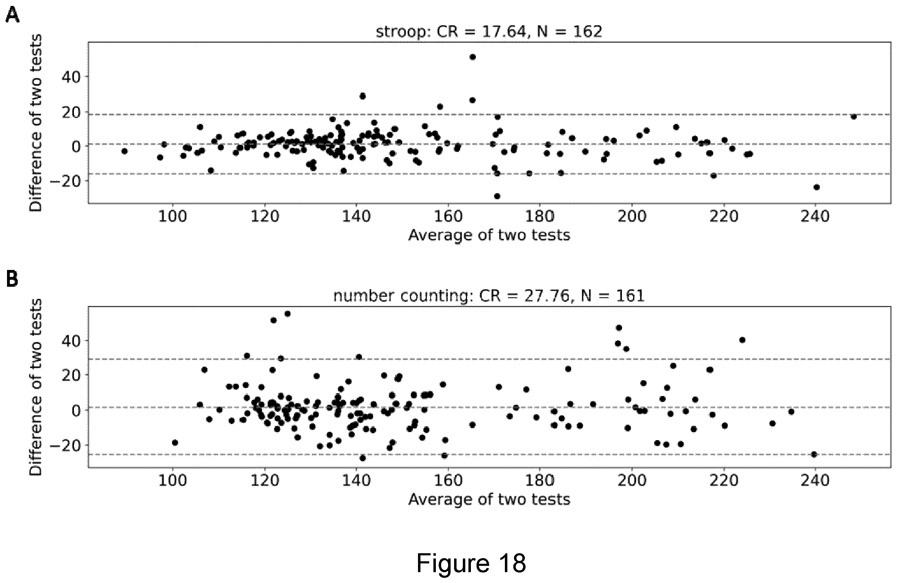

**Figure 18** shows Bland-Altman plots that assess the level of agreement of pitch measurements between a Stroop word reading test and a Stroop color reading test for 48 heart failure patients (A, total of 162 pairs of recordings analysed), and between a number counting test and a reverse number counting test for 48 heart failure patients (B, total of 161 pairs of recordings analysed). Each data point shows the difference between the average pitch (Hz) estimated using the respective tests. The dashed lines show the average difference (middle line) and the ±1.96 standard deviations (SD) interval. The repeatability is quantified using the consensus report (CR=2*SD), and is 27.76 for the number counting tests and 17.64 for the word reading tests.

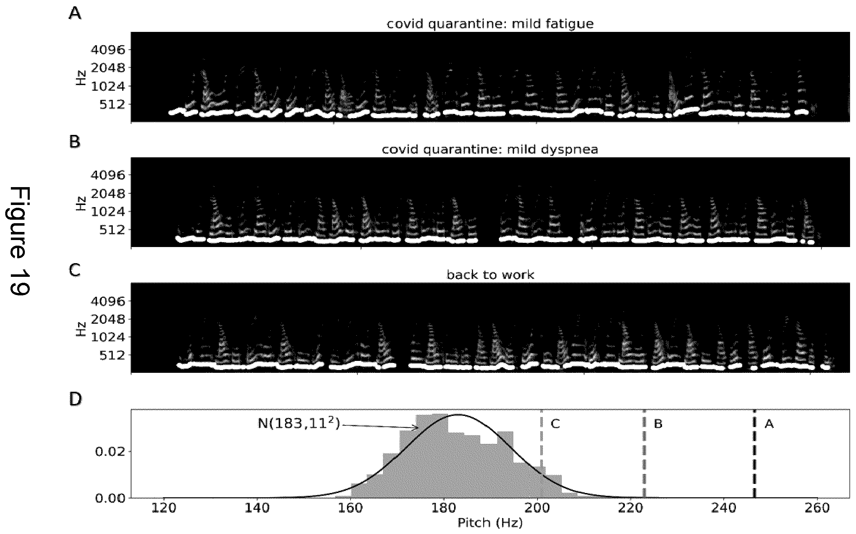

**Figure 19** shows the results (estimated voice pitch) of analysis of voice recordings from Stroop reading tests (interference condition) by the same subject during COVID-19 quarantine (A, B) and the day back to work (C). (A-C) shows the pitch contour (white dots) overlaid with Mel-spectrogram. (D) shows the data for the subject diagnosed with COVID-19, on the days in the quarantine with self-reported mild fatigue symptom (vertical lines - estimated pitch=247 Hz as shown in A) and mild dyspnea symptom (vertical lines - estimated pitch=223 Hz as shown in B) and no symptom on the day back to work (vertical lines - estimated pitch=201 Hz as shown in C), on a histogram showing the data for 10 healthy female volunteers (n = 1026 voice samples) and estimated normal distribution probability density function (mean = 183, sd = 11; estimated by fitting these 1026 samples using the fit function from scipy.stats.norm).

Where the figures laid out herein illustrate embodiments of the present invention, these should not be construed as limiting to the scope of the invention. Where appropriate, like reference numerals will be used in different figures to relate to the same structural features of the illustrated embodiments.

### Detailed Description

Specific embodiments of the invention will be described below with reference to the Figures.

**Figure 1** shows an exemplary computing system in which embodiments of the present invention may be used.

A user (not shown) is provided with a first computing device - typically a mobile computing device such as a mobile phone 1 or tablet. Alternatively, the computing device 1 may be fixed, such as e.g. a PC. The computing device 1 has at least one processor 101 and at least one memory 102 together providing at least one execution environment. Typically, a mobile device has firmware and applications run in at least one regular execution environment (REE) with an operating system such as iOS, Android or Windows. The computing device 1 may also be equipped with means 103 to communicate with other elements of computing infrastructure, for example via the public internet 3. These may comprise a wireless telecommunications apparatus for communication with a wireless telecommunications network and local wireless communication apparatus to communicate with the public internet 3 using e.g. Wi-Fi technology.

The computing device 1 comprises a user interface 104 which typically includes a display. The display 104 may be a touch screen. Other types of user interfaces may be provided, such as e.g. a speaker, keyboard, one or more buttons (not shown), etc. Further, the computing device 1 may be equipped with sound capture means, such as a microphone 105.

A second computing device 2 is also shown in Figure 1. The second computing device 2 may for example form part of an analysis provider computing system. The second computing device 2 typically comprises one or more processors 201 (e.g. servers), a plurality of switches (not shown), and one or more databases 202, and is not described further here as the details of the second computing device 2 used are not necessary for understanding how embodiments of the invention function and may be implemented. The first computing device 1 can be connected to the analysis provider computing device 2 by a network connection, such as via the public internet 3.

**Figure 2** is a flow chart illustrating a method of assessing the physiological and/or pathological state of a subject by determining the correct word rate from a word reading test. The method comprises obtaining 210 a voice recording from a word-reading test from the subject. The voice recording is from a word-reading test comprising reading a sequence of words drawn from a (closed) set of n words.

In embodiments, the words are color words. In some such embodiments, the words are displayed in a single color in the word reading test. In such a set-up, the total number of correctly read words over a predetermined period of time may match the Stroop word count from the first part (in the "consistent condition") of a three-part Stroop test. In embodiments, the words are color words displayed in a color that is not necessarily consistent with the meaning of the single words. For example, the words may be drawn randomly or pseudo-randomly from a set of color words, and may each be displayed in a color that is randomly or pseudo-randomly drawn from a set of colors. In embodiments, the words are color words displayed in a color that is inconsistent (or not necessarily consistent, i.e. chosen independently from) with the meaning of the single words. For example, the words may be drawn randomly or pseudo-randomly from a set of color words, and may each be displayed in a color that is randomly or pseudo-randomly drawn from a set of colors that excludes the color that matches the color word to be displayed. The colors in the set of colors for display may be identical or different from the colors in the set of color words. In such embodiments, the total number of correctly read words over a predetermined period of time may match the Stroop word count from the third part ("inconsistent condition") of a three-part Stroop test. In embodiments, the voice recording comprises a first recording from a word-reading test comprising reading a sequence of words drawn from a (closed) set of n words, wherein the words are color words displayed in a single color, and a second recording from a word-reading test comprising reading a sequence of words drawn from a (closed) set of n words, wherein the words are color words displayed in a color that is not necessarily consistent with the meaning of the single words (e.g. chosen independently from the meaning of the single words).The sequences of words used in the first and the second recording may be identical. As such, the words for the first word-reading test and the second-word reading test may be drawn once from a set of n words. This advantageously increases the amount of information available to identify segments and clusters (see below), and provides two recordings that can be used to measure one or more biomarkers that can subsequently be compared between the two recordings (for example in order to evaluate the stability of the measurement and/or to investigate effects that are more likely to impact one or more of the measurements for the first vs the second word-reading tests.

In embodiments, n is between 2 and 10, preferably between 2 and 5, such as 3. The number n of different words in the sequence of words is preferably at least 2 because otherwise no reading is required after the subject has read the first word. The number n of different words from which the set of words is produced is preferably 10 or under 10 because otherwise the number of time that each word is expected to appear in a voice recording may be so low as to negatively impact the accuracy of the clustering process (see below). Preferably, the number n of different words is chosen such that the number of times that each word is expected to appear in a set of words to be read by a subject is at least 10. As the skilled person understands, this may depend at least on the length of the set of words and on the expected length of recording that the subject is expected to be able to undertake considering their state (such as e.g. their level of fatigue and/or breathlessness). A suitable choice for the number n of different words and the length of the set of words may for example be obtained using a comparable training cohort.

The n words may be color words, such as e.g. the words for the colors "red", "green" and "blue" (i.e. in English: ['RED', 'GREEN', 'BLUE'], in German: ['ROT', 'GRUN', 'BLAU'],in Spanish: ['ROJO', 'VERDE', 'AZUL'], in French: ['ROUGE', 'VERT', 'BLEU'], in Danish: ['RØD', 'GRØN', 'BLÅ'], in Polish: ['CZERWONY','ZIELONY', 'NIEBIESKI'], in Russian: [' 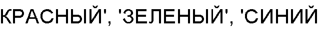 '], in Japanese: [' 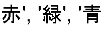 '], in Italian: ['ROSSO', 'VERDE', 'BLU'], in Dutch ['ROOD', 'GROEN', 'BLAUW'], etc). Color words are commonly used in the word reading part of a Stroop reading test. The words for the colors "red", "green" and "blue" are common choices for this test and as such may advantageously enable the results of the test to be compared to or integrated with existing implementations of the Stroop test in a clinical context.

In embodiments, the n words are chosen to each include a single vowel. In embodiments, the n words are chosen to include one or more vowels that are internal to the respective word. In embodiments, the words include a single emphasized syllable.

In preferred embodiments of any aspect, the words are monosyllabic words or disyllabic words. It may further be advantageous for all of the words to have the same number of syllables. For example, it may be advantageous for all words to be either monosyllabic or disyllabic. Embodiments using only monosyllabic words may be particularly advantageous because in such embodiments each segment corresponds to a single word. Such embodiments therefore advantageously result in counts of the number of segments that correspond to the number of words read and/or in the timing of segment being directly usable to obtain a speech rate (or any other feature associated with the rhythm of the speech). Further, the n words being monosyllabic may improve the accuracy of the clustering, as a single vector of values is expected for each word, resulting in n clusters that are expected to be relatively homogeneous. The use of monosyllabic words may also improve the accuracy of speech rate determination as it removes any potential problems that may be associated with identifying syllables that belong to the same word.

Embodiments using only disyllabic words may advantageously result in counts of the number of segments that can be related to the number of words read (and hence the speech rate / correct word rate) in a straightforward manner, and/or that can be compared across voice recordings from word-reading tests with the same characteristics.

In some embodiments using disyllabic words, the method may further comprise excluding segments that correspond to a specified one of two syllables in a word, prior to counting the number of segments identified in the voice recording and/or prior to determining the number of correctly read words in the voice recording. Segments that correspond to one of two syllables in a word may be identified based on the relative timing of two consecutive segments. For example, segments that closely follow each other such as e.g. segments that add up to less than a specific time (e.g. 400 ms), and/or that are separated by less than a specific time (e.g. 10 ms) may be assumed to belong to the same word. A specified segment to be excluded may further be identified as the first or second segment of two segments assumed to belong to the same word. Alternatively, a specified segment to be excluded may be identified based on the characteristics of the sound signal in the two segments. For example, the segment with lowest energy may be excluded. As another alternative, a specified segment to be excluded may be identified based on the relative length of the two segments. For example, the segment with shortest length may be excluded. Alternatively, the method may comprise merging segments that correspond to a specified one of two syllables in a word with a segment that closely follows or precedes it, such as e.g. segments that are within a specified time (e.g. 10 ms) of each other. Without wishing to be bound by any particular theory, it is believed that merging segments corresponding to syllables of the same word may be particularly difficult when analysing fast speech. As such, merging segments that are within a specified time of each other is believed to be particularly suitable for speech that has a speed similar to free speech or lower. In embodiments where the speech is expected to be relatively fast, it may be advantageous to use segments that are assumed to correspond to single syllables directly, rather than merging or excluding segments.

In embodiments using disyllabic words (or multi-syllabic words in general), the disyllabic words preferably have one emphasized syllable. Without wishing to be bound by theory, it is believed that the clustering step (see below) may have increased robustness to the presence of "noise" coming from segments corresponding to syllables rather than words when one of the syllable is emphasized. Indeed, in such case the signal from a non-emphasized syllable may be considered as noise in the clustering process, which will still produce clusters that are homogeneous in terms of the identity of the emphasized syllables assigned to each cluster.

In embodiments, the sequence of words comprises at least 20, at least 30, at least 40, at least 50 or about 60 words. In embodiments, the set of words are drawn at random from a set of n words. In embodiments, the method comprises drawing a set of words randomly from a set of n words and causing a computing device associated with the subject to display the set of words. In embodiments, the set of words are displayed in groups of m words on a line, where m can be e.g. 4. The display of 4 words per line was found herein to be convenient in the context of display on a typical smartphone screen. As the skilled person understand, the number of words (m) that is displayed as a group may be adjusted depending on the size of the screen/window on/in which the words are displayed and/or depending on preferences of the user (such as e.g. preferred font size). Such an adjustment may be automatic, for example through automatic detection of the screen or window size. Preferably, the groups of m words are displayed concomitantly. For example, all of the words in a line of e.g. 4 words are preferably displayed at the same time. This may reduce the risk that the results of the test are influenced by external parameters (i.e. parameters that are not indicative of a user's ability to perform a word reading test) such as e.g. lag in the display of successive words. In embodiments, a portion of the n words may be displayed concomitantly, which portion may be updated as the user progresses through the test, such as e.g. through the individual scrolling down. In embodiments, all of the n words are displayed concomitantly. Such embodiments may advantageously reduce the impact of external parameters such as e.g. lag in the display of successive words, delay in a user scrolling down or up to make new words appear or restart from the beginning of a set of words, etc.

In embodiments of any aspect, obtaining a voice recording comprises assessing the quality of the voice recording by determining the noise level and/or the signal-to-noise ratio of the recording. The signal (resp., noise) in the recording may be estimated based on (such as e.g. by taking the average of) the relative energy values assumed to correspond to signal (resp. noise). The relative energy values assumed to correspond to signal may be e.g. the top x (where x can be e.g. 10%) relative energy values observed in the recording. Similarly, the relative energy values assumed to correspond to background noise may be e.g. the bottom x (where x can be e.g. 10%) relative energy values observed in the recording. Advantageously, where a relative energy is used, a value for the signal and/or noise in decibels can be obtained as 10*log₁₀(relE), where relE is a relative energy value, such as the average relative energy value of the top 10% or bottom 10% of the relative energy values observed in a recording. As explained further below, relative energy values may be obtained by normalising the observed power (also referred to as energy) values against the highest value observed in the recording. This leads to the highest observed energy having a relative energy of 0 dB. In such embodiments, a signal to noise ratio may be determined as the ratio of the signal estimated as explained above (e.g. average relE for top x% of observed relE in a recording) to the noise as explained above (e.g. average relE for top x% of observed relE in a recording). This can be provided as a value in dB by taking the log₁₀ of this ratio and multiplying the result by 10. In some such embodiments, the method may comprise analysing the voice recording if the noise level is below a predetermined threshold and/or the signal level is above a predetermined threshold and/or the signal-to-noise ratio is above a predetermined threshold. A suitable threshold for noise level may be chosen as -70 dB, -60 dB, -50 dB, or -40 dB (preferably about -50 dB). A suitable threshold for signal-to-noise ratio may be chosen as 25 dB, 30 dB, 35 dB, or 40 dB (preferably above 30 dB). In embodiments, obtaining a voice recording comprises applying one or more pre-processing procedures to a previously acquired voice recording audio file. Within the context of the present invention, a "pre-processing procedure" refers to any step applied to the voice recording data prior to analysis according to the present invention (i.e. prior to identifying single word segments). In embodiments, obtaining a voice recording comprises applying one or more pre-processing procedures to reduce the size of a previously acquired voice recording audio file. For example, down-sampling may be used to reduce the size of the audio file used. The present inventors have found that voice recording audio files could be down-sampled to 16Hz without loss of performance of the method. This may be particularly advantageous where the analysis is performed on a remote computing device and the recording obtained at a user computing device, as it facilitates that transmission of the voice recording from the user computing device to the remote computing device.

At step 220, a plurality of segments of the voice recording that correspond to single words or syllables are identified. Step 220 may be performed as described below in relation to Figure 3 (step 320).

At steps 230-270, the correct word rate (number of words correctly read per unit of time) in the voice recording is determined.

In particular, at step 230, one or more Mel-frequency cepstral coefficients (MFCCs) are computed for each of the segments identified at step 220. As a result, a plurality of vectors of values is obtained, each vector being associated with a segment. In the embodiment shown on Figure 2, optional steps of normalising 232 the MFCCs across segments in the recording and compressing 234 each of the plurality of vectors to a common size are shown. In particular, a set of i MFCCs (e.g. 12 MFCCs: MFCCs 2 to 13) is computed for each frame of the segment and a set of j values (e.g. 12 values) is obtained for the segment by compressing the signal formed by each of the i MFCCs across the frames in the segment, to obtain a vector of ixj values (e.g. 144 values) for the segment.

At step 240, the plurality of vectors of values are clustered into n clusters (e.g. using k-means), where *n* is the expected number of different words in the word-reading test. A particular label (i.e. word identity) is not associated with each cluster. Instead, it is assumed that segments that correspond to the same word (in case of monosyllabic words) or to the same syllable of the same word (in the case of disyllabic words) will be captured by MFCCs that cluster together. In the case of disyllabic words, one of the syllables in a word may be dominant in the clustering, and it is assumed that segments corresponding to the same dominant syllable will be captured by MFCCs that cluster together. Non-dominant syllables may effectively act as noise in the clustering. Following these assumptions, each cluster should primarily group values corresponding to segments that contain one of the n words, and one of the n! possible permutation of the n labels for these clusters corresponds to the (unknown) true labels.

At step 250, a sequence of words in the voice recording is predicted for each of n! possible permutation of the n labels. For example, for a possible assignment of the n labels, a cluster is predicted for the identified segments and the corresponding label is predicted as the word that is captured in the identified segments. Some identified segments may not be associated with a cluster, for example because the MFCCs for the segment are not predicted to belong to a particular cluster with a high enough confidence. In such cases, no word may be predicted for this segment. This may be the case e.g. for segments that correspond to erroneous detections of syllables/words, or segments that correspond to a non-emphasized syllable of a multi-syllable word.

At step 260, a sequence alignment is performed (e.g. using the Smith-Waterman algorithm) between each of the predicted sequences of words and the sequence of words used in the word reading test. The sequence of words used in the word reading test may be retrieved from memory, or may be received (for example, together with the voice recording) by the processor implementing the steps of the method.

At step 270, the labels that result in the best alignment (for example, the labels that result in the highest alignment score) are selected and assumed to be the true labels for the cluster. The matches in the alignment are assumed to correspond to correctly read words in the voice recording, and can be used to calculate a correct word rate. The correct word rate may be obtained, for example, by dividing the total number of correctly read words (matches) by the total time of the recording. Alternatively, the correct word rate may be obtained by computing a plurality of local averages within respective time windows, then either considering the plurality of resulting correct word rate estimates, or obtaining a summarised metric (e.g. average, median, mode) for the plurality of correct word estimates. Preferably, the correct word rate may be estimated as the slope of a linear model fitted to the cumulative number of correct words read as a function of time. Such a count may be increased by one unit at the time corresponding to the start of any segment that is identified as corresponding to a word that is correctly read. In yet other embodiments, determining the correct word rate associated with the voice recording comprises dividing the recording into multiple equal time bins, computing the total number of correctly read words in each time bin, and computing a summarised measure of the correct word rate across time bins. For example, the average, trimmed average or median correct word rate across time bins may be used as a summarised measure of the correct word rate. The use of the median or trimmed average may advantageously reduce the effect of outliers such as e.g. bins that do not contain any words.

When a plurality of voice recordings are obtained, these may be analysed separately or at least partially together. In embodiments, a plurality of voice recordings are obtained for the same subject, and at least steps 220 and 230 are performed individually for each voice recording. In embodiments, a plurality of voice recordings are obtained for the same subject, and at least step 240 is performed jointly using the values from multiple recordings of the plurality of recordings. In embodiments, steps 250-270 are performed individually for each recording, using the results of a clustering step 240 performed using the values from one or more (such as all of) the plurality of recordings.

**Figure 3** is a flow chart illustrating a flow chart illustrating a method of assessing the physiological and/or pathological state of a subject by determining the voice pitch, breathing % and/or unvoicing/voicing ratio from a word reading test. The method comprises obtaining 310 a voice recording from a word-reading test from the subject. The voice recording may be from a word-reading test comprising reading a sequence of words drawn from a (closed) set of n words. In particular, the words preferably do not have any particular logical connection.

At step 320, a plurality of segments of the voice recording that correspond to single words or syllables are identified. It is particularly advantageous for the words used in the reading test to be monosyllabic as in such cases each segment may be assumed to correspond to a single word, and the timing of segments can therefore be directly related to speech rate. Where disyllabic words (or other multi-syllabic words) are used, it may be advantageous for all words to have the same number of syllables as this may simplify the calculation and/or interpretation of the speech rate.

At step 330, the breathing % and/or unvoicing/voicing ratio and/or voice pitch associated with the voice recording is determined at least in part using the segments identified in the voice recording.

The breathing percentage reflects the proportion of time in the recording that comprises voicing segments. This may be calculated as the ratio between the amount of time between segments identified in step 320, and the total amount of time in the recording, or the sum of the time within segments identified in step 320 and the time between segments identified in step 320. The unvoicing/voicing ratio represents the amount of time in the recording in which the subject is breathing or assumed to be breathing, relative to the amount of time in the recording in which the subject is producing vocalisations. The unvoicing/voicing ratio may be determined as the ratio of (i) the amount of time between segments identified in step 320, and (ii) the amount of time within segments identified in step 320.

The voice pitch associated with a voice recording or a segment thereof refers to an estimate of the fundamental frequency of the sound signal in the recording. Thus, the voice pitch may also be designated herein as F0 or f0, the "f" referring to frequency and the "0" index indicating that the frequency this is estimated is assumed to be the fundamental frequency. The fundamental frequency of a signal is the inverse of the fundamental period of the signal, where the fundamental period of the signal is the minimum repetition interval of the signal. A variety of computational methods are available to estimate the pitch of a signal (or its fundamental frequency), and all such methods may be used herein. Many computational pitch estimation methods estimate the pitch of a signal by dividing the signal into time windows, then for each window: (i) estimate the spectrum of the signal (e.g. using a short-time Fourier transform), (ii) compute a score for each pitch candidate within a predetermined range (e.g. by computing an integral transform over the spectrum), and (ii) pick the candidate with highest score as the estimated pitch. Such methods may result in a plurality of pitch estimates (one for each time window). Thus, the pitch estimate for the signal may be provided as a summarised estimate across windows (e.g. the mean, mode or median pitch across windows) and/or as a range. More recently, methods based on deep learning have been proposed, some of which determine a pitch estimate for a signal (i.e. providing as output a predicted pitch for the signal rather than for each of a plurality of windows in the signal). Determining the voice pitch may comprise obtaining a voice pitch estimate or an estimated range of voice pitches for each segment identified in step 320. A voice pitch for a segment may be a summarised estimate of voice pitch across the segment, such as the mean, median or mode of a plurality of voice pitch estimates for the segment. A voice pitch range for a segment may be a range of voice pitches within which a predetermined proportion of a plurality of voice pitch estimates for the segment may be expected to fall. For example, a voice pitch range for a segment may be the interval between the lowest and the highest pitch estimates from a plurality of voice pitch estimates for the segment. Alternatively, a voice pitch range for a segment may be the interval between the xth percentile and the yth percentile of a plurality of voice pitch estimates for the segment. As another alternative, a voice pitch range for a segment may be the interval corresponding to a confidence interval around the mean voice pitch of a plurality of voice pitch estimates for the segment. Such a confidence interval may be obtained by applying a range around the mean value, wherein the range is expressed in units of the estimated standard deviation around the mean (e.g. mean ± n SD, where SD is the standard deviation and n can be any predetermined value). Determining the voice pitch may comprise obtaining a summarised voice pitch estimate or a summarised estimated range of voice pitches across segments identified in step 320 and for which a voice pitch estimate or estimated range of voice pitches has been obtained. A summarised voice pitch estimate across a plurality of segments may be obtained as the mean, median or mode of a plurality of voice pitch estimates for respective segments. A summarised estimated range of voice pitches across segments may be obtained as explained above, using estimated voice pitches for the respective segments (whether comprising one -e.g. summarised - or a plurality of voice pitch estimates per segment).

A voice pitch (or plurality of voice pitches) for a segment may be estimated using any method known in the art. In particular, a voice pitch for a segment may be estimated using the SWIPE or SWIPE' method as described in Camacho and Harris (2008). Preferably, a voice pitch estimate for a segment is obtained by applying SWIPE' to the segment. This method was found to strike a good balance between accuracy and speed of computation. Compared to SWIPE, SWIPE' uses only the first and prime harmonics of the signal, thereby reducing subharmonic errors. Alternatively, pitch estimation may be performed using a deep learning approach, such as the CREPE method as described in Kim et al. (2018). This method was found to lead to robust pitch estimates, albeit with an increased computational burden compared to methods such as SWIPE or SWIPE'. Alternative methods may also be used, such as e.g. PYIN (as described in Mauch and Dixon (2014) or the method described in Ardaillon and Roebel (2019)). Pitch estimation is typically applied using the signal from windows of time (as described above, also referred to as "frames"). Thus, pitch estimation for a segment may produce a plurality of estimates, each corresponding to a frame. Suitably, a plurality of pitch estimates (such as e.g. corresponding to a plurality of frames in a segment) may be further processed to reduce estimation error, for example by applying a median filter. The inventors have found a median filter applied using a 50 ms window to be particularly suitable. The average of such filtered estimates for a segment may be used as a pitch estimate for the segment.

A method that can be used to identify a plurality of segments of the voice recording that correspond to single words or syllables will now be described. Other methods exist in the art, and such other methods may also be used in other embodiments. In the embodiment illustrated on Figure 3, at step 322, a power Mel-spectrogram of the voice recording is obtained. This is typically achieved by defining frames along the voice recording (where a frame can correspond to the signal in a sliding window of fixed width applied along the time axis) and computing a power spectrum on a Mel scale for each frame (typically by obtaining a spectrogram for each frame then mapping the spectrogram to a Mel scale using overlapping triangular filters along a range of frequencies assumed to correspond to the human hearing range). This process results in a matrix of values of power per Mel unit per time bin (where a time bin corresponds to one of the positions of the sliding window). Thus, in embodiments of any aspect, obtaining a power Mel-spectrogram of the voice recording comprises applying a sliding window (preferably with a size of 15 ms and a step size of 10 ms) and 138 triangular filters spanning the range of 25.5 Hz to 8 kHz. Without wishing to be bound by theory, it is believed that using relatively narrow time windows (e.g. 10-15 ms, as opposed to e.g. 25 ms and above) may be useful in the context of identifying segments that correspond to single words or syllables, and in particular for the purpose of identifying segment boundaries that correspond to the start of words or syllables. This is because using relatively narrow time windows may increase the sensitivity of the detection, whereas wider time windows may smooth out small signals that may be informative.

As the skilled person understand, the overlapping triangular filters (typically 138) applied to a frequency spectrogram (Hz scale) are commonly used to obtain a spectrogram in Mel scale. Further, spanning the range of 25.5 Hz to 8 kHz has been found to be advantageous as this adequately captures the human hearing range.

Optionally, the power Mel-spectrogram may be normalised 323, for example by dividing the values for each frame by the highest energy value observed in the recording. At step 324, the maximum intensity projection of the Mel spectrogram along the frequency axis is obtained. Segment boundaries are identified 326 as time points where the maximum intensity projection of the Mel spectrogram along the frequency axis crosses a threshold. In particular, a set of two consecutive boundaries that are such that the maximum intensity projection of the Mel spectrogram crosses the threshold from a lower to a higher value at the first boundary, and the maximum intensity projection of the Mel spectrogram crosses the threshold from a higher to a lower value at the second boundary may be considered to define a segment that corresponds to a single word or syllable. The threshold used at step 326 may optionally be dynamically determined at step 325 (where the word "dynamically determined" refers to the threshold being determined for a particular voice recording, depending on features of the particular voice recording, rather than being predetermined independently of the particular recording).

Thus, in embodiments, the threshold is dynamically determined for each recording. Preferably, the threshold is determined as a function of the maximum intensity projection values for the recording. For example, the threshold may be determined as a weighted average of the relative energy values assumed to correspond to signal and the relative energy values assumed to correspond to background noise. The relative energy values assumed to correspond to signal may be e.g. the top x (where x can be e.g. 10%) relative energy values observed in the recording. Similarly, the relative energy values assumed to correspond to background noise may be e.g. the bottom x (where x can be e.g. 10%) relative energy values observed in the recording. The use of the average value of the top 10% relative energy values across frames and the average value of the bottom 10% relative energy values across frames may be particularly convenient. Alternatively, a predetermined value of relative energy assumed to correspond to signal (i.e. voice signal) may be used. For example, a value of about -10 dB has been commonly observed by the inventors and could be usefully chosen. Similarly, a predetermined value of relative energy assumed to correspond to background noise (may be used. For example, a value of about -60 dB has been commonly observed by the inventors and could be usefully chosen.

Where the threshold is determined as a weighted average of the relative energy values assumed to correspond to signal and the relative energy values assumed to correspond to background noise, the weight for the latter may be chosen between 0.5 and 0.9, and the weights for the former may be chosen between 0.5 and 0.1. In embodiments, the weight for the background noise contribution may be higher than the weight for the signal contribution. This may be particularly advantageous when the voice recording has been pre-processed by performing one or more noise-cancelling steps. Indeed, in such cases the bottom part of the signal (low relative energies) may contain more information than expected for a signal that has not been pre-processed for noise cancelling. Many modern computing devices including mobile devices may produce voice recordings that are already pre-processed to some extent in this way. As such, it may be useful to emphasise the bottom end of the relative energy values to some extent. Weights of about 0.2 and about 0.8, respectively for the signal and background noise contributions, may be advantageous. Further, thresholds that are advantageous may be determined by trial-and-error and/or formal training using training data. Without wishing to be bound by theory, it is believed that the use of a dynamically determined threshold may be particularly advantageous where the voice recording comprises a reference tone and/or where the signal-to-noise ratio is good (e.g. above a predetermined threshold, such as 30 dB). Conversely, the use of a predetermined threshold may be particularly advantageous where the voice recording does not comprise a reference tone and/or the signal-to-noise ratio is poor.

In other embodiments, the threshold is predetermined. In embodiments, the predetermined threshold is chosen between -60 dB and -40 dB, such as e.g. -60 dB, -55 dB, -50 dB, -45 dB, or -40 dB. Preferably, the predetermined threshold is about -50 dB. The inventors have found that this threshold stroke a good balance between sensitivity and specificity of word/syllable boundary identification in good quality voice recordings, and particularly in voice recordings that have been pre-processed using one or more noise cancellation steps.

Optionally, the segments may be "refined" by analysing separate segments identified in step 326 and determining whether further (internal) boundaries can be found. Thus, identifying segments of the voice recording that correspond to single words or syllables may further comprise performing onset detection for each of the segments and defining a further boundary whenever an onset is detected within a segment, thereby forming two new segments.

This may be performed by performing 327 onset detection for at least one of the segments by computing a spectral flux function over the Mel-spectrogram for the segment and 328 defining a further (internal) boundary whenever an onset is detected within a segment, thereby forming two new segments. Onset detection using a spectral flux function is commonly used for the analysis of music recordings, for beat detection. As the skilled person understands, onset detection using the spectral flux function is a method that looks at the derivative of the energy signal. In other words, the spectral flux function measures how quickly the power spectrum of the signal is changing. As such, it may be particularly useful to identify "valleys" in the signal (sudden changes in the energy signal) that may correspond to the start of new words or syllables within a segment. This may advantageously "refine" the segmentation where necessary. This approach may be particularly useful as a "refinement step" where words/syllable boundaries have already been identified using a less sensitive approach resulting in "coarse" segments. This is at least in part because the approach can be applied independently to a segment, with appropriate parameters (e.g. threshold for onset detection) for the segment,

Performing 327 onset detection may comprise computing 327a a spectral flux function or onset strength function, normalising 327b the onset strength function for the segment to a value between 0 and 1, smoothing 327c the (normalised) onset strength function and applying 327d a threshold to the spectral flux function or a function derived therefrom, wherein an onset is detected where the function increases above the threshold. Thus, performing onset detection may comprise applying a threshold to the spectral flux function or a function derived therefrom, wherein an onset is detected where the function increases above the threshold. In embodiments, performing onset detection comprises normalising the onset strength function for the segment to a value between 0 and 1 and separating segments into sub-segments if the normalised onset strength is above a threshold. A threshold of between 0.1 and 0.4, such as between 0.2 and 0.3 may result in particularly low rates of false positives when applied to the normalised onset strength function. An appropriate threshold may be defined as a threshold that minimises the rate of false positive detections when the method is applied to training data.

In embodiments, performing onset detection comprises computing an onset strength (based on the spectral flux function but including a spectral-trajectory tracking stage to the common spectral flux calculation method) over time from the power Mel-spectrogram, using the superflux method described in Böck S and Widmer G (2013). In embodiments, performing onset detection comprises computing the onset strength function over time from the power Mel-spectrogram, using the superflux method as implemented in the LibROSA library (https://librosa.github.io/librosa/, see function *librosa.onset.onset_strength*; McFee et al.(2015)). Preferably, performing onset detection further comprises normalising the onset strength function for the segment to a value between 0 and 1. This may be achieved for example by dividing each value of the onset strength function by the maximum onset strength within the segment. Normalising the onset strength function may result on a reduction of the number of false positive detections.

In embodiments, performing onset detection further comprises smoothing the (optionally normalised) onset strength function for the segment. For example, smoothing may be obtained by calculating a moving average with a fixed window size. For example, a window size of 10-15 ms, such as e.g. 11 ms may be useful. Smoothing may further reduce the rate of false positives detected.

An optional erroneous detection removal step 329 is shown on Figure 3. The process of identifying correctly read words as described herein is advantageously tolerant to the presence of erroneously detected segments at least to some extent. This is at least in part because the alignment step can include gaps for erroneous detections without significantly impacting the overall accuracy of the method. Thus, in embodiments the erroneous detection removal step may be omitted. In the embodiment shown on Figure 3, the erroneous detection removal step comprises computing 329a one or more Mel-frequency cepstral coefficients (MFCCs) for the segments (preferably the first 3 MFCCs as these are expected to capture features that distinguish between noise and true utterances) to obtain a plurality of vectors of values, each vector being associated with a segment, and excluding 329b all segments whose vector of values is above a predetermined distance from the remaining vectors of values. This approach assumes that the majority of segments are correct detections (i.e. correspond to true utterances), and that segments that do not contain true utterances with have different MFCC features from correct detections. Other outlier detection methods may be applied to exclude some of the plurality of vectors of values assumed to be associated with erroneous detections.

In embodiments, identifying segments of the voice recording that correspond to single words/syllables further comprises excluding segments that represent erroneous detections by removing segments shorter than a predetermined threshold and/or with mean relative energy below a predetermined threshold. For example, segments shorter than 100 ms may advantageously be excluded. Similarly, segments with a mean relative energy below -40 dB may advantageously be excluded. Such an approach may simply and efficiently exclude segments that do not correspond to words or syllables. Preferably, the segments are filtered to exclude short and/or low energy segments prior to calculating MFCCs for segments and applying an outlier detection method as explained above. Indeed, this advantageously avoids the unnecessary step of computing MFCCs for erroneous segments, and prevents such erroneous segments from introducing further noise in the outlier detection method.

Computing one or more Mel-frequency cepstral coefficients (MFCCs) for a segment typically comprises defining frames along the segment of voice recording (where a frame can correspond to the signal in a window of fixed width applied along the time axis). The window is typically a sliding window, i.e. a window of set length (e.g. 10-25 ms, such as 25 ms) moved along the time axis with a defined step length (e.g. 3-10 ms, such as 10 ms), resulting in frames that are partially overlapping. Computing one or more MFCCs typically further comprises, for each frame, computing a Fourier transform (FT) of the signal within the frame, mapping the powers of the spectrum thus obtained onto the Mel scale (e.g. using triangular overlapping filters), taking the log of the powers at each of the Mel frequencies and performing a discrete cosine transform of the signal thus obtained (i.e. obtaining a spectrum-of-spectrum). The amplitudes of the resulting spectrum represent the MFCCs for the frame. As explained above, a set of 138 Mel values is commonly obtained for the power Mel-spectrum (i.e. the frequency range is commonly mapped to 138 Mel scale values using 138 overlapping triangular filters). However, through the process of calculating MFCCs, this information is compressed into a smaller set of values (the MFCCs), typically 13 values. In many cases, the information contained in multiple of the 138 Mel values will be correlated such that compression of this signal does not result in a detrimental loss of informative signal.

In particular, computing one or more Mel-frequency cepstral coefficients (MFCCs) for a segment may be performed as described in Rusz et al. (2015). Computing one or more Mel-frequency cepstral coefficients (MFCCs) for a segment may be performed as implemented in the LibROSA library (https://librosa.github.io/librosa/; McFee et al. (2015); see *librosa.feature.mfcc*)*.* Alternatively, computing one or more MFCCs for a segment may be performed as implemented in the library "python_speech_features" (James Lyons et al., 2020).

In embodiments, computing one or more Mel-frequency cepstral coefficients (MFCCs) for a segment comprises computing at least the first 3 MFCCs (optionally, all 13 MFCCs) for each frame of the segment and computing a summarised measured for each MFCC across frames in the segment, thereby obtaining a vector of at least 3 values (one for each MFCC used) for the segment. The number and/or identity of the at least 3 MFCCs used for the outlier detection method may be determined using training data and/or internal control data. For example, the at least 3 MFCCs may be chosen as the minimal set of MFCCs that is sufficient to remove a percentage (e.g. at least 90%, or at least 95%) of erroneous detections in training data. As another example, the at least 3 MFCCs may be chosen as the minimal set of MFCCs that is sufficient to remove a segment corresponding to an internal control (such as e.g. a reference tone as will be described further below). Preferably, only the first 3 MFCCs are used for the outlier detection method. This advantageously captures the information that enables the separation of true words/syllables from erroneous detections (e.g. respiration, non-speech sound), without introducing information that could result in different words forming separate distributions of points that could confuse the outlier detection process.

In embodiments, applying an outlier detection method to the plurality of vectors of values comprises excluding all segments whose vector of values is above a predetermined distance from the remaining vectors of values. The distance between a specific vector of values and the remaining vectors of values may be quantified using the Mahalanobis distance. The Mahalanobis distance is a convenient measure of the distance between a point and a distribution. It has the advantage of being unitless, scale-invariant, and taking into account the correlations in the data. Alternatively, the distance between a specific vector of values and the remaining vectors of values can be quantified using the distance (e.g. Euclidian distance, Manhattan distance) between the specific vector of values and a representative value (e.g. the mean or medoid) for the remaining vectors of values. The values may optionally be scaled, such as e.g. to have unit variance along each coordinate, prior to applying outlier detection. The predetermined distance may be chosen depending on the observed variability in the plurality of vectors of value. For example, the predetermined distance may be a multiple of a measure of variability in the data, such as the standard deviation, or the value of a chosen quantile. In such embodiments, the predetermined distance may be chosen depending on the expected proportion of erroneous detections. A threshold of between 1 and 3 standard deviations around the mean of the plurality of vectors of values may be selected and may enable accurate removal of outliers. A threshold of 2 standard deviations was found advantageous, particularly when an expected proportion of erroneous detections is around 5 %.

An arguably similar approach to erroneous detection removal is described in Rusz et al. (2015). However, the approach described in this document is significantly more complex than that of the present disclosure. In particular, it relies on an iterative process where at each iteration inliers and outliers are identified using a quantile-based threshold on the distribution of mutual distances, then outliers are excluded using a quantile-based threshold on the distribution of distances between inliers and outliers as previously defined. A simpler approach as described herein may be advantageous in the context of the present invention. Without wishing to be bound by theory, the approach to erroneous detection removal described herein is believed to be particularly advantageous in the present context because the proportion of erroneous detection is low. This may be due in part to the segment detection approach described herein having very high accuracy. Without wishing to be bound by theory, the approach to syllable segmentation used in Rusz et al. (2015) (which relies on parametrizing the signal to 12 MFCCs inside a sliding window of 10 ms length-3ms step, searching for a low frequency spectral envelope which can be described using the first 3 MFCCs, then computing the mean of each of the 3MFCCs inside each envelope and using k-means to separate these points into syllables and pauses) may not be as accurate as that described herein. This at least in part because it is designed to identify a contrast between pauses and words, where the words are all identical, and partially because the approach in Rusz et al. (2015) heavily relies on the iterative outlier detection process to increase the overall accuracy of the true positive segment identification process. Indeed, the Rusz et al. (2015) approach was developed specifically to deal with syllable detection using voice recordings where the patient is asked to repeat the same syllable at a comfortable pace. As such, the data consists of only two expected categories of segments (pauses and syllables) of homogeneous content. In such cases using the first 3 MFCCs in combination with a complex iterative error detection process for segment identification may achieve good accuracy. However, this may have lower accuracy in the context of analysing a voice recording from a word-reading test, at least because more than one type of syllables are expected.

The segments identified in step 320 may be used to determine words that are correctly read, and hence a correct word rate, in a word reading test as described in relation to Figure 2 (steps 230-270).

The present inventors have identified that the breathing %, unvoicing/voicing, voice pitch and correct word rate determined as explained in relation to Figures 2 and 3 may be used as biomarkers that are indicative of a subject's physiological or pathological status. In particular, the biomarkers measured as described herein were found to be particularly sensitive indicators of a subject's level of dyspnea and/or fatigue, in particular the breathing %, unvoicing/voicing and correct word rate biomarkers. Further, the methods of obtaining a voice pitch estimate as described herein were found to lead to particularly reliable estimates, which could therefore be used as biomarkers or any physiological or pathological status that is associated with voice pitch variations. As such, the methods described herein may find uses in the diagnosing, monitoring or treatment of any conditions, diseases or disorders that are associated with dyspnea, fatigue and/or voice pitch variation.

**Figure 4** illustrates schematically a method of monitoring, diagnosing or providing a prognosis in relation to a disease, disorder or condition in a subject. The disease, disorder or condition is one that affects breathing, vocal tone, fatigue and/or cognitive ability.

The method comprises obtaining 410 a voice recording from a word-reading test from the subject. In the illustrated embodiment, obtaining a voice recording comprises causing 310a a computing device associated with the subject (e.g. computing device 1) to display a set of words (e.g. on display 104) and causing 310b the computing device 1 to record a voice recording (e.g. through microphone 105). Optionally, obtaining a voice recording may further comprises causing 310c the computing device to emit a reference tone. Obtaining 310 a voice recording from a word-reading test from the subject may instead or in addition comprise receiving a voice recording from a computing device associated with the subject (e.g. computing device 1).

The method further comprises identifying 420 a plurality of segments of the voice recording that correspond to single words or syllables. This may be performed as explained in relation to Figure 3. The method optionally further comprises determining 430 the speech rate associated with the voice recording, at least in part by counting the number of segments identified in the voice recording. The method further comprises determining 470 the correct word rate in the voice recording, as explained in relation to Figure 2 (steps 230-270). The correct word rate derived from the voice recording may be indicative of the level of cognitive impairment, fatigue and/or breathlessness of the subject. The method optionally comprises determining 430a the breathing percentage in the voice recording, as explained in relation to Figure 3 (steps 320-330). The breathing percentage derived from the voice recording may be indicative of the level of cognitive impairment, fatigue and/or breathlessness of the subject. The method optionally comprises determining 430b the unvoicing/voicing ratio in the voice recording, as explained in relation to Figure 3 (steps 320-330). The unvoicing/voicing ratio derived from the voice recording may be indicative of the level of cognitive impairment, fatigue and/or breathlessness of the subject. The method optionally comprises determining 430c the voice pitch in the voice recording, as explained in relation to Figure 3 (steps 320-330). The voice pitch derived from the voice recording may be indicative of the physiological and/or pathological state of the subject, such as e.g. the subject experiencing dyspnea, heart failure decompensation, an infection (especially a pulmonary infection), etc. The method may further comprise comparing 480 the metrics obtained at steps 430 and 470 with one or more previously obtained values for the same subject, or with one or more reference values. The one or more reference values may comprise one or more values for the one or more metrics, previously obtained for the same subject. Thus, any method described herein may comprise the step of repeating the method (such as e.g. repeating steps 410-480) for the same subject, at one or more further tie points. The one or more reference values may comprise one or more values for the one or more metrics, previously obtained from one or more reference populations (e.g. one or more training cohorts).

The comparison with previously obtained values for the same subject may be used to monitor a disease, disorder or condition in a subject who has been diagnosed as having the disease, disorder or condition, including in particular to monitor the symptoms of a disease, disorder or condition (such as e.g. dyspnea and/or fatigue) and/or the progression, recovery or treatment of the disease, disorder or condition, or to diagnose the subject as potentially having a condition that includes symptoms such as e.g. dyspnea and/or fatigue. Alternatively, the comparison with previously obtained values for the same subject may be used to diagnose a disease, disorder or condition. The comparison with one or more reference values may be used to diagnose the subject as having the disease, disorder or condition, or to monitor the progression, recovery or treatment of a disease, disorder or condition, including in particular monitoring the symptoms of a disease, disorder or condition. For example, the reference values may correspond to a diseased population and/or a healthy population. The monitoring of a disease, disorder or condition in a subject may be used to automatically assess a course of treatment, for example to determine whether a treatment is effective.

Any of the steps of identifying 420 a plurality of segments of the voice recording that correspond to single words or syllables, determining 430 the breathing %, unvoicing/voicing or pitch associated with the voice recording, and determining 470 the correct word rate in the voice recording may be performed by the user computing device 1, or by the analysis provider computer 2.

Thus, the disclosure relates in some embodiments to a method of monitoring a subject who has been diagnosed as having or being at risk of having a condition that affects breathing, vocal tone, fatigue and/or cognitive ability, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values. In embodiments of any aspect, the method further comprises treating the subject for the disease, disorder or condition.

The subject may be undergoing or may have undergone a particular course of treatment. Thus, references to monitoring a subject may comprise monitoring a subject's treatment, for example by measuring the one or more biomarkers disclosed herein at a first and further time point and determining by comparing the measured biomarkers at the first and further time points whether one or more symptoms of the subjects have improved between the first and further time point. Such methods may further comprise modifying the subject's course of treatment, or recommending that the subject's course of treatment is modified, if the comparison indicates that one or more symptoms of the subjects have not improved or have not sufficiently improved.

Also disclosed are methods of diagnosing a subject as having a condition that affects breathing, vocal tone, fatigue and/or cognitive ability, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values. In embodiments, the one or more biomarkers are selected from the breathing %, unvoicing/voicing ratio and correct word rate, and the one or more reference value are predetermined values associated with patients having the condition and/or patients not having the condition (e.g. healthy subjects). The predetermined values associated with patients having the condition and/or patients not having the condition may have been obtained previously using one or more training cohorts. In embodiments, the one or more biomarkers include the voice pitch, and the one or more reference values are values previously obtained from the same subject.

The condition may be a condition that is associated with dyspnea and/or fatigue. Thus, the disclosure also provides a method of monitoring a subject who has been diagnosed as having or being at risk of having a condition that is associated with dyspnea and/or fatigue, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values. Similarly, also disclosed herein is a method of assessing the level of dyspnea and/or fatigue in a subject, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values.

The condition may be a cardiovascular disease, such as heart failure, coronary heart disease, myocardial infarction (heart attack), atrial fibrillation, arrhythmia (heart rhythm disorders), and heart valve disease. In particular embodiments, the condition is heart failure. Thus, the disclosure also provides a method of identifying a subject with heart failure as having decompensated heart failure, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values. In embodiments, the one or more biomarkers are selected from the breathing %, unvoicing/voicing ratio and correct word rate, and the one or more reference value are predetermined values associated with decompensated heart failure patients and/or stable heart failure patients. The predetermined values associated with decompensated heart failure patients and/or stable heart failure patients may have been obtained previously using one or more training cohorts. In embodiments, the one or more biomarkers include the voice pitch, and the one or more reference values are values previously obtained from the same subject.

In embodiments, the disclosure also provides a method of monitoring a subject with decompensated heart failure, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values. In embodiments, the one or more biomarkers are selected from the breathing %, unvoicing/voicing ratio and correct word rate, and the one or more reference value are predetermined values associated with decompensated heart failure patients and/or stable heart failure patients and/or recovering decompensated heart failure patients. The predetermined values associated with decompensated heart failure patients and/or stable heart failure patients and/or recovering decompensated heart failure patients may have been obtained previously using one or more training cohorts. In embodiments, the one or more biomarkers include the voice pitch, and the one or more reference values are values previously obtained from the same subject. For example, the one or more reference values may comprise one or more values obtained when the subject was diagnosed with decompensated heart failure.

In embodiments, the one or more biomarkers include the breathing %, and a breathing % that is above a predetermined reference value or range of values indicates that the subject is likely to have a condition that is associated with dyspnea and/or fatigue, wherein the predetermined reference value or range of values are associated with a subject or group of subjects that are not likely to have the condition. In embodiments, the one or more biomarkers include the breathing %, and a breathing % that is below a predetermined reference value or range of values indicates that the subject is likely to be recovering from a condition that is associated with dyspnea and/or fatigue, wherein the predetermined reference value or range of values are associated with a subject or group of subjects that have the condition. In embodiments, the one or more biomarkers include the breathing %, and a breathing % that is below a predetermined reference value or range of values indicates that the subject is likely to be recovering from a condition that is associated with dyspnea and/or fatigue, wherein the subject has been diagnosed as having the condition and the predetermined reference value or range of values have been previously obtained from the same subject, such as e.g. at the time of diagnosing the subject as having the condition. In embodiments, the subject has been diagnosed as having a condition that is associated with dyspnea and/or fatigue and is undergoing treatment for the condition, the one or more biomarkers include the breathing %, and a breathing % that is below a predetermined reference value or range of values indicates that the subject is likely to be responding to the treatment. The predetermined reference value or range of values may have been previously obtained from the same subject, such as e.g. at the time of diagnosing the subject as having the condition, or from a group of subjects known to have the condition. In embodiments, the subject has been diagnosed as having a condition that is associated with dyspnea and/or fatigue and is undergoing treatment for the condition, the one or more biomarkers include the breathing %, and a breathing % that is at or above a predetermined reference value or range of values indicates that the subject is likely to not be responding to the treatment. The predetermined reference value or range of values may have been previously obtained from the same subject, such as e.g. at the time of diagnosing the subject as having the condition, or from a group of subjects known to have the condition. The condition may be decompensated heart failure.

In embodiments, the one or more biomarkers include the unvoicing/voicing ratio, and an unvoicing/voicing ratio that is above a predetermined reference value or range of values indicates that the subject is likely to have a condition that is associated with dyspnea and/or fatigue, wherein the predetermined reference value or range of values are associated with a subject or group of subjects that are not likely to have the condition. In embodiments, the one or more biomarkers include the unvoicing/voicing ratio, and an unvoicing/voicing ratio that is below a predetermined reference value or range of values indicates that the subject is likely to be recovering from a condition that is associated with dyspnea and/or fatigue, wherein the predetermined reference value or range of values are associated with a subject or group of subjects that have the condition. In embodiments, the one or more biomarkers include the unvoicing/voicing ratio, and an unvoicing/voicing ratio that is below a predetermined reference value or range of values indicates that the subject is likely to be recovering from a condition that is associated with dyspnea and/or fatigue, wherein the subject has been diagnosed as having the condition and the predetermined reference value or range of values have been previously obtained from the same subject, such as e.g. at the time of diagnosing the subject as having the condition. In embodiments, the subject has been diagnosed as having a condition that is associated with dyspnea and/or fatigue and is undergoing treatment for the condition, the one or more biomarkers include the unvoicing/voicing ratio, and an unvoicing/voicing ratio that is below a predetermined reference value or range of values indicates that the subject is likely to be responding to the treatment. The predetermined reference value or range of values may have been previously obtained from the same subject, such as e.g. at the time of diagnosing the subject as having the condition, or from a group of subjects known to have the condition. In embodiments, the subject has been diagnosed as having a condition that is associated with dyspnea and/or fatigue and is undergoing treatment for the condition, the one or more biomarkers include the unvoicing/voicing ratio, and an unvoicing/voicing ratio that is at or above a predetermined reference value or range of values indicates that the subject is likely to not be responding to the treatment. The predetermined reference value or range of values may have been previously obtained from the same subject, such as e.g. at the time of diagnosing the subject as having the condition, or from a group of subjects known to have the condition. The condition may be decompensated heart failure.

In embodiments, the one or more biomarkers include the correct word rate, and a correct word rate that is below a predetermined reference value or range of values indicates that the subject is likely to have a condition that is associated with dyspnea and/or fatigue, wherein the predetermined reference value or range of values are associated with a subject or group of subjects that are not likely to have the condition. In embodiments, the one or more biomarkers include the correct word rate, and a correct word rate that is above a predetermined reference value or range of values indicates that the subject is likely to be recovering from a condition that is associated with dyspnea and/or fatigue, wherein the predetermined reference value or range of values are associated with a subject or group of subjects that have the condition. In embodiments, the one or more biomarkers include the correct word rate, and a correct word rate that is above a predetermined reference value or range of values indicates that the subject is likely to be recovering from a condition that is associated with dyspnea and/or fatigue, wherein the subject has been diagnosed as having the condition and the predetermined reference value or range of values have been previously obtained from the same subject, such as e.g. at the time of diagnosing the subject as having the condition. In embodiments, the subject has been diagnosed as having a condition that is associated with dyspnea and/or fatigue and is undergoing treatment for the condition, the one or more biomarkers include the correct word rate, and a correct word rate that is above a predetermined reference value or range of values indicates that the subject is likely to be responding to the treatment. The predetermined reference value or range of values may have been previously obtained from the same subject, such as e.g. at the time of diagnosing the subject as having the condition, or from a group of subjects known to have the condition. In embodiments, the subject has been diagnosed as having a condition that is associated with dyspnea and/or fatigue and is undergoing treatment for the condition, the one or more biomarkers include the correct word rate, and a correct word rate that is at or below a predetermined reference value or range of values indicates that the subject is likely to not be responding to the treatment. The predetermined reference value or range of values may have been previously obtained from the same subject, such as e.g. at the time of diagnosing the subject as having the condition, or from a group of subjects known to have the condition. The condition may be decompensated heart failure.

In embodiments, the one or more biomarkers include the voice pitch, and a voice pitch that is significantly different from a predetermined reference value or range of values indicates that the subject is likely to have a condition that is associated with dyspnea and/or fatigue, wherein the predetermined reference value or range of values are associated with a subject or group of subjects that are not likely to have the condition. In embodiments, the one or more biomarkers include the voice, and a voice that is significantly different from a predetermined reference value or range of values indicates that the subject is likely to be recovering from a condition that is associated with dyspnea and/or fatigue, wherein the predetermined reference value or range of values are associated with a subject or group of subjects that have the condition. In embodiments, the one or more biomarkers include the voice pitch, and a voice pitch that is significantly different from a predetermined reference value or range of values indicates that the subject is likely to be recovering from a condition that is associated with dyspnea and/or fatigue, wherein the subject has been diagnosed as having the condition and the predetermined reference value or range of values have been previously obtained from the same subject, such as e.g. at the time of diagnosing the subject as having the condition. In embodiments, the subject has been diagnosed as having a condition that is associated with dyspnea and/or fatigue and is undergoing treatment for the condition, the one or more biomarkers include the voice pitch, and a voice pitch that is significantly different from a predetermined reference value or range of values indicates that the subject is likely to be responding to the treatment. The predetermined reference value or range of values may have been previously obtained from the same subject, such as e.g. at the time of diagnosing the subject as having the condition, or from a group of subjects known to have the condition. In embodiments, the subject has been diagnosed as having a condition that is associated with dyspnea and/or fatigue and is undergoing treatment for the condition, the one or more biomarkers include the voice pitch, and a voice pitch that is significantly different from a predetermined reference value or range of values indicates that the subject is likely to not be responding to the treatment. The predetermined reference value or range of values may have been previously obtained from the same subject, such as e.g. at the time of diagnosing the subject as having the condition, or from a group of subjects known to have the condition. Preferably, the predetermined reference value or range of values is/have been previously obtained from the same subject.

The condition may be a respiratory disease, such as obstructive lung disease (e.g. asthma, chronic bronchitis, bronchiectasis and chronic obstructive pulmonary disease (COPD)), chronic respiratory diseases (CRDs), respiratory tract infections, and lung tumours), respiratory infections (such as e.g. COVID-19, pneumonia, etc.), obesity, dyspnea (such as e.g. dyspnea associated with heart failure, panic attacks (anxiety disorders), pulmonary embolism, physical restriction or damage to the lungs (such as e.g. broken ribs, collapsed lungs, pulmonary fibrosis, etc.), pulmonary hypertension, or any other disease, disorder or condition that affects lung / cardiopulmonary function (for example as measurable by spiroergometry), etc..

Thus, also disclosed herein is a method of assessing pulmonary or cardiopulmonary function in a subject, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values. Also disclosed herein is a method of diagnosing a subject as having a respiratory disease, the method comprising obtaining a voice recording from a word reading test from the subject, identifying a plurality of single word/syllable segments, determining the value of one or more biomarkers selected from the breathing %, unvoicing/voicing ratio, voice pitch and correct word rate at least in part based on the identified segments, and comparing the value of the one or more biomarkers with one or more respective reference values. In embodiments, the one or more biomarkers are selected from the breathing %, unvoicing/voicing ratio and correct word rate, and the one or more reference value are predetermined values associated with patients having a respiratory disease and/or patient not having a respiratory disease (e.g. heathy subjects). The predetermined values may have been obtained previously using one or more training cohorts. In embodiments, the one or more biomarkers include the voice pitch, and the one or more reference values are values previously obtained from the same subject. Instead or in addition to this, the one or more biomarkers may include the voice pitch, and the one or more reference values may include values associated with patients having a respiratory disease and/or patient not having a respiratory disease (e.g. heathy subjects). The respiratory disease is preferably a disease that is associated with dyspnea. In embodiments, the disease is COVID-19.

Any condition that affect a subject's breathing ability (including e.g. psychiatric diseases such as anxiety disorders), fatigue (including e.g. psychiatric diseases such as depression and chronic fatigue syndrome), and/or cognitive ability (including e.g. psychiatric diseases such as attention deficit disorders) may advantageously be diagnosed or monitored using the methods of the present invention. Thus, in particular, the condition may be a neurovascular disease or disorders such as stroke, neurodegenerative diseases, myopathy, diabetic neuropathy, etc., a psychiatric disease or disorder such as depression, sleepiness, attention deficit disorder, chronic fatigue syndrome, or a condition that affect an individual's fatigue state or cognitive ability through systemic mechanisms such as pain, abnormal glucose levels (such as e.g. due to diabetes melitus), impairment of kidney function (such as e.g. in the context of chronic renal failure or renal replacement therapy), etc.

### Examples

### Example 1: Development of an automated smartphone-based Stroop word-reading test for the remote monitoring of disease symptoms

In this example, the inventors developed an automated smartphone-based Stroop word-reading test (SWR) and tested the feasibility of remote monitoring of disease symptoms in Huntington's Disease. In the smartphone-based SWR test, color words were displayed in black on the screen according to a randomly generated sequence (4 words per row and total 60 words are displayed). Speech data were recorded with built-in microphone and uploaded via WiFi to cloud. The inventors developed a language-independent approach to segment and classify individual words from speech signal. Finally, by comparing the displayed-word sequence with the predicted-word sequence, they were able to reliably estimate the number of correct words using the Smith-Waterman algorithm, commonly used for genomic sequence alignment.

### Methods

*Subjects and relative clinical assessments:* Forty-six patients were recruited from three sites, including Canada, Germany and the United Kingdom, as part of the HD OLE (open-label extension) study (NCT03342053). All patients underwent an extensive neurological and neuropsychological examination at the baseline visit. The Unified Huntington's Disease Rating Scale (UHDRS) was used to quantify disease severity. In particular, Stroop word-reading test (SCWT1-Word Raw Score) is part of the UHDRS cognitive assessment and dysarthria (UHDRS-dysarthria score) is part of the UHDRS motor assessment. The language spoken locally at each site was used (i.e. English in Canada and the United Kingdom n = 27, German in Germany n = 19).

*Smartphone App and* self-*administrated speech recordings:* A smartphone-based Stroop word-reading test was developed as a custom Android application (Galaxy S7; Samsung, Seoul, South Korea). At the baseline visit, patients received a smartphone and completed a test in a teaching session. The speech tests were then performed remotely at home weekly. Speech signals were acquired at 44.1 kHz with 16-bit resolution and down sampled to 16 kHz for analysis. Data was securely transferred via WiFi to a remote location, where it was processed and analysed. Data presented in this example were the first self-administered home tests (n = 46) only. A total of 60 color words (4 words per row) were displayed in black according to a randomly generated sequence and stored as metadata explicitly. Patients read the words after a brief reference tone (1.1 kHz, 50 ms) for a given 45-second period. Patients were instructed to restart reading the words from the beginning if the finished reading the 60 words within the 45 seconds time. All recordings analysed here were with a low ambient noise level (-56.7 ± 7.4 dB, n = 46) and good signal-to-noise ratio (44.5 ± 7.8 dB, n = 46).

*Language-independent approach for analysing the Stroop word-reading test:* With consideration of potential usage in multi-language and various diseased population settings, an algorithm was designed without any pre-trained models. Words were segmented directly from the speech signal in the absence of any contextual cues. At the classification stage, word label was chosen such that it maximizes partial overlaps between displayed and predicted sequence. The fully-automated approach for the Stroop word-reading test can be divided into four parts. Briefly, the inventors first introduced a two-step approach to obtain a highly sensitive segmentation of individual words. The inventors then deployed an outlier removal step to filter out error detections mainly caused by imprecise articulation, respirations and non-speech sound. They then converted each putative word represented by 144 (12 x 12) Mel-frequency cepstral coefficient (MFCC) features and performed a three-class K-means clustering. Finally, the inventors adopted the Smith-Waterman algorithm, a local sequence alignment method, to estimate the number of correct words. Each of these steps will be explained in further detail below.

*Identifying word boundaries:* In this particular example, each color word used consisted of a single syllable, i.e. /red/, /green/, /blue/ in English and /rot/, /grün/, /blau/ in German. The word segmentation therefore becomes a general syllable detection problem. According to phonology, the nucleus of a syllable also called the peak, is the central part of a syllable (most commonly a vowel), whereas consonants form the boundaries in between (Kenneth, 2003). A number of automatic syllable detection methods have been described for connected speech (see e.g. Xie and Niyogi, 2006; Wang and Narayanan, 2007; Rusz et al., 2016). For example, syllabic nuclei were identified mainly based upon either the wide-band energy envelope (Xie and Niyogi, 2006) or the sub-band energy envelope (Wang and Narayanan, 2007). However, for fast speech, the transition between different syllables is difficult to identify by energy envelope alone. When considering the fast tempo and syllable repetition in the word-reading task, there is still a need for more sensitive syllable nuclei identification.

The two-step approach was motivated by how hand-label syllable boundaries were performed - visual inspection of intensity and spectral flux of a spectrogram. Briefly, a power Mel-spectrogram was first computed with a sliding window size of 15 ms and a step size of 10 ms, 138 triangular filters that span the range of 25.5 Hz to 8 kHz, and normalized against the strongest frame energy in a 45 s period. The maximal energy of a speech frame was then derived to represent intensity that is equivalent to a maximum intensity projection of the Mel-spectrogram along frequency axis. In this way, the loudest frame will have relative energy value of 0dB and others will have values below it. For example, as shown in **Figure 5A****,** all syllabic nuclei have relative energy over -50dB. Coarse word boundaries were identified by thresholding on the relative energy measure.

Subsequently, the spectral flux of the Mel-spectrogram was calculated to identify the precise boundary of each word. This is equivalent to a vertical edge detection on a Mel-spectrogram. The onset strength was computed with the superflux method developed by Böck and Widmer (2013) and normalized to a value between 0 and 1. If the onset strength is over a threshold i.e. 0.2, the segment is divided into sub-segments. One coarsely segmented word (highlighted in grey) was divided into two putative words based on the onset strength shown in **Figure 5B****.**

All of the calculations were performed in Python, using the Librosa library (https://librosa.github.io/librosa/, McFee et al., 2015]) or the python_speech_features library (https://github.com/jameslyons/python speech features, James Lyons et al., 2020). For the computation of the onset strength, the function *librosa.onset.onset_strength* was used with parameters lag = 2 (time lag for computing differences) and max_size = 3 (size of the local max filter). In the example shown on Figures 5A-B, 68 coarse segments were identified in the first step, and a further 10 were identified in the refinement step.

In order to remove erroneous detections mainly caused by imprecise articulation, respirations and non-speech sound, an outlier removal step was implemented. Observations shorter than 100 ms and mean relative energy value less than -40 dB were firstly removed. Mel-frequency cepstral coefficients (MFCCs) are commonly used as features in speech recognition system (Davis and Mermelstein, 198; Huang et al., 2001). Here, a matrix of 13 MFCCs was computed with a sliding window size of 25 ms and a step size of 10 ms for each putative word. Audible noises are expected to differ from true words by the first three MFCCs (Rusz et al., 2015). The words were therefore parameterized using the means of the first three MFCC. Outlier detection was performed on these based on the Mahalanobis distance. A cut-off value of 2 standard deviations was used to identify outliers. **Figure 6** illustrates this step, with inliers (putative words) shown in grey and outliers (non-speech sounds) in black in the 3D scatterplot.

*K-means clustering:* K-means is an unsupervised clustering algorithm which divides observations into k clusters (Lloyd, 1982). The inventors assumed that words pronounced by a subject in a given recording will have a similar spectral representation within a word-cluster, and a different pattern between word-clusters. In this way, one can divide words into n clusters, where n is equal to the number of unique color words (here n=3). However, the duration of words may vary from one to another (mean duration between 0.23 and 0.35 ms). The steps to generate an equal sized feature representation for each word are as follows: starting from a previously computed 13 MFCCs matrix, the first MFCC (related to power) was removed from the matrix. The remaining 12 MFCCs matrix with various frame number was treated as an image and resized to a fixed-size image (12 x 12 pixels, reduced to 40%-60% of its width) by a linear interpolation along the time axis. As a result, each word was transformed to a total 144 MFCC values (12 x 12 = 144) regardless of its duration. By applying K-means clustering, putative words from one recording were classified into three different clusters. **Figure 7** illustrates the visual appearance of words in three distinctive clusters shown in upper graphs (one word per row) and the corresponding cluster centres shown in lower graphs, particularly Figure 7A represents 3 word-clusters extracted from one test in English (words = 75) and Figure 7B represents 3 word-clusters extract from one test in German (words = 64).

*Word sequence alignment:* Speech recognition refers to understand the speech content. In principle, it is possible to use deep learning models (e.g. Mozilla's free speech recognition project DeepSpeech) and hidden Markov models (e.g. Carnegie Mellon University's Sphinx toolkit) to perform speech recognition. However, such pre-trained models are built on healthy population and are language dependent, and might not be very accurate when applied to patients with speech impairments. In this study, the inventors introduced an end-to-end model-free solution to infer speech content. They converted such a word recognition task to a genomic sequence alignment problem. The closed-set of color words are like the letters of the DNA code. Reading errors and system errors introduced during segmentation and clustering steps are like mutations, deletions, or insertions occurring in the DNA sequence of a gene. Instead of performing isolated word recognition, the objective was to maximize the overlapping sequence between the displayed and predicted sequence, so that the entire speech content is leveraged as a whole.

The Smith-Waterman algorithm performs a local sequence alignment (i.e. some characters may not be considered), thus it is appropriate for partially overlapping sequences (Smith and Waterman, 1981). The algorithm enables to compare segments of all possible lengths and optimizes the similarity measure based on a scoring metric, e.g. a gap cost =2 match score=3. In this study, the number of segmented words defines the search space in the displayed sequence. In a three-class scenario, there are 6 (3!=6) possible permutations of word labels. For each permutation, it is possible to generate a predicted sequence, align with the displayed sequence, and trace back the segment that has the highest similarity score. The inventors made the assumption that subjects read words as displayed most of the time. Therefore, the segment length becomes the measure to maximize in the problem. In other words, the optimal choice of a label for a given cluster is found in a way that maximizes the overlapping sequences. Consequently, each word can be classified according to respective cluster labels. Moreover, the exact matches found in the partially overlapping sequences provides a good estimation of the correct words read by the subject. **Figure 8** takes the alignment of displayed sequence RRBGGRGBRRG and predicted sequence BRBGBGBRRB as an example and returns 5 correct words out of 10 read words.

*Manual level ground truth:* Manual annotations of all segmented words (1938 words from 27 recordings in English, 1452 words from 19 recordings in German) were performed blindly via audio playback. Manual label was performed after the algorithm was designed and was not used for parameter tuning. The beginning/end time of each word was obtained by the proposed two-step approach. Words were labelled with respective text accordingly, with /r/ for /red/ and /rot/, /g/ for /green/, and /grün/ and /b/ for /blue/ and /blau/. Words that were difficult to annotate for some reasons (e.g. imprecise syllable separations, respirations, other words etc.) were labelled as /n/, as a "garbage" class.

*Outcome measures:* Based on the word segmentation and classification results, two complementary test-level outcome measures were designed: the number of correct words for quantifying processing speed as part of the cognitive measures and the speech rate for quantifying speech motor performance. In particular, the speech rate was defined as the number of words per second and computed as the slope of the regression line on the cumulative sum of segmented words in time.

Statistical analyses: The Shapiro-Wilk test was used to test for a normal distribution. Pearson correlation was applied to examine significant relationships. The criteria used to evaluate Pearson correlation coefficient were fair (values of 0.25-0.5), moderate to good (values of 0.5-0.75) and excellent (values of 0.75 and above). ANOVA and unpaired t-test for independent samples were performed for comparison between groups. Effect sizes were measured with Cohen's d with d = 0.2 indicating a small, d = 0.5 a medium and d = 0.8 a large effect.

### Results

*Evaluation of word classification performance:* To estimate the classification accuracy of the proposed model-free word recognition algorithm, manual annotations and labels obtained by the automated algorithm were compared. The overall classification accuracy was high, with an average score of 0.83 in English and 0.85 in German. The normalized confusion matrices in **Figure 9** shows the performance of the model-free word classifier at word level. The high classification accuracy suggests that the proposed word recognizer could learn all the components of a speech recognizer including the pronunciation, acoustic and language content directly from a 45-second speech recording. It leverages an unsupervised classifier and a dynamic local sequence alignment strategy to tag each word. This means, during deployment, there is no need to carry around a language model making it very practical for applications in multi-language and various diseased population settings.

*Clinical validation of two complementary outcome measures:* The number of correct words determined by the fully-automated approach was compared with the standard clinical UHDRS-Stroop word score. In general, in term of the number of correct words, the smartphone and clinical measures are highly correlated (Pearson's correlation coefficient r = 0.81, p < 0.001) shown in **Figure 10****.**

*Evaluation of performance in further languages:* the results obtained in this study were further expanded upon in a study including HD patients speaking 10 different languages. In particular, the methods described in this example were applied to this multi-lingual cohort using the following words: 'English': ['RED', 'GREEN', 'BLUE'], 'German': ['ROT', 'GRUN', 'BLAU'], 'Spanish': ['ROJO', 'VERDE', 'AZUL'], 'French': ['ROUGE', 'VERT', 'BLEU'], 'Danish': ['RØD', 'GRØN', 'BLÅ'], 'Polish': ['CZERWONY','ZIELONY', 'NIEBIESKI'], 'Russian': [' 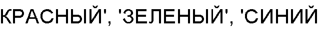 '], 'Japanese': [' 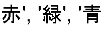 '], 'Italian': ['ROSSO', 'VERDE', 'BLU'], 'Dutch': ['ROOD', 'GROEN', 'BLAUW']. Of note, for some of these languages all of the words used were monosyllabic (e.g. English, German), whereas for other languages some of the words were disyllabic (e.g. Italian, Spanish). **Figure 11A** shows the distribution of number of correctly read words determined from sets of recordings in English, French, Italian and Spanish, and **Figure 11B** shows the distribution of the number of segments identified (directly prior to clustering, i.e. after refinement and outlier removal) in each of these languages. The data shows that the number of correctly read words identified according to the method described above is robust to variations in the length of the words (Figure 11A), even though multiple syllables in single words are identified as separate entities (Figure 11B).

### Conclusion

This example describes and shows the clinical applicability of an automated (smartphone-based) Stroop word-reading test that can be self-administered remotely from patient's home. The fully-automated approach enables to run offline analysis of speech data. The approach is language-independent using an unsupervised classifier and a dynamic local sequence alignment strategy to tag each word with respect to language content. Words were classified with a high overall accuracy of 0.83 in English speaking and 0.85 in German speaking patients, without any pre-trained models. The approach is shown to enable the assessment of cognitive function and speech motor function in patients with HD. Two complementary outcome measures were clinically validated, one for assessing cognitive capability and one for evaluating speech motor impairments, in 46 patients of the HD OLE study. In summary, the approach described herein succeeded to set the ground for self-assessment of disease symptoms using smartphone based speech tests in large populations. This may ultimately bring great benefit for patients to improve quality of life for most and clinical trials to find effective treatments.

### Example 2: Automated Stroop word-reading test - interference condition

In this example, the inventors tested whether the approach outlined in Example 1 could be used to automatically perform the interference part of the Stroop word-reading test. A cohort of healthy volunteers underwent both a Stroop word reading test as described in relation to Example 1, and a Stroop color word reading test. Further, the inventors tested the performance of the method by analysing recordings for a Stroop word reading test and a Stroop color word reading test using the same sequence of words, the words being displayed in black for the former and in inconsistent colors for the latter (see **Figures 12A** **and** **12B****).** The results of applying the methods described in Example 1 to the two voice recordings obtained from an individual performing those matched tests are shown in Figures 12A and 12B. In these figures, segments are highlighted in the middle panel of each figure as colored sections of signal, and the word predictions are indicated in the middle panel of each figure by the color of the segments. The data shows that the segment identification and correct word counting processes performs equally well for both the consistent condition and the interference condition. Indeed, there is no discrepancy in cluster assignment between the word reading and interference tests, despite the presence of incorrect words read by the individual in the interference tests. Further, as can also be seen on Figure 12B, the predicted numbers of correctly read words obtained using the described automated assessment method highly correlated with the ground truth data obtained by manual annotation of the voice recordings.

### Example 3: Automated web-based Stroop word-reading test for the remote monitoring of breathing symptoms, and the monitoring of disease symptoms in heart failure patients

In this example, the inventors implemented the automated Stroop word-reading test (SWR) described above in the context of remote monitoring of dyspnea, and disease symptoms in heart failure patients.

A similar set up as in Example 1 was used, except that the solution was deployed through a web based application. The set-up of the web-based test is shown on **Figure 13****.** Participants were asked to record themselves through their computing device while performing a plurality of tasks: (i) a reading task (reading of a patient consent statement, see top panel on Figure 13), (ii) a number counting task (reading numbers between 1 and 10), (iii) a reverse number counting test (reading numbers between 10 and 1), and (iv) two word-reading tests: a Stroop word reading test (consistent condition, color words randomly drawn from a set of 3 and displayed in black, as explained in Example 1) and a Stroop color word reading test (interference condition, color words randomly drawn from a set of 3 and displayed in randomly drawn colors).

Contrary to example 1, the recordings for the word reading tests were not of a fixed length of time. Instead, each recording is as long as it takes the individual to read all of the words displayed (in this case, 40 words). This is advantageous many patients with heart conditions or dyspnea may not have the physical strength to perform long tests. Further, the words displayed in the Stroop word reading test and the Stroop color word reading test were identical, only the color changes in the Stroop color word reading test. This advantageously enabled comparison of the recordings from the two tests as their vocal content should be similar, as well as enabling to obtain additional data for excellent accuracy in the clustering step. Indeed, the two recordings (i.e. 80 words in total, 40 from each of the Stroop word reading test and the Stroop color word reading test) were combined and used for each patient in the clustering step, in order to ensure that the clustering step is performed using enough words to have excellent accuracy. The segment identification steps were performed separately for the two recordings, as was the alignment step. Further, the segment identification step described in Example 1 was also applied to the reading task and number counting / reverse number counting recordings. The results of the alignment step were then used together with the segment information to calculate, for the Stroop word reading test and the Stroop color word reading test individually, the correct word rate (calculated as the number of correct words per second). The correct word rate was estimated as number of correct words read divided by the test duration. The cumulative number of words read was increased by 1 at the time corresponding to the start of every segment that was identified as corresponding to a correctly read word. The speech rate (i.e. all words, not only correct words) was also computed, as described in Example 1, using the slope of a linear model fitted to the cumulative number of words read.

The segment information was then used to assess, for each test individually: the breathing percentage (breathing %, calculated as 100*(time between segments)/(time within segments + time within segments)), the unvoicing/voicing ratio (calculated as (time between segments/time within segments)), and the mean voice pitch (calculated as the average of the individual voice pitches estimated for each segment). In each segment, the voice pitch was estimated using SWIPE' as implemented in the Speech Signal Processing Toolkit (http://sp-tk.sourceforge.net/) via the r9y9 Python wrapper (https://github.com/r9y9/pysptk). An alternative method (CREPE), as implemented in the Python package available at https://github.com/marl/crepe was also tested. The results showed here are using SWIPE'. A median filter with a size of 5 (corresponding to 50 ms time windows) was applied to the pitch estimations from the voicing segments, in order to reduce pitch estimation error. Finally, a single mean value was obtained for a given recording.

The method was first tested on a healthy subject who performed the test on a number of days, before and after moderate exercise (climbing 4 flights of stairs). This situation simulates the effect of dyspnea, and therefore tests the ability of the metrics described above to act as biomarkers of dyspnea. The results of this analysis are shown in Table 1 below and on **Figure 14** for the Stroop color word test (interference condition - panels A-D, and average of results for interference and coherent condition - panels A'-D') recordings on multiple days (rows), where panels A and A' show the pitch estimates, panels B and D' show the correct word rate, panels C and C' show the unvoicing/voicing ratio, and panels D and D' show the breathing %. The Cohen's d was calculated for each metric between the pre- and post-exercise results, to quantify the effect size associated with shortness of breath on each metric. For the pitch metric, the effect size (Cohen's d) was 3.47 for the combined test data and Cohen's d=2.75 for the interference condition alone. For the correct word rate, the Cohen's d was -2.26 for the combined test data and Cohen's d=-1.57 for the interference condition. For the unvoicing/voicing, the Cohen's d was 1.25 for the combined test data and Cohen's d=1.44 for the interference condition. For the breathing %, the Cohen's d was 1.26 for the combined test data and Cohen's d=1.43 for the interference condition. Thus, each of these metric shows a marked difference between rest and breathless status (whether using data from recordings of color word tests in the interference condition alone, or combining data from recordings of color word tests in the interference and coherent condition), and can therefore be used to monitor dyspnea.

**Table 1. Analysis of speech test recordings from healthy individuals at rest and after moderate exercise (p value=permutation test p value, mod.ex.=moderate exercise).**

| **Test** | **Group** | **Metric** | **Value (average ± standard deviation)** |
|---|---|---|---|
| word reading | rest | pitch | 179.8 ± 2.9, n = 15 |
| word reading | moderate exercise | pitch | 193.3 ± 4.1, n = 15 |
| color word reading | rest | pitch | 180.1 ± 3.3, n = 15 |
| color word reading | moderate exercise | pitch | 191.7 ± 4.8, n = 15 |
| word reading | rest | correct word rate (Stroop score) | 2.9 ± 0.2, n = 15 |
| word reading | moderate exercise | correct word rate | 2.4 ± 0.2, n = 15 |
| color word reading | rest | correct word rate | 2.7 ± 0.2, n = 15 |
| color word reading | moderate exercise | correct word rate | 2.4 ± 0.2, n = 15 |
| word reading | rest | unvoicing/voicing | 0.4 ± 0.1, n = 15 |
| word reading | moderate exercise | unvoicing/voicing | 0.5 ± 0.1, n = 15 |
| color word reading | rest | unvoicing/voicing | 0.4 ± 0.0, n = 15 |
| color word reading | moderate exercise | unvoicing/voicing | 0.5 ± 0.1, n = 15 |
| word reading | rest | breathing % | 30.5 ± 4.6, n = 15 |
| word reading | moderate exercise | breathing % | 33.9 ± 3.1, n = 15 |
| color word reading | rest | breathing % | 28.9 ± 2.4, n = 15 |
| color word reading | moderate exercise | breathing % | 32.7 ± 2.8, n = 15 |

| **Comparison** | **Condition** | **Metric** | **Cohen's d / p value** |
|---|---|---|---|
| color word vs. word | moderate exercise | pitch | -0.34 / 0.1816 |
| color word vs. word | moderate exercise | correct word rate | -0.25 / 0.2527 |
| color word vs. word | moderate exercise | unvoicing/voicing | -0.38 / 0.1518 |
| color word vs. word | moderate exercise | breathing % | -0.37/ 0.1579 |
| color word vs. word | rest | pitch | 0.10 / 0.3851 |
| color word vs. word | rest | correct word rate | -0.91 / 0.0077 |
| color word vs. word | rest | unvoicing/voicing | -0.46 / 0.1097 |
| color word vs. word | rest | breathing % | -0.41 / 0.1436 |
| mod.ex. vs. rest | color word reading | pitch | 2.75 / 0.0000 |
| mod.ex. vs. rest | color word reading | correct word rate | -1.57 / 0.0001 |
| mod.ex. vs. rest | color word reading | unvoicing/voicing | 1.44 / 0.0002 |
| mod.ex. vs. rest | color word reading | breathing % | 1.43 / 0.0001 |
| mod.ex. vs. rest | word reading | pitch | 3.68 / 0.0000 |
| mod.ex. vs. rest | word reading | correct word rate | -2.08 / 0.0000 |
| mod.ex. vs. rest | word reading | unvoicing/voicing | 0.82 / 0.0150 |
| mod.ex. vs. rest | word reading | breathing % | 0.85 / 0.0142 |

The data in Table 1 shows that each of the metrics tested shows a marked difference between rest and breathless status, and that this is consistent across a word test (color words, coherent condition) and color word test (color words, interference condition) (apart from the correct word rate which is of course likely to be higher in the coherent condition, and where comparison of the coherent and interference condition can rovide further indications in relation to cognitive ability). Thus, these metrics can therefore be used (alone for either the word test or color word test, or combining the two) to monitor dyspnea.

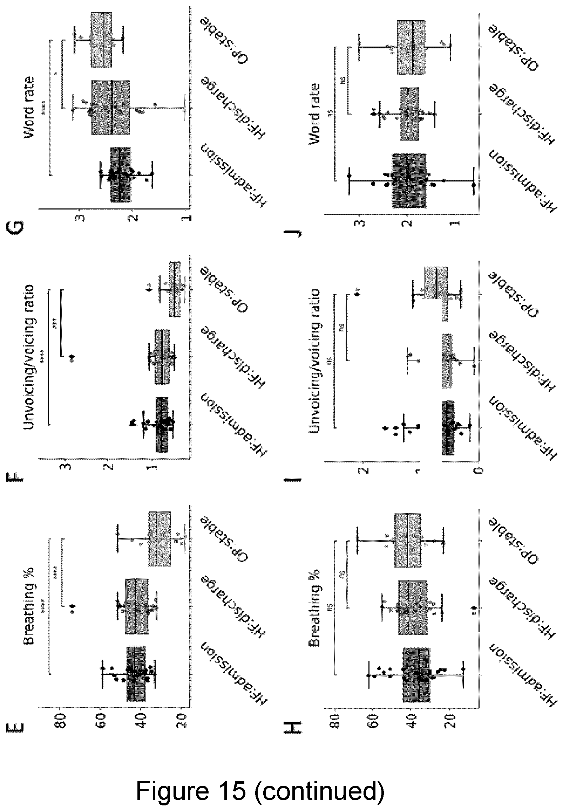

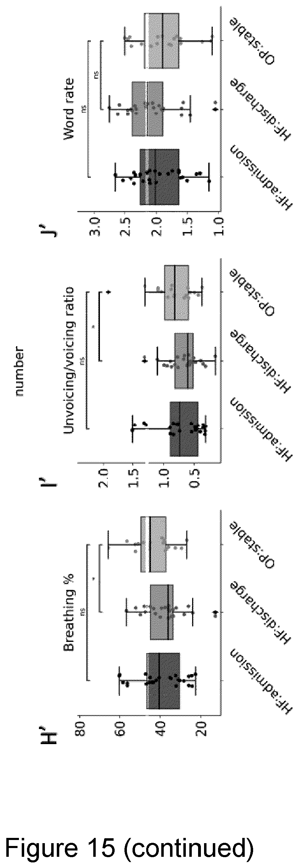

Thus, the inventors set out to determine whether these biomarkers could also be used to monitor heart failure patients. The metrics were obtained as explained in two cohorts of heart failure patients: a cohort of heart failure patients admitted to hospital for decompensation (n=25), and a cohort of stable heart failure outpatients (n=19). The former were assessed both upon admission to hospital (HF:admission) and on discharge (HF:discharge). The results of this analysis are shown in Tables 2 and 3, and on **Figures 15** **and** **16****-17.** The data on Figure 15, panels A-D and A'-D' shows that the selected metrics derived from the Stroop word reading tests (A-D: interference condition alone, A'D': average of interference and coherent condition) were significantly different between the decompensated heart failure patients and the stable outpatients. Further, the breathing %, unvoicing/voicing and correct word rate metrics were particularly sensitive metrics to differentiate these groups of patients. The characteristics of the data on Figure 15A'-D' and A-D are shown below.

Stroop score: number of correct words per second (combined color word reading tests, Fig. 15C'):
HF:admission (average ± standard deviation): 1.5 ± 0.4, n = 25
HF:discharge (average ± standard deviation): 1.6 ± 0.4, n = 25
OP:stable (average ± standard deviation): 1.9 ± 0.2, n = 19
HF:admission vs OP:stable: Cohens d: -1.09, permutation test p value = 0.0002
HF:discharge vs OP:stable: Cohens d: -0.81, permutation test p value = 0.0053
HF: admission vs HF:discharge: Cohens d: -0.21, permutation test p value = 0.2276

Stroop score: number of correct words per second (color word reading test, interference condition, Fig. 15C):
HF:admission (average ± standard deviation): 1.5 ± 0.4, n = 25
HF:discharge (average ± standard deviation): 1.6 ± 0.4, n = 25
OP:stable (average ± standard deviation): 1.9 ± 0.2, n = 19
HF:admission vs OP:stable: Cohen's d=-1.14, permutation test p-value=0.0001
HF:discharge vs OP:stable: Cohen's d=-0.87, permutation test p-value=0.0035
HF: admission vs HF:discharge: Cohens d: -0.28, permutation test p value = 0.1600

This data shows that the correct word rate from word reading test recordings can be used to differentiate decompensating heart failure patients from stable heart failure patients. Further, this metric can also be used to monitor the recovery of patients from the decompensated state.

RST (speech rate): number of words per second (combined color word reading tests, Fig. 15D'):
HF:admission (average ± standard deviation): 1.8 ± 0.3, n = 25
HF:discharge (average ± standard deviation): 1.8 ± 0.3, n = 25
OP:stable (average ± standard deviation): 2.0 ± 0.2, n = 19
HF:admission vs OP:stable: Cohens d: -0.92, permutation test p value = 0.0019
HF:discharge vs OP:stable: Cohens d: -0.95, permutation test p value = 0.0013
HF: admission vs HF:discharge: Cohens d: -0.07, permutation test p value = 0.4033

RST (speech rate): number of words per second (color word reading test, interference condition, Fig. 15D):
HF:admission (average ± standard deviation): 1.8 ± 0.3, n = 25
HF:discharge (average ± standard deviation): 1.7 ± 0.4, n = 25
OP:stable (average ± standard deviation): 2.0 ± 0.2, n = 19
HF:admission vs OP:stable: Cohen's d=-0.89, permutation test p-value=0.0019
HF:discharge vs OP:stable: Cohen's d=-0.98, permutation test p-value=0.0011
HF: admission vs HF:discharge: Cohens d: 0.11, permutation test p value = 0.3374

This data shows that the speech rate (rate of speech timing, RST) from word reading test recordings can be used to differentiate decompensating heart failure patients from stable heart failure patients. However, this metric cannot be used to monitor the recovery of patients from the decompensated state through to the recovery state at which patients can be discharged from hospital, and is not as sensitive as the correct word rate. The speech rate was determined by computing a cumulative sum of the number of identified segments in the voice recording over time, and computing the slope of a linear regression model fitted to the cumulative sum data.

Thus, this data shows that by combing effects associated with breathlessness but also fatigue (through a metric that is more sensitive to cognitive abilities while also capturing breathlessness related effects), a more sensitive biomarker for heart failure status can be obtained.

Breathing % in word reading test (combined color word reading tests, Fig. 15A'):
HF:admission (average ± standard deviation): 41.9 ± 8.2, n = 25
HF:discharge (average ± standard deviation): 42.0 ± 7.5, n = 25
OP:stable (average ± standard deviation): 29.6 ± 5.1, n = 19
HF:admission vs OP:stable: Cohens d: 1.71, permutation test p value = 0.0000
HF:discharge vs OP:stable: Cohens d: 1.85, permutation test p value = 0.0000
HF: admission vs HF:discharge: Cohens d: -0.02, permutation test p value = 0.4767

Breathing % in word reading test (color word reading test, interference condition, Fig. 15A):
HF:admission vs OP:stable: Cohen's d=1.75, permutation test p-value=0.0000
HF:discharge vs OP:stable: Cohen's d=1.77, permutation test p-value=0.0000
HF: admission vs HF:discharge: Cohens d: -0.00, permutation test p value = 0.4973

Unvoicing/voicing ratio in word reading test (combined color word reading tests, Fig. 15B'):
HF:admission (average ± standard deviation): 0.8 ± 0.3, n = 25
HF:discharge (average ± standard deviation): 0.8 ± 0.2, n = 25
OP:stable (average ± standard deviation): 0.4 ± 0.1, n = 19
HF:admission vs OP:stable: Cohens d: 1.41, permutation test p value = 0.0000
HF:discharge vs OP:stable: Cohens d: 1.70, permutation test p value = 0.0000
HF: admission vs HF:discharge: Cohens d: 0.02, permutation test p value = 0.4760

Unvoicing/voicing ratio in word reading test (color word reading test, interference condition, Fig. 15B):
HF:admission vs OP:stable: Cohen's d=1.31, permutation test p-value=0.0000
HF:discharge vs OP:stable: Cohen's d=1.52, permutation test p-value=0.0000
HF: admission vs HF:discharge: Cohens d: 0.03, permutation test p value = 0.4659

The data above shows that the breathing % and unvoicing/voicing ratio from a word reading test recording can be used to differentiate decompensating heart failure patients from stable heart failure patients. These metrics are both very sensitive to the difference between decompensating heart failure patients and stable heart failure patients, but do not vary significantly between admission and discharge from hospital. Note that these two metrics are related through a quadratic relationship.

Thus, together the metrics above can be used to identify a decompensating heart failure patient or a stable heart failure patient (using any of the correct word rate, the breathing % and the voicing/unvoicing ratio), to identify a decompensating heart failure patient that requires hospitalisation (using the correct word rate), to identify a heart failure patient that has recovered enough to be discharged from hospital but is not yet stable (and hence may require further/more extensive monitoring, using the correct word rate optionally in combination with the breathing % and/or the unvoicing/voicing ratio), and to monitor recovery during and after hospitalisation (using the correct word rate during hospitalisation, and any of the correct word rate, the breathing % and the voicing/unvoicing ratio after hospitalisation).

The biomarkers from the word reading test were also compared to corresponding metrics obtained from the number counting and reading tests. The results of these are shown on **Figures 15E-J** and **Figure 18****.** The characteristics of the data on Figure 15E-J are shown below.
breathing % in the reading task (Fig. 15E):
HF:admission vs OP:stable: Cohen's d=1.54, permutation test p-value=0.0000
HF:discharge vs OP:stable: Cohen's d=1.28, permutation test p-value=0.0000
HF: admission vs HF:discharge: Cohens d: 0.09, permutation test p value = 0.3810

Unvoicing/voicing ratio in the reading task (Fig. 15F):
HF:admission vs OP:stable: Cohen's d=1.35, permutation test p-value=0.0000
HF:discharge vs OP:stable: Cohen's d=0.89, permutation test p-value=0.0002
HF: admission vs HF:discharge: Cohens d: -0.03, permutation test p value = 0.4734

Speech rate (number of words per second) in the reading task (Fig. 15G):
HF:admission vs OP:stable: Cohen's d=-1.60, permutation test p-value=0.0000
HF:discharge vs OP:stable: Cohen's d=-0.64, permutation test p-value=0.0190
HF: admission vs HF:discharge: Cohens d: -0.40, permutation test p value = 0.0848
breathing % in the reverse counting task (Fig. 15H):
HF:admission vs OP:stable: Cohen's d=-0.24, permutation test p-value=0.2151
HF:discharge vs OP:stable: Cohen's d=-0.21, permutation test p-value=0.2537)
HF: admission vs HF:discharge: Cohens d: -0.05, permutation test p value = 0.4321

Unvoicing/voicing ratio in the reverse counting task (Gig. 15I):
HF:admission vs OP:stable: Cohen's d=-0.19, permutation test p-value=0.2718
HF:discharge vs OP:stable: Cohen's d=-0.26, permutation test p-value=0.2126
HF: admission vs HF:discharge: Cohens d: 0.04, permutation test p value = 0.4472

Speech rate in the reverse counting task (Fig. 15J):
HF:admission vs OP:stable: Cohen's d=0.19, permutation test p-value=0.2754
HF:discharge vs OP:stable: Cohen's d=0.22, permutation test p-value=0.2349
HF: admission vs HF:discharge: Cohens d: 0.01, permutation test p value = 0.4797

The above data shows that the breathing %, unvoicing/voicing ratio and speech rate in the reading test can each be used to differentiate decompensating heart failure patients from stable heart failure patients. However, none of these metrics can be used to differentiate decompensating heart failure patients on admission from decompensating heart failure patients on discharge from hospital. Further, due to the nature of the task, no metric equivalent to the correct word rate can be obtained using this test. As such, the set of biomarkers derived from the reading test are not as sensitive as those derived from the word reading test.

The data further shows that breathing %, unvoicing/voicing ratio and speech rate in the number counting tests cannot be used to differentiate decompensating heart failure patients from stable heart failure patients. As such, the set of biomarkers derived from the number counting tests are not as sensitive as those derived from the word reading test.

**Table 2. Analysis of speech test recordings from heart failure patients.**

| **Test** | **Group** | **Metric** | **Value (average ± standard deviation)** |
|---|---|---|---|
| word reading | OP:stable | pitch | 144.3 ± 34.3, n = 19 |
| word reading | HF:admission | pitch | 150.0 ± 34.1, n = 25 |
| word reading | HF:discharge | pitch | 147.4 ± 37.6, n = 25 |
| color word reading | OP:stable | pitch | 143.5 ± 32.9, n = 19 |
| color word reading | HF:admission | pitch | 150.7 ± 34.5, n = 25 |
| color word reading | HF:discharge | pitch | 148.2 ± 38.6, n = 25 |
| word reading | OP:stable | correct word rate | 1.9 ± 0.3, n = 19 |
| word reading | HF:admission | correct word rate | 1.6 ± 0.4, n = 25 |
| word reading | HF:discharge | correct word rate | 1.7 ± 0.4, n = 25 |
| color word reading | OP:stable | correct word rate | 1.9 ± 0.2, n = 19 |
| color word reading | HF:admission | correct word rate | 1.5 ± 0.4, n = 25 |
| color word reading | HF:discharge | correct word rate | 1.6 ± 0.4, n = 25 |
| word reading | OP:stable | speech rate | 2.1 ± 0.3, n = 19 |
| word reading | HF:admission | speech rate | 1.8 ± 0.3, n = 25 |
| word reading | HF:discharge | speech rate | 1.8 ± 0.3, n = 25 |
| color word reading | OP:stable | speech rate | 2.0 ± 0.2, n = 19 |
| color word reading | HF:admission | speech rate | 1.8 ± 0.3, n = 25 |
| color word reading | HF:discharge | speech rate | 1.7 ± 0.4, n = 25 |
| word reading | OP:stable | unvoicing/voicing | 0.4 ± 0.1, n = 19 |
| word reading | HF:admission | unvoicing/voicing | 0.7 ± 0.2, n = 25 |
| word reading | HF:discharge | unvoicing/voicing | 0.7 ± 0.2, n = 25 |
| color word reading | OP:stable | unvoicing/voicing | 0.4 ± 0.1, n = 19 |
| color word reading | HF:admission | unvoicing/voicing | 0.8 ± 0.4, n = 25 |
| color word reading | HF:discharge | unvoicing/voicing | 0.8 ± 0.3, n = 25 |
| word reading | OP:stable | breathing % | 29.9 ± 5.8, n = 19 |
| word reading | HF:admission | breathing % | 40.5 ± 8.0, n = 25 |
| word reading | HF:discharge | breathing % | 40.8 ± 7.2, n = 25 |
| color word reading | OP:stable | breathing % | 29.4 ± 4.9, n = 19 |
| color word reading | HF:admission | breathing % | 43.2 ± 9.3, n = 25 |
| color word reading | HF:discharge | breathing % | 43.2 ± 9.2, n = 25 |
| number counting | OP:stable | pitch | 142.4 ± 25.6, n = 19 |
| number counting | HF:admission | pitch | 153.4 ± 36.5, n = 25 |
| number counting | HF:discharge | pitch | 152.4 ± 39.1, n = 25 |
| reverse number | OP:stable | pitch | 144.1 ± 34.8, n = 19 |
| reverse number | HF:admission | pitch | 157.2 ± 34.3, n = 25 |
| reverse number | HF:discharge | pitch | 151.5 ± 37.9, n = 25 |
| number counting | OP:stable | speech rate | 1.9 ± 0.4, n = 19 |
| number counting | HF:admission | speech rate | 1.9 ± 0.4, n = 25 |
| number counting | HF:discharge | speech rate | 2.2 ± 0.6, n = 25 |
| reverse number | OP:stable | speech rate | 1.9 ± 0.4, n = 19 |
| reverse number | HF:admission | speech rate | 2.0 ± 0.5, n = 25 |
| reverse number | HF:discharge | speech rate | 2.0 ± 0.3, n = 25 |
| number counting | OP:stable | unvoicing/voicing | 0.9 ± 0.3, n = 19 |
| number counting | HF:admission | unvoicing/voicing | 0.8 ± 0.5, n = 25 |
| number counting | HF:discharge | unvoicing/voicing | 0.6 ± 0.3, n = 25 |
| reverse number | OP:stable | unvoicing/voicing | 0.8 ± 0.4, n = 19 |
| reverse number | HF:admission | unvoicing/voicing | 0.7 ± 0.4, n = 25 |
| reverse number | HF:discharge | unvoicing/voicing | 0.7 ± 0.3, n = 25 |
| number counting | OP:stable | breathing % | 45.3 ± 9.1, n = 19 |
| number counting | HF:admission | breathing % | 41.0 ± 13.2, n = 25 |
| number counting | HF:discharge | breathing % | 37.2 ± 10.1, n = 25 |
| reverse number | OP:stable | breathing % | 41.1 ± 10.7, n = 19 |
| reverse number | HF:admission | breathing % | 38.2 ± 12.2, n = 25 |
| reverse number | HF:discharge | breathing % | 38.7 ± 10.5, n = 25 |
| reading text | OP:stable | pitch | 151.4 ± 27.2, n = 19 |
| reading text | HF:admission | pitch | 152.6 ± 35.0, n = 25 |
| reading text | HF:discharge | pitch | 154.9 ± 33.8, n = 25 |
| reading text | OP:stable | speech rate | 2.6 ± 0.2, n = 19 |
| reading text | HF:admission | speech rate | 2.2 ± 0.3, n = 25 |
| reading text | HF:discharge | speech rate | 2.3 ± 0.5, n = 25 |
| reading text | OP:stable | unvoicing/voicing | 0.5 ± 0.2, n = 19 |
| reading text | HF:admission | unvoicing/voicing | 0.8 ± 0.3, n = 25 |
| reading text | HF:discharge | unvoicing/voicing | 0.8 ± 0.5, n = 25 |
| reading text | OP:stable | breathing % | 31.5 ± 8.5, n = 19 |
| reading text | HF:admission | breathing % | 43.6 ± 7.1, n = 25 |
| reading text | HF:discharge | breathing % | 42.9 ± 8.8, n = 25 |

**Table 3. Analysis of speech test recordings from heart failure patients - comparisons between groups (p value=permutation test p value, HF:A=HF:admission, HF:D=HF:discharge).**

| **Comparison** | **Condition** | **Metric** | **Cohen's d / p value** |
|---|---|---|---|
| HF:A vs OP:stable | number counting | pitch | 0.33 / 0.1407 |
| HF:A vs OP:stable | number counting | correct word rate | - |
| HF:A vs OP:stable | number counting | unvoicing/voicing | -0.21 / 0.2419 |
| HF:A vs OP:stable | number counting | breathing % | -0.36 / 0.1177 |
| HF:A vs OP:stable | number counting | speech rate | 0.12 / 0.3519 |
| HF:A vs OP:stable | reverse number | pitch | 0.37 / 0.1195 |
| HF:A vs OP:stable | reverse number | correct word rate | - |
| HF:A vs OP:stable | reverse number | unvoicing/voicing | -0.19 / 0.2718 |
| HF:A vs OP:stable | reverse number | breathing % | -0.24 / 0.2151 |
| HF:A vs OP:stable | reverse number | speech rate | 0.19 / 0.2754 |
| HF:A vs OP:stable | reading text | pitch | 0.04 / 0.4549 |
| HF:A vs OP:stable | reading text | correct word rate | - |
| HF:A vs OP:stable | reading text | unvoicing/voicing | 1.35 / 0.0000 |
| HF:A vs OP:stable | reading text | breathing % | 1.54 / 0.0000 |
| HF:A vs OP:stable | reading text | speech rate | -1.60 / 0.0000 |
| HF:A vs OP:stable | color word reading | pitch | 0.21 / 0.2481 |
| HF:A vs OP:stable | color word reading | correct word rate | -1.14 / 0.0001 |
| HF:A vs OP:stable | color word reading | unvoicing/voicing | 1.31 / 0.0000 |
| HF:A vs OP:stable | color word reading | breathing % | 1.75 / 0.0000 |
| HF:A vs OP:stable | color word reading | speech rate | -0.89 / 0.0019 |
| HF:A vs OP:stable | word reading | pitch | 0.16 / 0.3027 |
| HF:A vs OP:stable | word reading | correct word rate | -0.85 / 0.0042 |
| HF:A vs OP:stable | word reading | unvoicing/voicing | 1.33 / 0.0000 |
| HF:A vs OP:stable | word reading | breathing % | 1.45 / 0.0000 |
| HF:A vs OP:stable | word reading | speech rate | -0.81 / 0.0060 |
| HF:D vs OP:stable | number counting | pitch | 0.29 / 0.1774 |
| HF:D vs OP:stable | number counting | correct word rate | - |
| HF:D vs OP:stable | number counting | unvoicing/voicing | -0.76 /0.0076 |
| HF:D vs OP:stable | number counting | breathing % | -0.82 /0.0053 |
| HF:D vs OP:stable | number counting | speech rate | 0.581 / 0.0320 |
| HF:D vs OP:stable | reverse number | pitch | 0.20 / 0.2584 |
| HF:D vs OP:stable | reverse number | correct word rate | - |
| HF:D vs OP:stable | reverse number | unvoicing/voicing | -0.26 /0.2126 |
| HF:D vs OP:stable | reverse number | breathing % | -0.21 /0.2537 |
| HF:D vs OP:stable | reverse number | speech rate | 0.22 /0.2349 |
| HF:D vs OP:stable | reading text | pitch | 0.11 /0.3721 |
| HF:D vs OP:stable | reading text | correct word rate | - |
| HF:D vs OP:stable | reading text | unvoicing/voicing | 0.89 /0.0002 |
| HF:D vs OP:stable | reading text | breathing % | 1.28 /0.0000 |
| HF:D vs OP:stable | reading text | speech rate | -0.64 /0.0190 |
| HF:D vs OP:stable | color word reading | pitch | 0.13 /0.3449 |
| HF:D vs OP:stable | color word reading | correct word rate | -0.87 /0.0035 |
| HF:D vs OP:stable | color word reading | unvoicing/voicing | 1.52 /0.0000 |
| HF:D vs OP:stable | color word reading | breathing % | 1.77 /0.0000 |
| HF:D vs OP:stable | color word reading | speech rate | -0.98 /0.0011 |
| HF:D vs OP:stable | word reading | pitch | 0.08 /0.3998 |
| HF:D vs OP:stable | word reading | correct word rate | -0.65 /0.0170 |
| HF:D vs OP:stable | word reading | unvoicing/voicing | 1.54 /0.0000 |
| HF:D vs OP:stable | word reading | breathing % | 1.61 /0.0000 |
| HF:D vs OP:stable | word reading | speech rate | -0.80 /0.0045 |

The data on **Figure 16** shows the vocal pitch estimates from the word reading tests (average of estimates from color word reading tests in the interference and coherent condition, the error bar indicating the standard deviation between normal condition and interference condition) for the decompensating heart failure patients (on the left, shown as two points: on admission (black) and on discharge (dark grey)), and for the stable heart failure outpatients (on the right, light grey points). The data on **Figure 17** shows the vocal pitch estimates (average of estimates from color word reading tests in the interference and coherent condition) for decompensating heart failure patients on different days from admission (enrolment). The data shows that for most decompensating heart failure patients, recovery in hospital is associated with a change in pitch estimate from a word reading test. However, the particular trend may differ between heart failure patients, with some patients showing increasing pitch during hospitalisation, and others showing a decreasing pitch. Note that most patients showed a decreasing pitch during recovery. Thus, the vocal pitch derived from word reading tests can be used to monitor recovery during heart failure hospitalisation.

The data on **Figure 18** shows Bland-Altman plots that assess the level of agreement between pitch measurements in a number counting test and in a reverse number counting test for 48 heart failure patients (B, total of 161 pairs of recordings analysed), and between pitch measurements in a Stroop word reading test (color words, coherent condition) and a Stroop color reading test (color words, interference condition) for 48 heart failure patients (A, total of 162 pairs of recordings analysed). Each data point shows the difference between the average pitch (Hz) estimated using the respective tests. The dashed lines show the average difference (middle line) and the ±1.96 standard deviations (SD) interval. The repeatability is quantified using the consensus report (CR=2*SD), and is 27.76 for the number counting tests and 17.64 for the word reading tests. Smaller values of the CR are indicative of higher levels of repeatability. Thus, this data shows that pitch estimates obtained from voice recordings of word reading tests are more reliable (less variable) than pitch estimates obtained from voice recordings of other reading tests such as e.g. number counting tests. The inventors believe that this may be at least in part because the word reading tests are less influenced by effects associated with the subject getting used to the sequence of words and/or the pitch being affected by cognitive content of a text being read. Further, the words used in this example (color words) advantageously contain single vowels within the context of a word, where the pitch associated with the manner in which the same subject utters the vowel within the word is less likely to be affected by external factors than e.g. vowel repeating tests that are commonly used to evaluate pitch. In other words, the use of a limited set of words that contain sounds that are suitable for pitch estimation, but where these sounds are present within the normalizing context of a word, but without the biasing context of a set of a sentence that has cognitive content or a logical connection (all of which can influence voice pitch and hence act as confusing factor when the pitch is to be used as a biomarker) advantageously result in a more reliable voice biomarker.

A similar conclusion applies (to various extents) to the breathing %, speech rate and unvoicing/voicing ratio metrics, which are more consistent when derived from a word reading test vs color word reading test (i.e. color words read in the coherent vs interference condition; breathing % CR=13.06, N=162; speech rate CR=0.50, N=162; unvoicing/voicing CR=0.56, N=162) than when derived from a number counting vs reverse number counting task (breathing % CR=19.39, N=161; speech rate: CR=1.00, N=161; unvoicing/voicing CR=0.60, N=161).

Finally, the potential of the present method to diagnose or monitor COVID-19 status was also evaluated. The present biomarkers were obtained as explained in a cohort of 10 healthy volunteers and in a patient diagnosed with COVID-19. The biomarkers were measured in the patient diagnosed with COVID-19 on a plurality of days comprising a day where the patient did not yet show any symptoms (, and a plurality of days comprising a period where the patient only reported mild fatigue or dyspnea. The results of this analysis are shown on **Figure 19****.** This data shows that the voice pitch estimate for the patient with very mild or even no symptoms was different (significantly higher) from the voice pitch estimate for the healthy volunteers cohort, and that the voice pitch estimate for the patient with mild symptoms was also different from the voice pitch estimate from the recovered patient with no symptoms .

Thus the data on Figure 19 suggests that the voice pitch biomarker can be used to identify patients with COVID-19 even if they are not symptomatic, and to monitor progression of the disease (such as e.g. recovery).

### References

1. Maor et al. (2018). Vocal Biomarker Is Associated With Hospitalization and Mortality Among Heart Failure Patients. Journal of the American Heart Association. 2020;9:e013359.
2. Laguarta et al. (2020). COVID-19 Artificial Intelligence Diagnosis using only Cough Recordings. Open Journal of Engineering in Medicine and Biology. DOI: 10.1109/OJEMB.202.3026928.
3. Mauch and Dixon (2014)
4. Murton et al. (2017). Acoustic speech analysis of patients with decompensated heart failure: A pilot study. J. Acoust. Soc. Am. 142 (4).
5. Saeed et al. (2018), Study of voice disorders in patients with bronchial asthmas and chronic obstructive pulmonary disease. Egyptian Journal of Bronchology, Vol. 12, No. 1, pp 20-26.
6. Camacho and Harris (2008). A sawtooth waveform inspired pitch estimator for speech and music. The Journal of the Acoustical Society of America, 124(3), pp. 1638-1652.
7. Ardaillon and Roebel (2019). Fully-Convolutional Network for Pitch Estimation of Speech Signals. Insterspeech 2019, Sep 2019, Graz, Austria. ff10.21437/Interspeech.2019-2815ff. ffhal-02439798
8. Kim et al. (2018). CREPE: A Convolutional Representation for Pitch Estimation. 2018 IEEE International Conference on Acoustics, Speech and Signal Processing (ICASSP), Calgary, AB, 2018, pp. 161-165, doi: 10.1109/ICASSP.2018.8461329
9. Kenneth, D.J., Temporal constraints and characterising syllable structuring. Phonetic Interpretation: Papers in Laboratory Phonology VI., 2003: p. 253-268.
10. Xie, Z.M. and P. Niyogi, Robust Acoustic-Based Syllable Detection. Interspeech 2006 and 9th International Conference on Spoken Language Processing, Vols 1-5, 2006: p. 1571-1574.
11. Wang, D. and S.S. Narayanan, Robust speech rate estimation for spontaneous speech. leee Transactions on Audio Speech and Language Processing, 2007. 15(8): p. 2190-2201.
12. Rusz, J., et al., Quantitative assessment of motor speech abnormalities in idiopathic rapid eye movement sleep behaviour disorder. Sleep Med, 2016. 19: p. 141-7.
13. Bock, S. and G. Widmer, Maximum filter vibrato suppression for onset detection. 16th International Conference on Digital Audio Effects, Maynooth, Ireland, 2013.
14. Davis, S.B. and P. Mermelstein, Comparison of Parametric Representations for Monosyllabic Word Recognition in Continuously Spoken Sentences. leee Transactions on Acoustics Speech and Signal Processing, 1980. 28(4): p. 357-366.
15. Huang, X., A. Acero, and H. Hon, Spoken Language Processing: A guide to theory, algorithm, and system development. Prentice Hall, 2001.
16. Rusz, J., et al., Automatic Evaluation of Speech Rhythm Instability and Acceleration in Dysarthrias Associated with Basal Ganglia Dysfunction. Front Bioeng Biotechnol, 2015. 3: p. 104.
17. Lloyd, S.P., Least-Squares Quantization in Pcm. leee Transactions on Information Theory, 1982. 28(2): p. 129-137.
18. Smith, T.F. and M.S. Waterman, Identification of common molecular subsequences. J Mol Biol, 1981. 147(1): p. 195-7.
19. Hlavnicka, J., et al., Automated analysis of connected speech reveals early biomarkers of Parkinson's disease in patients with rapid eye movement sleep behaviour disorder. Sci Rep, 2017. 7(1): p. 12.
20. Stroop, J.R., Studies of interference in serial verbal reactions. Journal of Experimental Psychology, 1935. General(18): p. 19.
21. McFee, B. et al., librosa: Audio and Music Signal Analysis in Python. PROC. OF THE 14th PYTHON IN SCIENCE CONF. (SCIPY 2015).
22. James Lyons et al. (2020, January 14). jameslyons/python_speech_features: release v0.6.1 (Version 0.6.1). Zenodo. http://doi.org/10.5281/zenodo.3607820

The terms "computer system" includes the hardware, software and data storage devices for embodying a system or carrying out a method according to the above described embodiments. For example, a computer system may comprise a central processing unit (CPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

Unless context dictates otherwise, the descriptions and definitions of the features set out above are not limited to any particular aspect or embodiment of the invention and apply equally to all aspects and embodiments which are described.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof. For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

## Claims

1. A computer-implemented method of assessing the pathological and/or
physiological state of a subject, the method comprising:
obtaining a voice recording from a word-reading test from the subject, wherein the voice recording is from a word-reading test comprising reading a sequence of words drawn from a set of *n* words, wherein the sequence of words are not connected so as to form a sentence and are drawn randomly or pseudo-randomly from the set of n words; and
analysing the voice recording, or a portion thereof, by:
identifying a plurality of segments of the voice recording that correspond to single words or syllables;
determining the value of one or more metrics selected from the breathing percentage, unvoicing/voicing ratio, voice pitch and correct word rate at least in part based on the identified segments;
comparing the value of the one or more metrics with one or more respective reference values.

2. The method of claim 1, wherein identifying segments of the voice recording that correspond to single words or syllables comprises:
obtaining a power Mel-spectrogram of the voice recording;
computing the maximum intensity projection of the Mel spectrogram along the frequency axis; and
defining a segment boundary as the time point where the maximum intensity projection of the Mel spectrogram along the frequency axis crosses a threshold.

3. The method of any preceding claim, wherein determining the value of one or more metrics comprises determining a breathing percentage associated with the recording as the percentage of time in the voice recording that is between the identified segments, or the ratio of the time between the identified segments in the recording and the sum of the time between the identified segments and within identified segments in the recording.

4. The method of any preceding claim, wherein determining the value of one or more metrics comprises determining a unvoicing/voicing ratio associated with the recording as the ratio of the time between the identified segments in the recording and the time within identified segments in the recording.

5. The method of any preceding claim, wherein determining the value of one or more metrics comprises determining a voice pitch associated with the recording by obtaining one or more estimates of the fundamental frequency for each of the identified segments.

6. The method of claim 5, wherein determining the value of the voice pitch comprises obtaining a plurality of estimates of the fundamental frequency for each of the identified segment, and applying a filter to the plurality of estimates to obtain a filtered plurality of estimates, and/or wherein determining the value of the voice pitch comprises obtaining a summarised voice pitch estimate for a plurality of segments, such as e.g. the mean, median or mode of the, optionally filtered, plurality of estimates for the plurality of segments.

7. The method of any preceding claim, wherein determining the value of one or more metrics comprises determining the correct word rate associated with the voice recording by computing the ratio of the number of identified segments corresponding to correctly read words divided by the time duration between the start of the first identified segment and the end of the last identified segment, or by computing a cumulative sum of the number of identified segments corresponding to correctly read words in the voice recording overtime, and computing the slope of a linear regression model fitted to the cumulative sum data.

8. The method of any preceding claim, wherein determining the value of one or more metrics comprises determining a correct word rate associated with the recording, wherein determining the correct word rate comprises:
computing one or more Mel-frequency cepstral coefficients**,** MFCCs, for each of the identified segments to obtain a plurality of vectors of values, each vector being associated with a segment, optionally wherein computing one or more MFCCs to obtain a vector of values for a segment comprises: computing a set of i MFCCs for each frame of the segment for each *i* and obtaining a set of *j* values for the segment by interpolation, preferably linear interpolation, to obtain a vector of *ixj* values for the segment;
clustering the plurality of vector of values into n clusters, wherein each cluster has n possible labels corresponding to each of the n words, optionally wherein clustering the plurality of vector of values into n clusters is performed using k-means;
for each of the n! permutations of labels, predicting a sequence of words in the voice recording using the labels associated with the clustered vectors of values, and performing a sequence alignment between the predicted sequence of words and the sequence of words used in the word reading test, optionally wherein the sequence alignment step is performed using a local sequence alignment algorithm, preferably the Smith-Waterman algorithm; and
selecting the labels that result in the best alignment, wherein matches in the alignment correspond to correctly read words in the voice recording, optionally wherein performing a sequence alignment comprises obtaining an alignment score and the best alignment is the alignment with the highest alignment score.

9. The method of any preceding claim, wherein identifying segments of the voice recording that correspond to single words or syllables further comprises:
(i) normalising the power Mel-spectrogram of the voice recording, preferably against the frame that has the highest energy in the recording; and/or
(ii) performing onset detection for at least one of the segments by computing a spectral flux function over the Mel-spectrogram of the segment, and
defining a further boundary whenever an onset is detected within a segment, thereby forming two new segments; and/or
(iii) excluding segments that represent erroneous detections by computing one or more Mel-frequency cepstral coefficients, MFCCs, for the segments to obtain a plurality of vectors of values, each vector being associated with a segment, and applying an outlier detection method to the plurality of vectors of values; and/or
(iv) excluding segments that represent erroneous detections by removing segments shorter than a predetermined threshold and/or with mean relative energy below a predetermined threshold.

10. The method of any preceding claim, wherein the n words:
(i) are monosyllabic or disyllabic, and/or
(ii) each include one or more vowels that are internal to the respective word; and/or
(iii) each include a single emphasized syllable; and/or
(iv) are color words, optionally wherein the words are displayed in a single color in the word reading test, or wherein the words are displayed in a color independently chosen from a set of m colors in the word reading test.

11. The method of any preceding claim, wherein obtaining a voice recording from a word-reading test from the subject comprises:
(i) obtaining a voice recording from a first word-reading test, and a voice recording from a second word-reading test, wherein the word-reading tests comprise reading a sequence of words drawn from a set of n words that are color words, wherein the words are displayed in a single color in the first word reading test, and in a color independently chosen from a set of*m* colors in the second word reading test, optionally wherein the sequence of words in the second word reading test is the same as the sequence of words in the first word reading test; and/or
(ii) receiving a word recording from a computing device associated with the subject, optionally wherein obtaining a voice recording further comprises causing a computing device associated with the subject to display the sequence of words, and/or to record a voice recording and/or to emit a fixed length tone, then to record a voice recording.

12. The method of any preceding claim, wherein the sequence of words comprises a predetermined number of words, optionally at least 20, at least 30 or about 40 words.

13. A computer-implemented method of monitoring a subject with heart failure, or diagnosing a subject as having worsening of heart failure or decompensated heart failure, the method comprising:
assessing the pathological and/or physiological state of the subject using the method of any one of the preceding claims, wherein the one or more respective reference values are values for the same metrics previously obtained for the same subject or wherein the one or more respective reference values comprise values for the same metrics associated with decompensated heart failure patients, stable heart failure patients and/or recovering decompensated heart failure patients.

14. A computer-implemented method of monitoring a subject that has been diagnosed as having or being at risk of having a condition associated with dyspnea and/or fatigue, or assessing the level of dyspnea and/or fatigue in a subject, the method comprising:
assessing the pathological and/or physiological state of the subject using the method of any one of claims 1 to 12, wherein the one or more respective reference values are values for the same metrics previously obtained for the same subject or wherein the one or more respective reference values comprise values for the same metrics associated with patients having the condition and/or values for the same metrics associated with patients that do not have the condition.

15. A system comprising:
at least one processor; and
at least one non-transitory computer readable medium containing instructions that, when executed by the at least one processor, cause the at least one processor to perform operations comprising the operations described in any of claims 1 to 14.

## Patentansprüche

1. Computerimplementiertes Verfahren zur Beurteilung des pathologischen und/oder physiologischen Zustands eines Subjekts, wobei das Verfahren Folgendes umfasst:
Erhalten einer Sprachaufzeichnung eines Wortlesetests von dem Subjekt, wobei die Sprachaufzeichnung von einem Wortlesetest stammt, der das Lesen einer Sequenz von Wörtern umfasst, die aus einer Gruppe von n Wörtern entnommen sind, wobei die Sequenz von Wörtern nicht zusammenhängend ist, um einen Satz zu bilden, und zufällig oder pseudozufällig aus der Gruppe von n Wörtern entnommen ist; und
Analysieren der Sprachaufzeichnung oder eines Teils davon durch:
Identifizieren einer Vielzahl von Segmenten der Sprachaufzeichnung, die einzelnen Wörtern oder Silben entsprechen;
Bestimmen des Werts von einer oder mehreren Messgrößen, die aus dem Atmungsprozentsatz, dem Stimmlos/Stimmhaft-Verhältnis, der Stimmtonhöhe und der korrekten Wortrate ausgewählt sind, zumindest teilweise basierend auf den identifizierten Segmenten;
Vergleichen des Werts der einen oder mehreren Messgrößen mit einem oder mehreren jeweiligen Referenzwerten.

2. Verfahren nach Anspruch 1, wobei das Identifizieren von Segmenten der Sprachaufzeichnung, die einzelnen Wörtern oder Silben entsprechen, Folgendes umfasst:
Erhalten eines Leistungs-Mel-Spektrogramms der Sprachaufzeichnung;
Berechnen der maximalen Intensitätsprojektion des Mel-Spektrogramms entlang der Frequenzachse; und
Definieren einer Segmentgrenze als den Zeitpunkt, an dem die maximale Intensitätsprojektion des Mel-Spektrogramms entlang der Frequenzachse einen Schwellenwert überschreitet.

3. Verfahren nach einem der vorhergehenden Ansprüche, wobei das Bestimmen des Werts von einer oder mehreren Messgrößen das Bestimmen eines Atmungsprozentsatzes, der mit der Aufzeichnung assoziiert ist, als den Prozentsatz der Zeit in der Sprachaufzeichnung, die zwischen den identifizierten Segmenten liegt, oder das Verhältnis der Zeit zwischen den identifizierten Segmenten in der Aufzeichnung und der Summe der Zeit zwischen den identifizierten Segmenten und innerhalb der identifizierten Segmente in der Aufzeichnung umfasst.

4. Verfahren nach einem der vorhergehenden Ansprüche, wobei das Bestimmen des Werts von einer oder mehreren Messgrößen das Bestimmen des Stimmlos/Stimmhaft-Verhältnisses, das mit der Aufzeichnung assoziiert ist, als das Verhältnis der Zeit zwischen den identifizierten Segmenten in der Aufzeichnung und der Zeit innerhalb der identifizierten Segmente in der Aufzeichnung umfasst.

5. Verfahren nach einem der vorhergehenden Ansprüche, wobei das Bestimmen des Werts von einer oder mehreren Messgrößen das Bestimmen einer Stimmtonhöhe, die mit der Aufzeichnung assoziiert ist, durch Erhalten einer oder mehrerer Schätzungen der Grundfrequenz für jedes der identifizierten Segmente umfasst.

6. Verfahren nach Anspruch 5, wobei das Bestimmen des Werts der Stimmtonhöhe das Erhalten einer Vielzahl von Schätzungen der Grundfrequenz für jedes der identifizierten Segmente und das Anwenden eines Filters auf die Vielzahl von Schätzungen umfasst, um eine gefilterte Vielzahl von Schätzungen zu erhalten, und/oder wobei das Bestimmen des Werts der Stimmtonhöhe das Erhalten einer zusammengefassten Stimmtonhöhenschätzung für eine Vielzahl von Segmenten, wie z. B. den Mittelwert, Median oder Modus der gegebenenfalls gefilterten Vielzahl von Schätzungen für die Vielzahl von Segmenten, umfasst.

7. Verfahren nach einem der vorhergehenden Ansprüche, wobei das Bestimmen des Werts von einer oder mehreren Messgrößen das Bestimmen der korrekten Wortrate, die mit der Sprachaufzeichnung assoziiert ist, durch Berechnen des Verhältnisses der Anzahl von identifizierten Segmenten, die korrekt gelesenen Wörtern entsprechen, dividiert durch die Zeitdauer zwischen dem Anfang des ersten identifizierten Segments und dem Ende des letzten identifizierten Segments oder durch Berechnen einer kumulativen Summe der Anzahl von identifizierten Segmenten, die korrekt gelesenen Wörtern in der Sprachaufzeichnung entsprechen, über die Zeit und Berechnen der Steigung eines linearen Regressionsmodells, das an die kumulativen Summendaten angepasst ist, umfasst.

8. Verfahren nach einem der vorhergehenden Ansprüche, wobei das Bestimmen des Werts von einer oder mehreren Messgrößen das Bestimmen einer korrekten Wortrate, die mit der Aufzeichnung assoziiert ist, umfasst, wobei das Bestimmen der korrekten Wortrate Folgendes umfasst:
Berechnen eines oder mehrerer Mel-Frequenz-Cepstralkoeffizienten, MFCCs, für jedes der identifizierten Segmente, um eine Vielzahl von Vektoren von Werten zu erhalten, wobei jeder Vektor mit einem Segment assoziiert ist, gegebenenfalls wobei das Berechnen eines oder mehrerer MFCCs, um einen Vektor von Werten für ein Segment zu erhalten, Folgendes umfasst: Berechnen einer Gruppe von *i* MFCCs für jeden Rahmen des Segments für jedes *i* und Erhalten einer Gruppe von *j* Werten für das Segment durch Interpolation, vorzugsweise lineare Interpolation, um einen Vektor von *ixj* Werten für das Segment zu erhalten;
Clustern der Vielzahl von Vektoren von Werten in *n* Cluster, wobei jedes Cluster *n* mögliche Labels aufweist, die jedem der n Wörter entsprechen, wobei das Clustern der Vielzahl von Vektoren von Werten in n Cluster gegebenenfalls unter Verwendung von k-Mitteln durchgeführt wird;
Vorhersagen einer Sequenz von Wörtern in der Sprachaufzeichnung unter Verwendung der Labels, die mit den geclusterten Vektoren von Werten assoziiert sind, und Durchführen einer Sequenzausrichtung zwischen der vorhergesagten Sequenz von Wörtern und der Sequenz von Wörtern, die in dem Wortlesetest verwendet werden, für jede der n! Permutationen von Labels, wobei der Sequenzausrichtungsschritt gegebenenfalls unter Verwendung eines lokalen Sequenzausrichtungsalgorithmus, vorzugsweise des Smith-Waterman-Algorithmus, durchgeführt wird; und
Auswählen der Labels, die zu der besten Ausrichtung führen, wobei Übereinstimmungen in der Ausrichtung korrekt gelesenen Wörtern in der Sprachaufzeichnung entsprechen, wobei das Durchführen einer Sequenzausrichtung gegebenenfalls das Erhalten einer Ausrichtungsbewertung umfasst und die beste Ausrichtung die Ausrichtung mit der höchsten Ausrichtungsbewertung ist.

9. Verfahren nach einem der vorhergehenden Ansprüche, wobei das Identifizieren von Segmenten der Sprachaufzeichnung, die einzelnen Wörtern oder Silben entsprechen, ferner Folgendes umfasst:
(i) Normalisieren des Leistungs-Mel-Spektrogramms der Sprachaufzeichnung, vorzugsweise gegen den Rahmen, der die höchste Energie in der Aufzeichnung aufweist; und/oder
(ii) Durchführen einer Onset-Erkennung für mindestens eines der Segmente durch Berechnen einer spektralen Flussfunktion über das Mel-Spektrogramm des Segments, und
Definieren einer weiteren Grenze, wann immer ein Onset innerhalb eines Segments erkannt wird, wodurch zwei neue Segmente gebildet werden; und/oder
(iii) Ausschließen von Segmenten, die fehlerhafte Erkennungen darstellen, durch Berechnen eines oder mehrerer Mel-Frequenz-Cepstralkoeffizienten, MFCCs, für die Segmente, um eine Vielzahl von Vektoren von Werten zu erhalten, wobei jeder Vektor mit einem Segment assoziiert ist, und Anwenden eines Ausreißer-Erkennungsverfahrens auf die Vielzahl von Vektoren von Werten; und/oder
(iv) Ausschließen von Segmenten, die fehlerhafte Erkennungen darstellen, durch Entfernen von Segmenten, die kürzer als ein vorbestimmter Schwellenwert sind und/oder eine mittlere relative Energie unter einem vorbestimmten Schwellenwert aufweisen.

10. Verfahren nach einem der vorhergehenden Ansprüche, wobei die n Wörter:
(i) monosyllabisch oder disyllabisch sind und/oder
(ii) jeweils einen oder mehrere Vokale einschließen, die im jeweiligen Wort enthalten sind; und/oder
(iii) jeweils eine einzelne betonte Silbe einschließen; und/oder
(iv) Farbwörter sind, wobei die Wörter gegebenenfalls in einer einzelnen Farbe in dem Wortlesetest angezeigt werden, oder wobei die Wörter in einer Farbe angezeigt werden, die unabhängig aus einer Gruppe von m Farben in dem Wortlesetest ausgewählt ist.

11. Verfahren nach einem der vorhergehenden Ansprüche, wobei das Erhalten einer Sprachaufzeichnung eines Wortlesetests von dem Subjekt Folgendes umfasst:
(i) Erhalten einer Sprachaufzeichnung eines ersten Wortlesetests und einer Sprachaufzeichnung eines zweiten Wortlesetests, wobei die Wortlesetests das Lesen einer Sequenz von Wörtern umfassen, die aus einer Gruppe von n Wörtern entnommen sind, die Farbwörter sind, wobei die Wörter in dem ersten Wortlesetest in einer einzelnen Farbe und in dem zweiten Wortlesetest in einer Farbe angezeigt werden, die unabhängig aus einer Gruppe von m Farben ausgewählt ist, wobei die Sequenz von Wörtern in dem zweiten Wortlesetest gegebenenfalls die gleiche wie die Sequenz von Wörtern in dem ersten Wortlesetest ist; und/oder
(ii) Empfangen einer Wortaufzeichnung von einem Rechner, der dem Subjekt zugeordnet ist, wobei das Erhalten einer Sprachaufzeichnung gegebenenfalls ferner das Veranlassen umfasst, dass ein Rechner, der dem Subjekt zugeordnet ist, die Sequenz von Wörtern anzeigt und/oder eine Sprachaufzeichnung aufzeichnet und/oder einen Ton mit fester Länge ausgibt und dann eine Sprachaufzeichnung aufzeichnet.

12. Verfahren nach einem der vorhergehenden Ansprüche, wobei die Sequenz von Wörtern eine vorbestimmte Anzahl von Wörtern umfasst, gegebenenfalls mindestens 20, mindestens 30 oder etwa 40 Wörter.

13. Computerimplementiertes Verfahren zum Überwachen eines Subjekts mit Herzinsuffizienz oder zur Diagnose einer Verschlechterung der Herzinsuffizienz oder einer dekompensierten Herzinsuffizienz bei einem Subjekt, wobei das Verfahren Folgendes umfasst:
Beurteilen des pathologischen und/oder physiologischen Zustands des Subjekts unter Verwendung des Verfahrens nach einem der vorhergehenden Ansprüche, wobei der eine oder die mehreren jeweiligen Referenzwerte Werte für dieselben Messgrößen sind, die zuvor für dasselbe Subjekt erhalten wurden, oder wobei der eine oder die mehreren jeweiligen Referenzwerte Werte für dieselben Messgrößen umfassen, die mit Subjekten mit dekompensierter Herzinsuffizienz, Subjekten mit stabiler Herzinsuffizienz und/oder genesenden Subjekten mit dekompensierter Herzinsuffizienz assoziiert sind.

14. Computerimplementiertes Verfahren zur Überwachung eines Subjekts, bei dem eine Erkrankung diagnostiziert wurde oder das Risiko besteht, eine Erkrankung zu haben, die mit Dyspnoe und/oder Fatigue einhergeht, oder zur Beurteilung des Ausmaßes der Dyspnoe und/oder Fatigue bei einem Subjekt, wobei das Verfahren Folgendes umfasst: Beurteilen des pathologischen und/oder physiologischen Zustands des Subjekts unter Verwendung des Verfahrens nach einem der Ansprüche 1 bis 12, wobei der eine oder die mehreren jeweiligen Referenzwerte Werte für dieselben zuvor für dasselbe Subjekt erhaltenen Messgrößen sind, oder wobei der eine oder die mehreren jeweiligen Referenzwerte Werte für dieselben Messgrößen umfassen, die mit Patienten assoziiert sind, die die Erkrankung haben, und/oder Werte für dieselben Messgrößen, die mit Patienten assoziiert sind, die die Erkrankung nicht haben.

15. System, das Folgendes umfasst:
mindestens einen Prozessor; und
mindestens ein nichtflüchtiges computerlesbares Medium, das Anweisungen enthält, die, wenn sie von dem mindestens einen Prozessor ausgeführt werden, den mindestens einen Prozessor veranlassen, Arbeitsschritte durchzuführen, die die in einem der Ansprüche 1 bis 14 beschriebenen Arbeitsschritte umfassen.

## Revendications

1. Procédé mis en œuvre par ordinateur d'évaluation de l'état pathologique et/ou physiologique d'un sujet, le procédé comprenant :
l'obtention d'un enregistrement vocal d'un test de lecture de mots du sujet, dans lequel l'enregistrement vocal provient d'un test de lecture de mots comprenant la lecture d'une séquence de mots tirés d'un ensemble de *n* mots, dans lequel la séquence de mots ne sont pas reliés de sorte à former une phrase et sont tirés de manière aléatoire ou pseudo-aléatoire de l'ensemble de n mots ; et
l'analyse de l'enregistrement vocal, ou d'une partie de celui-ci, par :
identification d'une pluralité de segments de l'enregistrement vocal qui correspondent aux mots ou syllabes uniques ;
détermination de la valeur d'une ou plusieurs mesures choisies parmi le pourcentage de respiration, le rapport dévoisement/voisement, la hauteur de voix et le débit de mots correct basées au moins en partie sur les segments identifiés ;
comparaison de la valeur de la ou des mesures avec une ou plusieurs valeurs de référence respectives.

2. Procédé selon la revendication 1, dans lequel l'identification des segments de l'enregistrement vocal qui correspondent aux mots ou syllabes uniques comprend :
l'obtention d'un spectrogramme Mel de puissance de l'enregistrement vocal ;
le calcul de la projection d'intensité maximale du spectrogramme Mel le long de l'axe des fréquences ; et
la définition d'une limite de segment comme le point temporel où la projection d'intensité maximale du spectrogramme Mel le long de l'axe des fréquences croise un seuil.

3. Procédé selon une quelconque revendication précédente, dans lequel la détermination de la valeur d'une ou plusieurs mesures comprend la détermination d'un pourcentage de respiration associé à l'enregistrement comme le pourcentage de temps dans l'enregistrement vocal qui est entre les segments identifiés, ou le rapport de temps entre les segments identifiés dans l'enregistrement et la somme de temps entre les segments identifiés et au sein des segments identifiés dans l'enregistrement.

4. Procédé selon une quelconque revendication précédente, dans lequel la détermination de la valeur d'une ou plusieurs mesures comprend la détermination d'un rapport dévoisement/voisement associé à l'enregistrement comme le rapport de temps entre les segments identifiés dans l'enregistrement et le temps au sein des segments identifiés dans l'enregistrement.

5. Procédé selon une quelconque revendication précédente, dans lequel la détermination de la valeur d'une ou plusieurs mesures comprend la détermination d'une hauteur de voix associée à l'enregistrement par l'obtention d'une ou plusieurs estimations de la fréquence fondamentale pour chacun des segments identifiés.

6. Procédé selon la revendication 5, dans lequel la détermination de la valeur de la hauteur de voix comprend l'obtention d'une pluralité d'estimations de la fréquence fondamentale pour chacun des segments identifiés, et l'application d'un filtre à la pluralité d'estimations pour obtenir une pluralité d'estimations filtrées, et/ou dans lequel la détermination de la valeur de la hauteur de voix comprend l'obtention d'une estimation de hauteur de voix synthétisée pour une pluralité de segments, telle que par exemple la moyenne, la médiane ou le mode de la pluralité d'estimations, éventuellement filtrées, pour la pluralité de segments.

7. Procédé selon une quelconque revendication précédente, dans lequel la détermination de la valeur d'une ou plusieurs mesures comprend la détermination du débit de mots correct associé à l'enregistrement vocal par le calcul du rapport du nombre de segments identifiés correspondant aux mots lus correctement divisé par la durée entre le début du premier segment identifié et la fin du dernier segment identifié, ou par le calcul d'une somme cumulée du nombre de segments identifiés correspondant aux mots lus correctement dans l'enregistrement vocal au fil du temps, et le calcul de la pente d'un modèle de régression linéaire ajusté aux données de somme cumulée.

8. Procédé selon une quelconque revendication précédente, dans lequel la détermination de la valeur d'une ou plusieurs mesures comprend la détermination d'un débit de mots correct associé à l'enregistrement, dans lequel la détermination du débit de mots correct comprend :
le calcul d'un ou plusieurs coefficients cepstraux de fréquence Mel, MFCC, pour chacun des segments identifiés afin d'obtenir une pluralité de vecteurs de valeurs, chaque vecteur étant associé à un segment, éventuellement dans lequel le calcul d'un ou plusieurs MFCC afin d'obtenir un vecteur de valeurs pour un segment comprend : le calcul d'un ensemble de *i* MFCC pour chaque trame du segment pour chaque *i* et l'obtention d'un ensemble de *j* valeurs pour le segment par interpolation, de préférence interpolation linéaire, afin d'obtenir un vecteur de *ixj* valeurs pour le segment ;
le regroupement de la pluralité de vecteurs de valeurs en n groupes, dans lequel chaque groupe a n étiquettes possibles correspondant à chacun des n mots, éventuellement dans lequel le regroupement de la pluralité de vecteurs de valeurs en n groupes est réalisé à l'aide des k-moyennes ;
pour chacune des n! permutations d'étiquettes, la prédiction d'une séquence de mots dans l'enregistrement vocal à l'aide des étiquettes associées aux vecteurs de valeurs regroupés, et la réalisation d'un alignement de séquences entre la séquence de mots prédite et la séquence de mots utilisée dans le test de lecture de mots, éventuellement dans lequel l'étape d'alignement de séquences est réalisée à l'aide d'un algorithme d'alignement de séquences local, de préférence l'algorithme de Smith-Waterman ; et
la sélection des étiquettes qui conduisent au meilleur alignement, dans lequel les correspondances dans l'alignement correspondent aux mots lus correctement dans l'enregistrement vocal, éventuellement dans lequel la réalisation d'un alignement de séquences comprend l'obtention d'un score d'alignement et le meilleur alignement est l'alignement avec le score d'alignement le plus élevé.

9. Procédé selon une quelconque revendication précédente, dans lequel l'identification des segments de l'enregistrement vocal qui correspondent aux mots ou syllabes uniques comprend en outre :
(i) la normalisation du spectrogramme Mel de puissance de l'enregistrement vocal, de préférence par rapport au cadre qui a l'énergie la plus élevée dans l'enregistrement ; et/ou
(ii) la réalisation d'une détection de début pour au moins l'un des segments par le calcul d'une fonction de flux spectral sur le spectrogramme Mel du segment, et
la définition d'une limite supplémentaire à chaque fois qu'un début est détecté au sein d'un segment, formant ainsi deux nouveaux segments ; et/ou
(iii) l'exclusion des segments qui représentent des détections erronées par le calcul d'un ou plusieurs coefficients cepstraux de fréquence Mel, MFCC, pour les segments afin d'obtenir une pluralité de vecteurs de valeurs, chaque vecteur étant associé à un segment, et l'application d'une méthode de détection des valeurs aberrantes à la pluralité de vecteurs de valeurs ; et/ou
(iv) l'exclusion des segments qui représentent des détections erronées par l'élimination des segments plus courts qu'un seuil prédéterminé et/ou ayant une énergie relative moyenne inférieure à un seuil prédéterminé.

10. Procédé selon une quelconque revendication précédente, dans lequel les n mots :
(i) sont monosyllabiques ou dissyllabiques, et/ou
(ii) comprennent chacun une ou plusieurs voyelles qui sont internes au mot respectif ; et/ou
(iii) comprennent chacun une syllabe mise en évidence unique ; et/ou
(iv) sont des mots colorés, éventuellement dans lequel les mots sont affichés dans une couleur unique dans le test de lecture de mots, ou dans lequel les mots sont affichés dans une couleur choisie indépendamment parmi un ensemble de m couleurs dans le test de lecture de mots.

11. Procédé selon une quelconque revendication précédente, dans lequel l'obtention d'un enregistrement vocal d'un test de lecture de mots du sujet comprend :
(i) l'obtention d'un enregistrement vocal d'un premier test de lecture de mots, et d'un enregistrement vocal d'un second test de lecture de mots, dans lequel les tests de lecture de mots comprennent la lecture d'une séquence de mots tirés d'un ensemble de n mots qui sont des mots colorés, dans lequel les mots sont affichés dans une couleur unique dans le premier test de lecture de mots, et dans une couleur choisie indépendamment parmi un ensemble de *m* couleurs dans le second test de lecture de mots, éventuellement dans lequel la séquence de mots dans le second test de lecture de mots est la même que la séquence de mots dans le premier test de lecture de mots ; et/ou
(ii) la réception d'un enregistrement de mots en provenance d'un dispositif informatique associé au sujet, éventuellement dans lequel l'obtention d'un enregistrement vocal comprend en outre le fait d'amener un dispositif informatique associé au sujet à afficher la séquence de mots, et/ou à enregistrer un enregistrement vocal et/ou à émettre une tonalité de longueur fixe, puis à enregistrer un enregistrement vocal.

12. Procédé selon une quelconque revendication précédente, dans lequel la séquence de mots comprend un nombre prédéterminé de mots, éventuellement au moins 20, au moins 30 ou environ 40 mots.

13. Procédé mis en œuvre par ordinateur de surveillance d'un sujet souffrant d'une insuffisance cardiaque, ou de diagnostic d'aggravation d'une insuffisance cardiaque ou d'une insuffisance cardiaque décompensée chez un sujet, le procédé comprenant :
l'évaluation de l'état pathologique et/ou physiologique du sujet à l'aide du procédé selon l'une quelconque des revendications précédentes, dans lequel la ou les valeurs de référence respectives sont des valeurs pour les mêmes mesures précédemment obtenues pour le même sujet ou dans lequel la ou les valeurs de référence respectives comprennent des valeurs pour les mêmes mesures associées aux patients souffrant d'une insuffisance cardiaque décompensée, aux patients souffrant d'une insuffisance cardiaque stable et/ou aux patients en phase de récupération après une insuffisance cardiaque décompensée.

14. Procédé mis en œuvre par ordinateur de surveillance d'un sujet qui a été diagnostiqué comme présentant ou à risque de présenter une affection associée à une dyspnée et/ou une fatigue, ou d'évaluation du niveau de dyspnée et/ou de fatigue chez un sujet, le procédé comprenant :
l'évaluation de l'état pathologique et/ou physiologique du sujet à l'aide du procédé selon l'une quelconque des revendications 1 à 12, dans lequel la ou les valeurs de référence respectives sont des valeurs pour les mêmes mesures précédemment obtenues pour le même sujet ou dans lequel la ou les valeurs de référence respectives comprennent des valeurs pour les mêmes mesures associées aux patients présentant l'affection et/ou des valeurs pour les mêmes mesures associées aux patients qui ne présentent pas l'affection.

15. Système comprenant :
au moins un processeur ; et
au moins un support lisible par ordinateur non transitoire contenant des instructions qui, lorsqu'elles sont exécutées par l'au moins un processeur, amènent l'au moins un processeur à réaliser des opérations comprenant les opérations décrites dans l'une quelconque des revendications 1 à 14.
